(19) Europäisches Patentamt
European Patent Office
Office européen des brevets

(11) EP 3 394 063 B1

(12) FASCICULE DE BREVET EUROPEEN

(45) Date de publication et mention de la délivrance du brevet:
**02.10.2019 Bulletin 2019/40**

(21) Numéro de dépôt: **16816710.4**

(22) Date de dépôt: **23.12.2016**

(51) Int Cl.:
*C07D 471/08* (2006.01) *C07D 471/18* (2006.01)
*A61K 31/439* (2006.01) *A61P 35/00* (2006.01)

(86) Numéro de dépôt international:
**PCT/EP2016/082644**

(87) Numéro de publication internationale:
**WO 2017/109217 (29.06.2017 Gazette 2017/26)**

(54) **LIGANDS MACROCYCLIQUES AVEC GROUPEMENT(S) PICOLINATE(S), LEURS COMPLEXES AINSI QUE LEURS UTILISATIONS MÉDICALES**

MAKROCYCLISCHE LIGANDEN MIT PICOLINATGRUPPE(N), KOMPLEXE DAVON UND MEDIZINISCHE VERWENDUNGEN DAVON

MACROCYCLIC LIGANDS WITH PICOLINATE GROUP(S), COMPLEXES THEREOF AND ALSO MEDICAL USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.12.2015 FR 1563346**

(43) Date de publication de la demande:
**31.10.2018 Bulletin 2018/44**

(73) Titulaires:
- **Guerbet**
  **93420 Villepinte (FR)**
- **Université de Bretagne Occidentale**
  **29200 Brest (FR)**
- **Centre National de la Recherche Scientifique**
  **75016 Paris (FR)**

(72) Inventeurs:
- **LE FUR, Mariane**
  **29530 Plonevez-Du-Faou (FR)**
- **TRIPIER, Raphaël**
  **29860 Kersaint Plabennec (FR)**
- **ROUSSEAUX, Olivier**
  **60300 Senlis (FR)**
- **BEYLER, Maryline**
  **29200 Brest (FR)**

(74) Mandataire: **Lavoix**
**2, place d'Estienne d'Orves**
**75441 Paris Cedex 09 (FR)**

(56) Documents cités:
**EP-A1- 0 391 766**

- **H. STETTER ET AL: "Darstellung und Komplexbildung von Polyazacycloalkan-N-essigsäuren", TETRAHEDRON, vol. 37, no. 4, 1981, pages 767-772, XP055265256, ISSN: 0040-4020, DOI: 10.1016/S0040-4020(01)97695-1**
- **SILVIO AIME ET AL: "Designing novel contrast agents for magnetic resonance imaging. Synthesis and relaxometric characterization of three gadolinium(III) complexes based on functionalized pyridine-containing macrocyclic ligands", HELVETICA CHIMICA ACTA, vol. 86, no. 3, 2003, pages 615-632, XP002355606, ISSN: 0018-019X, DOI: 10.1002/HLCA.200390061**
- **FRÉDÉRIC BELLOUARD ET AL: "cis-Diprotected cyclams and cyclens: a new route to symmetrically or asymmetrically 1,4-disubstituted tetraazamacrocycles and to asymmetrically tetrasubstituted derivatives", JOURNAL OF THE CHEMICAL SOCIETY, PERKIN TRANSACTIONS 1, no. 23, 1999, pages 3499-3505, XP055265406, ISSN: 0300-922X, DOI: 10.1039/a905701c cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- K. ESZTER BORBAS ET AL: "Synthesis of asymmetrically substituted cyclen-based ligands for the controlled sensitisation of lanthanides", ORGANIC & BIOMOLECULAR CHEMISTRY, vol. 5, no. 14, 2007, page 2274, XP055265272, ISSN: 1477-0520, DOI: 10.1039/b705757a

## Description

La présente invention concerne de nouveaux ligands macrocycliques ainsi que leurs complexes, notamment radioactifs, et leurs utilisations en imagerie médicale et/ou en thérapie, notamment en radiologie interventionnelle.

La présente invention concerne également un nouveau procédé de préparation des ligands tels que selon l'invention, ainsi que leurs intermédiaires de préparation.

Le besoin de traitements ciblés et personnalisés en oncologie conduit à développer des stratégies thérapeutiques nouvelles basées sur des outils de détection précoces associés à des traitements vectorisés plus spécifiques et plus efficaces.

La radiologie interventionnelle est un axe très prometteur de la médecine individualisée. Elle permet de combiner dans une même séquence un diagnostic précis de la lésion ou tumeur et/ou son traitement instantané, guidé et contrôlé par l'image. Elle est décrite comme une chirurgie minimalement invasive et peut être de ce fait réalisée en ambulatoire, ce qui permet d'économiser de nombreuses et onéreuses journées d'hospitalisation pour une efficacité souvent comparable à la chirurgie conventionnelle. La radiologie interventionnelle peut donc représenter une alternative ou un complément au traitement chirurgical conventionnel.

La radiologie interventionnelle permet d'accéder à une lésion ou tumeur située à l'intérieur de l'organisme pour effectuer un acte diagnostique (prélèvement par exemple) ou thérapeutique. L'imagerie par fluoroscopie, échographie, scanner ou IRM permet un repérage, un guidage et un contrôle optimal du geste médical.

Il existe donc un besoin pour de nouvelles molécules utilisables en imagerie médicale et/ou en thérapie, en particulier en radiologie interventionnelle. Plus particulièrement, il existe un besoin pour des ligands permettant de complexer des éléments chimiques, en particulier des métaux, de façon à obtenir des complexes utilisables en imagerie médicale et/ou en thérapie, en particulier en radiologie interventionnelle.

De tels ligands doivent notamment être stables et complexer de façon suffisamment forte les métaux pour que ceux-ci atteignent leur cible et ne diffusent pas dans d'autres organes ou tissus sensibles comme les os, les poumons et les reins.

La présente invention a pour but de fournir de nouveaux ligands permettant de complexer des éléments chimiques, en particulier des radioéléments.

La présente invention a également pour but de fournir de nouveaux complexes, en particulier des complexes radioactifs.

La présente invention a pour but de fournir des ligands et/ou des complexes particulièrement utiles en imagerie médicale et/ou en thérapie, notamment dans le traitement des cancers.

La présente invention a également pour but de fournir une composition pharmaceutique comprenant des complexes permettant l'imagerie médicale, le ciblage et/ou le traitement de cancers.

La présente invention a pour but de fournir un nouveau procédé de préparation de ces ligands.

La présente invention concerne un composé de formule générale (I) suivante :

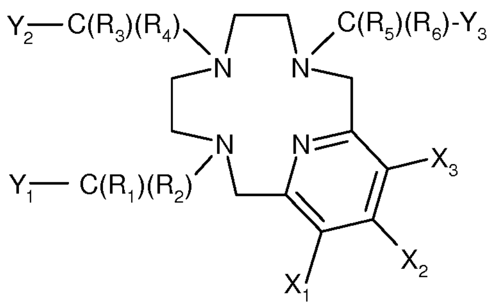

(I)

dans laquelle :

- $R_1$, $R_2$, $R_3$, $R_4$, $R_5$ et $R_6$ représentent indépendamment les uns des autres H, un groupement $(C_1\text{-}C_{20})$alkyle ou un groupement $(C_1\text{-}C_{20})$alkylène-$(C_6\text{-}C_{10})$aryle ;
  lesdits groupements alkyle, alkylène et aryle pouvant éventuellement être substitués par un ou plusieurs substituant(s) choisi(s) parmi les fonctions acides organiques, de préférence parmi le groupe constitué de -COOH, $-SO_2OH$, $-P(O)(OH)_2$ et-$O-P(O)(OH)_2$ ;

- $X_1$, $X_2$ et $X_3$ sont choisis indépendamment les uns des autres parmi le groupe constitué de : H, -C(O)N(Re)(Rd), $(C_1\text{-}C_{20})$alkyle, $(C_2\text{-}C_{20})$alcényle, $(C_2\text{-}C_{20})$alcynyle et $(C_6\text{-}C_{10})$aryle,

avec Re et Rd étant indépendamment l'un de l'autre H ou un groupement ($C_1$-$C_{20}$)alkyle,
lesdits groupements alkyle, alcényle et alcynyle pouvant éventuellement comprendre un ou plusieurs hétéroatome(s) et/ou un ou plusieurs ($C_6$-$C_{10}$)arylène(s) dans leur chaîne et pouvant être éventuellement substitués par un ($C_6$-$C_{10}$)aryle ;
lesdits groupements alkyle, alcényle, alcynyle et aryle pouvant éventuellement être substitués par un ou plusieurs substituant(s) choisi(s) parmi les fonctions acides organiques, de préférence parmi le groupe constitué de -COOH, -$SO_2$OH, -P(O)$(OH)_2$, et-O-P(O)$(OH)_2$ ;

- $Y_1$, $Y_2$ et $Y_3$ représentent indépendamment les uns des autres un groupement C(O)OH ou un groupement de formule (II) suivante :

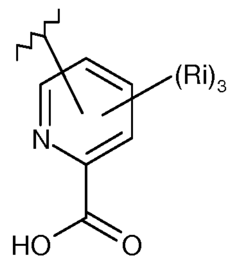

(II)

dans lequel :

- les radicaux Ri sont choisis indépendamment les uns des autres parmi le groupe constitué de : H, halogène, $N_3$, ($C_1$-$C_{20}$)alkyle, ($C_2$-$C_{20}$)alcényle, ($C_2$-$C_{20}$)alcynyle et ($C_6$-$C_{10}$)aryle,

lesdits groupements alkyle, alcényle et alcynyle pouvant éventuellement comprendre un ou plusieurs hétéroatome(s) et/ou un ou plusieurs ($C_6$-$C_{10}$)arylène(s) dans leur chaîne et pouvant être éventuellement substitués par un ($C_6$-$C_{10}$)aryle ;
lesdits groupements alkyle, alcényle, alcynyle et aryle pouvant éventuellement être substitués par un ou plusieurs substituant(s) choisi(s) parmi les fonctions acides organiques, de préférence parmi le groupe constitué de -COOH, -$SO_2$OH, -P(O)$(OH)_2$, et-O-P(O)$(OH)_2$ ;
et

l'un au moins des radicaux $Y_1$, $Y_2$ et $Y_3$ étant un groupement de formule (II) ;
ou l'un de ses sels pharmaceutiquement acceptables.

Selon un mode de réalisation, la présente invention concerne un composé de formule générale (I) suivante :

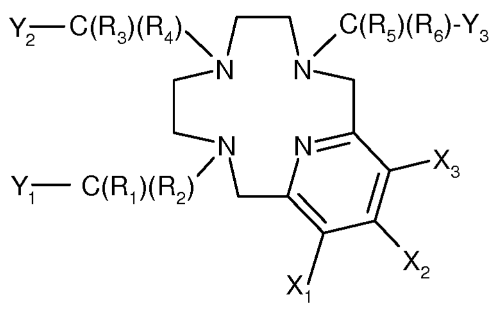

(I)

dans laquelle :

- $R_1$, $R_2$, $R_3$, $R_4$, $R_5$ et $R_6$ représentent indépendamment les uns des autres H, un groupement ($C_1$-$C_{20}$)alkyle ou un groupement ($C_1$-$C_{20}$)alkylène-($C_6$-$C_{10}$)aryle ;
- $X_1$, $X_2$ et $X_3$ sont choisis indépendamment les uns des autres parmi le groupe constitué de : H, -C(O)N(Re)(Rd), ($C_1$-$C_{20}$)alkyle, ($C_2$-$C_{20}$)alcényle, ($C_2$-$C_{20}$)alcynyle et ($C_6$-$C_{10}$)aryle,
avec Re et Rd étant indépendamment l'un de l'autre H ou un groupement ($C_1$-$C_{20}$)alkyle,

lesdits groupements alkyle, alcényle et alcynyle pouvant éventuellement comprendre un ou plusieurs hétéroatome(s) et/ou un ou plusieurs ($C_6$-$C_{10}$)arylène(s) dans leur chaîne et pouvant être éventuellement substitués par un ($C_6$-$C_{10}$)aryle ;

- $Y_1$, $Y_2$ et $Y_3$ représentent indépendamment les uns des autres un groupement C(O)OH ou un groupement de formule (II) suivante :

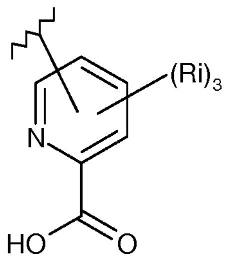

(II)

dans lequel :

- les radicaux Ri sont choisis indépendamment les uns des autres parmi le groupe constitué de : H, halogène, $N_3$, ($C_1$-$C_{20}$)alkyle, ($C_2$-$C_{20}$)alcényle, ($C_2$-$C_{20}$)alcynyle et ($C_6$-$C_{10}$)aryle,

lesdits groupements alkyle, alcényle et alcynyle pouvant éventuellement comprendre un ou plusieurs hétéroatome(s) et/ou un ou plusieurs ($C_6$-$C_{10}$)arylène(s) dans leur chaîne et pouvant être éventuellement substitués par un ($C_6$-$C_{10}$)aryle ; et
l'un au moins des radicaux $Y_1$, $Y_2$ et $Y_3$ étant un groupement de formule (II) ;
ou l'un de ses sels pharmaceutiquement acceptables.

Les inventeurs ont mis au point de nouveaux complexes ligands-métal (complexes appelés également chélates) à partir du macrocycle pyclène (3,6,9,15-tétraazabicyclo[9.3.1]pentadeca-1(15),11,13-triène), diversement substitué par des groupements acétate et/ou picolinate (acide méthylène-6-pyridine-2-carboxylique). Le macrocycle pyclène est de formule suivante :

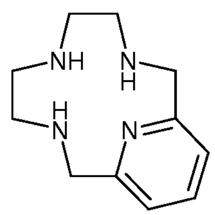

De façon surprenante, les complexes selon l'invention présentent une bonne stabilité thermodynamique ainsi qu'une bonne inertie cinétique. De plus, de façon tout aussi surprenante, les inventeurs ont découvert que les complexes selon l'invention pouvaient être solubilisés dans une huile iodée telle que LIPIODOL®, une huile iodée fabriquée et commercialisée par la société Guerbet et qui est constituée par des esters éthyliques des acides gras iodés de l'huile d'oeillette. Ainsi, les complexes selon l'invention solubilisés dans une huile iodée telle que LIPIODOL® peuvent être vectorisés notamment vers le foie et peuvent permettre de visualiser et/ou traiter les cancers, par exemple les cancers du foie.

Ces complexes présentent également un bon rendement radiochimique de l'extraction dans une huile iodée telle que LIPIODOL®. Ils présentent en particulier une bonne incorporation de la radioactivité dans une huile iodée telle que LIPIODOL® et une bonne stabilité de la solution radioactive de LIPIODOL® lors de tests *in vitro.*

En particulier, la combinaison des propriétés de vectorisation de LIPIODOL®, d'efficacité thérapeutique des radioéléments, et la bonne tolérance de ces produits permettent de proposer un traitement thérapeutique des cancers sûr et plus facile à mettre en oeuvre.

La vectorisation des complexes selon l'invention par une huile iodée telle que LIPIODOL® permet notamment d'éviter une mauvaise délivrance des complexes en diminuant le risque d'effets indésirables dans les organes sains, en particulier le foie sain ou dans les organes extra-hépatiques, et permet d'atteindre la dose de radioactivité efficace dans la tumeur.

Plus particulièrement, cette vectorisation facilite le travail du radiologue interventionnel au moment de l'injection

des complexes selon l'invention. Par exemple, lors d'une injection intra-artérielle suivie sous fluoroscopie, le geste du radiologue sera plus précis et plus sûr en permettant un ajustement de la vitesse de délivrance des complexes en fonction de la capture par la tumeur des complexes selon l'invention.

## Définitions

On entend par « ligand », un composé capable de complexer un élément chimique tel qu'un métal, de préférence un radioélément. Selon un mode de réalisation, les ligands au sens de l'invention sont sous forme anionique et peuvent complexer des radioéléments sous forme cationique, par exemple des cations métalliques au degré d'oxydation (III). Selon la présente invention, les composés de formule (I) sont des ligands.

On entend par « radioélément », tout radioisotope connu d'un élément chimique, qu'il soit naturel ou produit artificiellement. Selon un mode de réalisation, le radioélément est choisi parmi les radioisotopes de l'yttrium et des lanthanides. Par « lanthanides » sont désignés les atomes choisis parmi le groupe constitué de : La, Ce, Pr, Nd, Pm, Sm, Eu, Gd, Tb, Dy, Ho, Er, Tm, Yb et Lu.

On entend par « complexe », l'association d'un ligand tel que défini ci-dessus avec un élément chimique, de préférence un radioélément tel que défini ci-dessus. Le terme « complexe » étant synonyme de « chélate ».

La « stabilité thermodynamique » représente l'affinité du ligand pour un élément donné, en particulier un métal donné. Il s'agit de la constante d'équilibre de la réaction de complexation :

Métal + Ligand ⇄ Complexe

dont l'expression mathématique est la suivante :

$$K = \frac{[Métal] \times [Ligand]}{[Complexe]}$$

Les valeurs sont en général exprimées sous forme de logarithme décimal logK. Selon un mode de réalisation, les complexes selon l'invention sont de forte affinité. Selon un mode de réalisation, les complexes selon l'invention ont une constante thermodynamique d'équilibre au moins égale à 16 (LogK au moins égal à 16).

Les complexes formés selon la réaction d'équilibre décrite ci-dessus sont susceptibles de se dissocier sous l'action de différents facteurs (pH, présence de métaux ou ligands compétiteurs). Cette dissociation peut avoir des conséquences importantes dans le cadre de l'utilisation des complexes en médecine humaine car elle entraine une libération du métal dans l'organisme. Afin de limiter ce risque, des complexes à dissociation lente sont recherchés, c'est-à-dire des complexes ayant une bonne inertie cinétique. L'inertie cinétique peut être déterminée par des tests de dissociation en milieu acide. Ces expériences conduisent à la détermination pour chaque complexe d'un temps de demi-vie ($T_{1/2}$) dans des conditions définies.

Dans le contexte de l'invention, le terme « traiter », « traitement » ou « traitement de thérapeutique » signifie inverser, soulager, inhiber la progression de, du trouble ou l'affection auquel ce terme est applicable, ou un ou plusieurs symptômes d'un tel trouble.

Le terme « imagerie médicale » désigne les moyens d'acquisition et de restitution d'images du corps humain ou animal à partir de différents phénomènes physiques tels que l'absorption des rayons X, la résonance magnétique nucléaire, la réflexion d'ondes ultrasons ou la radioactivité. Selon un mode de réalisation, le terme « imagerie médicale » se réfère à l'imagerie par rayons X, l'IRM (Imagerie par Résonance Magnétique), la tomographie d'émission monophotonique (TEMP ou SPECT pour « Single Photon Emission Computed Tomography »), la tomoscintigraphie par émission de positons (TEP) et la luminescence. De préférence, la méthode d'imagerie médicale est l'imagerie par rayons X. Selon un mode de réalisation particulier, la méthode d'imagerie médicale est l'IRM si le complexe selon l'invention comprend du Gd(III), SPECT si le complexe selon l'invention comprend un émetteur gamma et TEP si le complexe selon l'invention comprend un émetteur béta+.

La capacité des agents de contraste à accélérer les vitesses de relaxation 1/T1 et 1/T2 des protons de l'eau est mesurée par une grandeur, la relaxivité. On définit notamment la relaxivité (r) d'un agent de contraste comme la vitesse de relaxation, normalisée par la concentration de l'agent de contraste.

Le terme « ($C_1$-$C_{20}$)alkyle » désigne des hydrocarbures aliphatiques saturés, qui peuvent être linéaires ou ramifiés et comprennent de 1 à 20 atomes de carbone. De préférence, les alkyles comprennent de 1 à 15 atomes de carbone, par exemple 6, 7, 8, 9, 10, 11, 12, 13, 14 ou 15 atomes de carbone. Par « ramifié », on entend qu'un groupement alkyle est substitué sur la chaîne alkyle principale.

Le terme « ($C_1$-$C_{20}$)alkylène » désigne un radical alkyle tel que défini ci-dessus, divalent.

Le terme « ($C_2$-$C_{20}$)alcène » désigne un alkyle tel que défini ci-dessus, comprenant au moins une double liaison

carbone-carbone.

Le terme « ($C_2$-$C_{20}$)alcyne » désigne un alkyle tel que défini ci-dessus, comprenant au moins une triple liaison carbone-carbone.

Le terme « ($C_6$-$C_{10}$)aryle » désigne des composés aromatiques hydrocarbonés monocycliques, bicycliques ou tricycliques, en particulier le phényle et le naphtyle.

Le terme « arylène » désigne un aryle tel que défini ci-dessus, divalent, en particulier le phénylène et le naphtylène.

Selon un mode de réalisation, « halogène » désigne F, Cl, Br, I et At.

Parmi les hétéroatomes, peuvent notamment être cités P, N, O et S, de préférence N et O. Selon un mode de réalisation particulier, les composés de formule générale (I) comprennent 1 ou 2 hétéroatome(s). De préférence, des groupements -O-($C_1$-$C_{20}$)alkyle (également appelés groupement alkoxy), -O-($C_2$-$_{20}$)alkényle et -O-($C_2$-$C_{20}$)alkynyle sont présents.

Le terme « LIPIODOL » se réfère à une huile iodée et de manière préférentielle à la spécialité pharmaceutique solution injectable fabriquée et commercialisée par Guerbet et constituée par des esters éthyliques des acides gras iodés de l'huile d'oeillette. LIPIODOL® est un produit notamment utilisé pour la visualisation, la localisation et/ou la vectorisation au cours de la chimioembolisation transartérielle du carcinome hépatocellulaire au stade intermédiaire, chez l'adulte ainsi que pour le diagnostic par voie artérielle hépatique sélective de l'extension hépatique des lésions malignes hépatiques ou non.

Par acide organique (ou fonction acide organique), on entend un composé organique (ou une fonction organique) présentant des propriétés acides, c'est-à-dire capable de libérer un cation $H^+$, ou $H_3O^+$ en milieux aqueux. Parmi les acides organiques, on peut citer les acides carboxyliques, les acides sulfoniques, les phosphates et les phosphonates. De préférence, les fonctions acides organiques selon l'invention sont choisies parmi le groupe constitué de -COOH, -$SO_2$OH, -P(O)OH, -P(O)$(OH)_2$, et -O-P(O)$(OH)_2$, et plus préférentiellement -COOH. De telles fonctions acides sont salifiables et peuvent se présenter sous leur forme basique. En particulier, ces fonctions acides se présentent sous forme de sels pharmaceutiquement acceptables, tels que définis ci-dessous ; par exemple, sous forme de sel de sodium ou de méglumine (1-Deoxy-1-(methylamino)-D-glucitol ou *N*-Methyl-D-glucamine).

**Les huiles iodées**

On entend par le terme « acide gras » désigner des acides carboxyliques aliphatiques saturés ou insaturés présentant une chaîne carbonée d'au moins 4 atomes de carbone. Les acides gras naturels possèdent une chaîne carbonée de 4 à 28 atomes de carbone (généralement un nombre pair). On parle d' « acide gras à longue chaîne » pour une longueur de 14 à 22 carbones et à très longue chaîne s'il y a plus de 22 carbones. On parle au contraire d' « acide gras à courte chaîne » pour une longueur de 4 à 10 carbones, notamment 6 à 10 atomes de carbone, en particulier 8 ou 10 atomes de carbone. L'homme du métier connaît la nomenclature associée et en particulier utilise :

- Ci-Cp pour désigner une fourchette d'acides gras en Ci à Cp
- Ci+Cp, le total des acides gras en Ci et des acides gras en Cp

Par exemple :

- les acides gras de 14 à 18 atomes de carbone s'écrivent « acides gras en C14-C18 »
- le total des acides gras en C16 et des acides gras en C18 s'écrit C16 +C18.
- pour un acide gras saturé, un homme du métier utilisera la nomenclature suivante Ci : 0, où i est le nombre d'atomes de carbone de l'acide gras. L'acide palmitique sera par exemple désigné par la nomenclature (C16 :0).
- pour un acide gras insaturé, un homme du métier utilisera la nomenclature suivante Ci : x n-N où N sera la position de la double liaison dans l'acide gras insaturé en partant du carbone opposé au groupe acide, i est le nombre d'atomes de carbone de l'acide gras, x est le nombre de double liaisons (insaturations) de cet acide gras. L'acide oléique, sera par exemple désigné par la nomenclature (C18 :1 n-9).

De façon avantageuse, l'huile iodée selon l'invention comprend ou est constituée de dérivés d'acides gras iodés, préférentiellement d'esters éthyliques d'acides gras iodés, plus préférentiellement d'esters éthyliques d'acides gras iodés d'huile d'oeillette, d'huile d'olive, d'huile de graines de colza, d'huile d'arachide, d'huile de graines de soja ou d'huile de noix, encore plus préférentiellement d'esters éthyliques d'acides gras iodés d'huile d'oeillette ou d'huile d'olive. Plus préférentiellement, l'huile iodée selon l'invention comprend ou est constituée d'esters éthyliques d'acides gras iodés d'huile d'oeillette (aussi appelée pavot noir ou *Papaver somniferum var. nigrum*). L'huile d'oeillette, aussi appelée huile de graines de pavot ou huile de graines d'oeillette, contient préférentiellement plus de 80% d'acides gras insaturés (en particulier de l'acide linoléique (C18 :2 n-6) et de l'acide oléique (C18 :1 n-9)) dont au moins 70% d'acide

linoléique et au moins 10% d'acide oléique. L'huile iodée est obtenue à partir de la complète iodation d'une huile telle que l'huile d'oeillette dans des conditions permettant une liaison d'un atome d'iode pour chaque double liaison des acides gras insaturés (Wolff et al. 2001, Medicine 80, 20-36) suivie d'une trans-estérification.

L'huile iodée selon l'invention contient préférentiellement de 29 à 53% (m/m), plus préférentiellement 37% à 39% (m/m) d'iode.

Comme exemples d'huiles iodées peuvent être cités le Lipiodol®, le Brassiodol® (issu de l'huile de graines de colza (*Brassica compestis*), le Yodiol® (issu de l'huile d'arachide), l'Oriodol® (issu de l'huile d'oeillette mais sous forme de triglycérides d'acides gras), le Duroliopaque® (issu de l'huile d'olive).

Préférentiellement, l'huile iodée est Lipiodol®, une huile iodée utilisée comme produit de contraste et dans certaines procédures de radiologie interventionnelle. Cette huile est un mélange d'esters éthyliques d'acides gras iodés et non-iodés d'huile de graines d'oeillette. Elle consiste majoritairement (en particulier, plus de 84%) en un mélange d'esters éthyliques d'acides gras iodés à longue-chaine (en particulier des acides gras en C18) issus de l'huile de graines d'oeillette, préférentiellement en un mélange de monoiodostéarate d'éthyle et de diiodostéarate d'éthyle. L'huile iodée peut aussi être une huile à base d'ester éthylique monoiodé d'acide stéarique (C18 :0) issu de l'huile d'olive. Un produit de ce type, s'appelant Duroliopaque® était commercialisé il y a quelques années.

Les caractéristiques principales de Lipiodol® sont les suivantes :

| **Composés** | **Proportions dans le mélange d'acide gras** |
|---|---|
| Palmitate d'éthyle (Ethyl C16 :0) | 4,6 à 6,7% (m/m), préférentiellement 4,8% (m/m) |
| Stéarate d'éthyle (Ethyl C18 :0) | 0,8 à 1,9% (m/m), préférentiellement 1,2% (m/m) |
| Monoiodostéarate d'éthyle | 11,3 à 15,3% (m/m), préférentiellement 13,4% (m/m) |
| Diiodostéarate d'éthyle | 73,5 à 82,8% (m/m), préférentiellement 78,5% (m/m) |

| **Autres caractéristiques du Lipiodol® :** | |
|---|---|
| Iode | 37% à 39% (m/m) (soit 480 mg/ml) |
| Viscosité<br>à 37°C<br>à 20°C | <br>25 mPa.s<br>50 mPa.s |
| Densité | 1,268 - 1,290 g/cm$^3$ à 20°C, préférentiellement 1,28 |

**Composés de formule générale (I)**

Les composés de formule générale (I) peuvent avoir des centres de chiralité et peuvent être sous forme racémique ou énantiomérique. Les composés de formule générale (I) sont compris sous leurs différentes formes : diastéréoisomères, énantiomères ou mélange racémique.

Selon un mode de réalisation, les composés de formule générale (I) sont sous forme de sel, de préférence sous forme de sel pharmaceutiquement acceptable.

Par « sel pharmaceutiquement acceptable », on désigne notamment des sels permettant de conserver les propriétés et l'efficacité biologique des composés selon l'invention. Des exemples de sels pharmaceutiquement acceptables se trouvent dans Berge, et al. ((1977) J. Pharm. Sd, vol. 66, 1). Par exemple, les composés de formule générale (I) sont sous forme de sel de sodium ou de méglumine (1-Deoxy-1-(methylamino)-D-glucitol ou *N*-Methyl-D-glucamine).

L'invention concerne également les isomères optiques (énantiomères), géométriques (cis/trans ou Z/E), les tautomères et les solvates tels que les hydrates des composés de formule (I).

Selon un mode de réalisation, $X_1$, $X_2$ et $X_3$ sont choisis indépendamment les uns des autres parmi le groupe constitué de : H, ($C_1$-$C_{20}$)alkyle, ($C_2$-$C_{20}$)alcényle et ($C_2$-$C_{20}$)alcynyle, lesdits groupements alkyle, alcényle et alcynyle pouvant éventuellement comprendre un ou plusieurs hétéroatome(s) dans leur chaîne.

Selon un mode réalisation particulier, $X_1$, $X_2$ et $X_3$ sont choisis indépendamment les uns des autres parmi le groupe constitué de : H et ($C_1$-$C_{20}$)alkyle. Plus particulièrement $X_1$, $X_2$ et $X_3$ sont H.

Selon un mode de réalisation, dans les composés de formule générale (I), lorsque les radicaux $Y_1$, $Y_2$ ou $Y_3$ représentent un groupement de formule (II), les radicaux correspondant $R_1$ et $R_2$, $R_3$ et $R_4$ ou $R_5$ et $R_6$ représentent H.

Selon un mode de réalisation, les radicaux $R_1$, $R_2$, $R_3$, $R_4$, $R_5$ et $R_6$ représentent indépendamment les uns des autres H ou un groupement ($C_1$-$C_{20}$)alkyle. Selon un mode de réalisation particulier, $R_1$, $R_2$, $R_3$, $R_4$, $R_5$ et $R_6$ représentent

H.

Selon un mode de réalisation, les radicaux Ri sont choisis indépendamment les uns des autres parmi le groupe constitué de : H, $(C_1-C_{20})$alkyle, $(C_2-C_{20})$alcényle et $(C_2-C_{20})$alcynyle, lesdits groupements alkyle, alcényle et alcynyle pouvant éventuellement comprendre un ou plusieurs hétéroatome(s) choisi(s) parmi N, O ou S.

Selon un mode de réalisation, les radicaux Ri sont choisis indépendamment les uns des autres parmi le groupe constitué de : H, $(C_1-C_{20})$alkyle, $(C_2-C_{20})$alcényle et $(C_2-C_{20})$alcynyle.

Selon un mode de réalisation particulier, les radicaux Ri sont choisis indépendamment les uns des autres parmi le groupe constitué de : H et $(C_2-C_{15})$alcynyle.

Selon un mode de réalisation, le groupement de formule (II) est le groupement suivant :

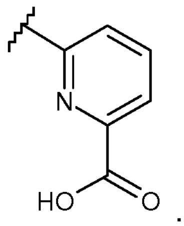

Selon un mode de réalisation particulier :

- $R_1$, $R_2$, $R_3$, $R_4$, $R_5$ et $R_6$ représentent H ; et
- les radicaux $Y_1$, $Y_2$, et $Y_3$ représentent soit un groupement C(O)OH, soit un composé de formule suivante :

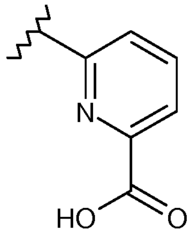

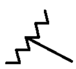 représente la liaison à l'atome de carbone du groupement $C(R_1)(R_2)$, $C(R_3)(R_4)$ ou $C(R_5)(R_6)$.

Selon un mode de réalisation, le ligand selon l'invention est de formule générale (I-1) suivante :

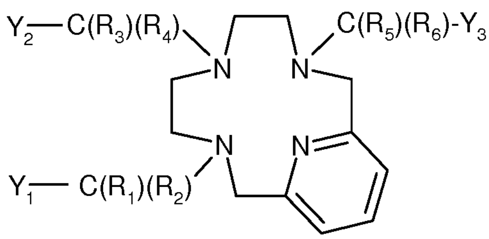

(I-1)

dans laquelle :

- $R_1$, $R_2$, $R_3$, $R_4$, $R_5$ et $R_6$ représentent indépendamment les uns des autres H ou un groupement $(C_1-C_{20})$alkyle ;
- $Y_1$, $Y_2$ et $Y_3$ représentent indépendamment les uns des autres un groupement C(O)OH ou un groupement de formule (II) suivante :

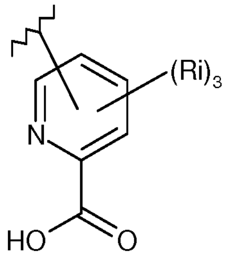

(II)

dans lequel :

- les radicaux Ri sont choisis indépendamment les uns des autres parmi le groupe constitué de : H, ($C_1$-$C_{20}$)alkyle, ($C_2$-$C_{20}$)alcényle et ($C_2$-$C_{20}$)alcynyle, lesdits groupements alkyle, alcényle et alcynyle pouvant éventuellement comprendre un ou plusieurs hétéroatome(s) dans leur chaîne; et

l'un au moins des radicaux $Y_1$, $Y_2$ et $Y_3$ étant un groupement de formule (II) ;
ou l'un de ses sels pharmaceutiquement acceptables.

Selon un mode de réalisation, les composés de formule générale (I) peuvent être symétriques ou dissymétriques. Les composés de formule générale (I) sont symétriques lorsque les groupements -$C(R_1)(R_2)$-$Y_1$ et -$C(R_5)(R_6)$-$Y_3$ sont identiques. Les composés de formule générale (I) sont dissymétriques lorsque les groupements -$C(R_1)(R_2)$-$Y_1$ et-$C(R_5)(R_6)$-$Y_3$ sont différents. Selon un mode de réalisation, les groupements alkyle, alcényle, alcynyle et aryle présents dans les radicaux Ri sont éventuellement substitués par un ou plusieurs substituant(s), de préférence un substituant, choisi(s) parmi le groupe constitué de -COOH, -$SO_2OH$, -$P(O)(OH)_2$, et -O-$P(O)(OH)_2$; tandis que les groupements alkyle, alcényle, alcynyle, alkylène et aryle des radicaux $R_1$ à $R_6$ et $X_1$ à $X_3$ ne sont pas substitués par lesdits groupes.

Selon un mode de réalisation, dans les radicaux $X_1$, $X_2$ et $X_3$, lesdits groupements alkyle, alcényle, alcynyle et aryle, de préférence alkyle, peuvent éventuellement être substitués par un ou plusieurs substituant(s), de préférence un substituant, choisi(s) parmi le groupe constitué de -COOH, -$SO_2OH$, -$P(O)(OH)_2$, et -O-$P(O)(OH)_2$.

Selon un mode de réalisation, dans les radicaux $R_1$, $R_2$, $R_3$, $R_4$, $R_5$ et $R_6$ les groupements alkyle, alkylène et aryle, préférentiellement alkyle, peuvent éventuellement être substitués par un ou plusieurs substituant(s), de préférence un substituant, choisi(s) parmi le groupe constitué de -COOH, -$SO_2OH$, -$P(O)(OH)_2$, et -O-$P(O)(OH)_2$.

Selon un mode de réalisation, dans les radicaux Ri, les groupements alkyle, alcényle, alcynyle et aryle, de préférence alcynyle, peuvent éventuellement être substitués par un ou plusieurs substituant(s), de préférence un substituant, choisi(s) parmi le groupe constitué de -COOH, -$SO_2OH$, -$P(O)(OH)_2$, et -O-$P(O)(OH)_2$.

De préférence, ledit substituant est un groupement -COOH.

Selon un mode de réalisation, le composé de formule (I) est choisi parmi le groupe constitué des composés suivants :

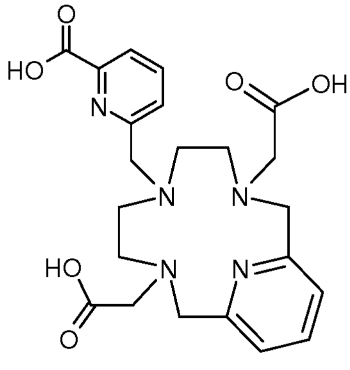

**Pc-2a1pa sym**

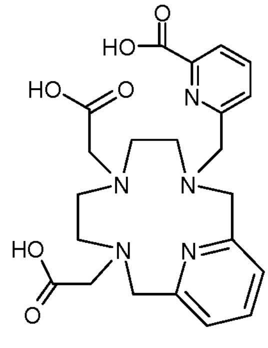

**Pc-2a1pa asym**

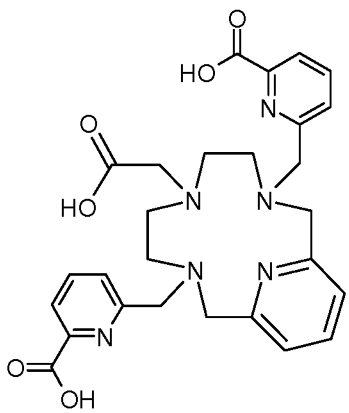

Pc-1a2pa sym

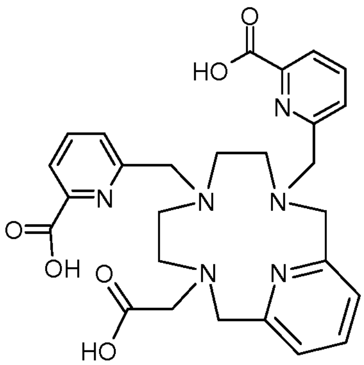

Pc-1a2pa asym

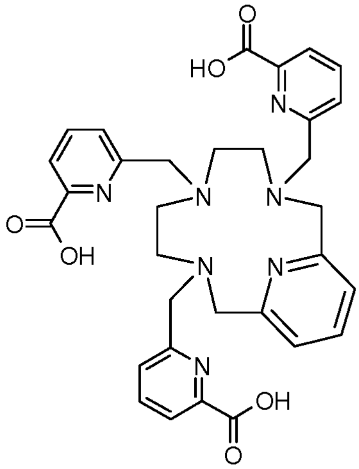

Pc3pa

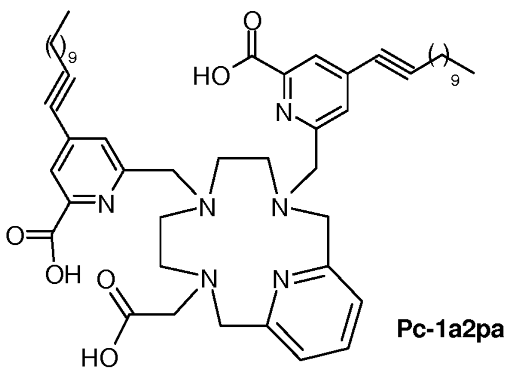

Pc-1a2pa asym C12

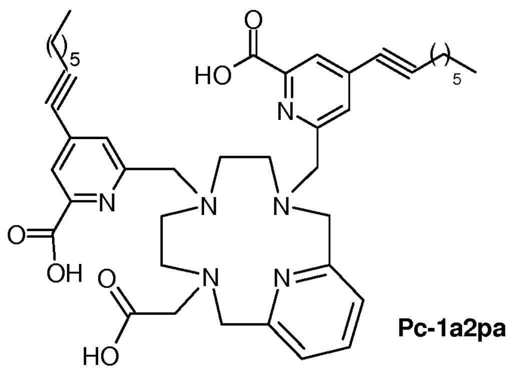

Pc-1a2pa asym C8

ou l'un de leurs sels pharmaceutiquement acceptables.

Selon un mode de réalisation particulier, le composé de formule (I) est le composé suivant :

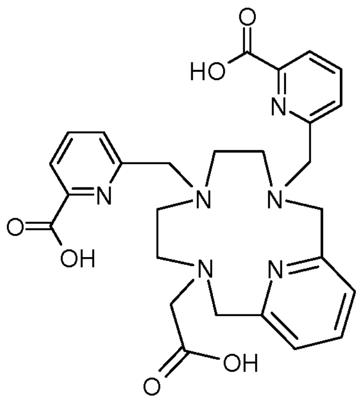

Pc-1a2pa asym

,

ou l'un de ses sels pharmaceutiquement acceptable.

**Complexes**

L'invention concerne également un complexe d'un composé de formule (I) ou d'un de ses sels, tel que défini ci-dessus, avec un élément chimique M, de préférence un métal.

Selon un mode de réalisation, l'élément chimique M est un cation métallique choisi parmi le groupe constitué du bismuth (III), plomb (II), cuivre (II), cuivre (I), gallium (III), zirconium (IV), technetium (III), indium (III), rhenium (VI), astate (III), yttrium (III), samarium (III), actinium (III), lutétium (III), terbium (III), holmium (III), gadolinium (III), europium(III) et yttrium (III), de préférence le gadolinium(III).

Selon un mode de réalisation particulier, l'élément chimique M est un radioélément choisi parmi le groupe constitué de $^{212}Bi(^{212}Pb)$, $^{213}Bi(III)$, $^{64}Cu(II)$, $^{67}Cu(II)$, $^{68}Ga(III)$, $^{89}Zr(IV)$, $^{99m}Tc(III)$, $^{111}In(III)$, $^{186}Re(VI)$, $^{188}Re(VI)$, $^{211}At(III)$, $^{225}Ac(III)$, $^{153}Sm(III)$, $^{149}Tb(III)$, $^{166}Ho(III)$, $^{212}Bi(^{212}Pb)$, $^{213}Bi(III)$, de préférence $^{177}Lu(III)$, $^{90}Y(III)$ et $^{166}Ho(III)$. De préférence, M est un radioélément choisi parmi les isotopes radioactifs de l'yttrium et des lanthanides.

En particulier, parmi les radioéléments selon l'invention, on peut citer : $^{177}Lu$, $^{149}Tb$, $^{152}Tb$, $^{155}Tb$, $^{161}Tb$, $^{86}Y$ et $^{153}Sm$. Selon un mode de réalisation particulier, M est un radioélément choisi parmi le groupe constitué de $^{166}Ho$, $^{177}Lu$ et $^{90}Y$.

Selon un mode de réalisation, ledit complexe est de formule générale (III) suivante :

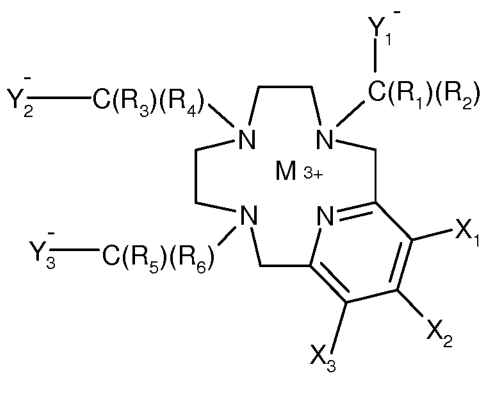

(III),

dans laquelle $R_1$, $R_2$, $R_3$, $R_4$, $R_5$, $R_6$, $X_1$, $X_2$, $X_3$, $Y_1$, $Y_2$, $Y_3$ et M sont tels que définis ci-dessus. En particulier, dans la formule générale (III), chacun des groupements $Y_1$, $Y_2$ et $Y_3$ comprend un groupement $C(O)O^-$, permettant la complexation à l'élément M.

**Composition pharmaceutique**

L'invention concerne également une composition pharmaceutique comprenant un composé de formule (I) tel que défini ci-dessus ou un complexe tel que défini ci-dessus, et éventuellement un ou plusieurs excipient(s) pharmaceutiquement acceptable(s).

La composition peut en outre comprendre un tampon choisi parmi les tampons d'usage établi comme par

exemple les tampons lactate, tartrate malate, maléate, succinate, ascorbate, carbonate, Tris((hydroxyméthyl)aminométhane), HEPES (acide 2-[4-(2-hydroxyéthyl)-1-pipérazine]éthanesulfonique), MES (acide 2-morpholino éthanesulfonique) et les mélanges de ceux-ci.

La composition pharmaceutique peut comprendre une phase huileuse, notamment une huile iodée. Selon un mode de réalisation particulier, la composition pharmaceutique comprend en outre des esters éthyliques d'acides gras iodés de l'huile d'oeillette.

Selon un mode de réalisation, la composition pharmaceutique selon l'invention est constituée d'une huile iodée et de complexes selon l'invention. Typiquement, la composition pharmaceutique selon l'invention est constituée de LIPIODOL® et de complexes selon l'invention. LIPIODOL® est constitué d'esters éthyliques d'acides gras iodés de l'huile d'oeillette.

De préférence, la composition pharmaceutique selon l'invention est radio-opaque, et donc visible par radiographie aux rayons X.

Selon un mode de réalisation particulier, la composition pharmaceutique est une composition injectable. Selon un mode de réalisation, la composition pharmaceutique selon l'invention est administrée par injection hépatique intra-artérielle.

L'invention concerne un complexe ou une composition pharmaceutique tels que définis ci-dessus, pour son utilisation dans le traitement de cancers.

L'invention concerne également un complexe ou une composition pharmaceutique tels que définis ci-dessus, pour son utilisation en imagerie médicale.

L'invention concerne l'utilisation d'un complexe tel que défini ci-dessus pour la préparation d'un médicament pour le traitement de cancers.

L'invention concerne également l'utilisation d'un complexe ou d'une composition pharmaceutique tels que définis ci-dessus en imagerie médicale.

L'invention concerne une méthode de traitement thérapeutique d'un patient atteint d'un cancer, comprenant l'administration audit patient d'un complexe ou d'une composition pharmaceutique tels que définis ci-dessus. En particulier ladite méthode de traitement ne comprend pas d'étape de traitement chirurgical.

L'invention concerne également une méthode d'imagerie médicale d'une tumeur comprenant :

- une étape d'administration à un patient atteint d'un cancer d'un complexe ou d'une composition pharmaceutique selon l'invention ; et
- une étape de détection de la tumeur par une méthode d'imagerie médicale.

Par « cancer », on entend une prolifération cellulaire anormale (également appelée tumeur) au sein d'un tissu normal de l'organisme. Ces cellules cancéreuses dérivent toutes d'un même clone, cellule initiatrice du cancer qui a acquis certaines caractéristiques lui permettant de se diviser indéfiniment. Au cours de l'évolution de la tumeur, certaines cellules cancéreuses peuvent migrer hors de leur lieu de production et former des métastases.

Parmi les cancers, on peut notamment citer les cancers du foie, en particulier les cancers primitifs du foie, de préférence les hépatocarcinomes. Selon un mode de réalisation particulier, on peut citer parmi les cancers, l'hépatocarcinome, l'hémangioendothéliome épithélioïde, le cholangiocarcinome, les tumeurs neuro-endocrines et les métastases d'autres cancers telles que les métastases du cancer colorectal.

Selon un mode de réalisation particulier, le cancer est un carcinome hépatocellulaire au stade intermédiaire, chez l'adulte.

**Procédé de préparation des composés de formule générale (I)** et **radiomarquage**

Les composés de formule générale (I) peuvent être préparés selon deux procédés, selon que les composés de formule générale (I) sont symétriques ou dissymétriques.

Dans ces procédés de préparation, les étapes de déprotection sont connues de l'homme du métier et correspondent à des réactions classiques d'hydrolyse d'un amide. Les étapes de fonctionnalisation sont également connues de l'homme du métier et correspondent à des réactions d'alkylation classiques (cf. Loic Bellouard J CHEM S Perkin 1, (23), 1999, pp. 3499-3505).

La première approche applicable pour les composés symétriques implique la synthèse totale du macrocycle pyclène avec nécessité de différencier l'atome d'azote central (6) des atomes d'azote en positions latérales (3 et 9).

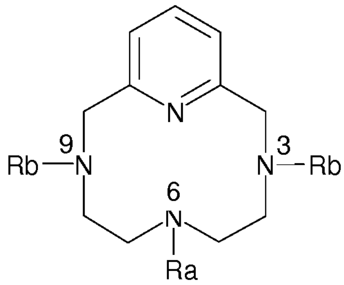

Pour cela, la méthode proposée par Siauge et coll. est applicable (Tetrahedron Volume 57 Issue 22 Pages 4713-4718). Cette méthode s'appuie d'une part, sur l'utilisation d'un groupement Nosyle (2-nitrophénylsulfonyle) à la place des groupes tosyles habituellement choisis pour réaliser des macrocyclisations selon la méthode générale de Richman et Atkins (J. Am. Chem. Soc. 1974, 96, 2268-2270) et sur la réactivité sélective des amines primaires de la diéthylène triamine. Selon ce principe, il est possible de préparer des intermédiaires dérivés de la diéthylènetriamine différemment substitués sur l'amine centrale secondaire (préfigurant la substitution en position 6). Ces composés sont des intermédiaires clés qui pourront ensuite être engagés dans la réaction de macrocyclisation. La présence des groupements Nosyles, plus facile à déprotéger que les analogues Tosyles, autorise l'introduction sur le macrocycle d'une plus grande diversité de substituants en position 6. Le schéma 1 ci-dessous illustre ce procédé de préparation.

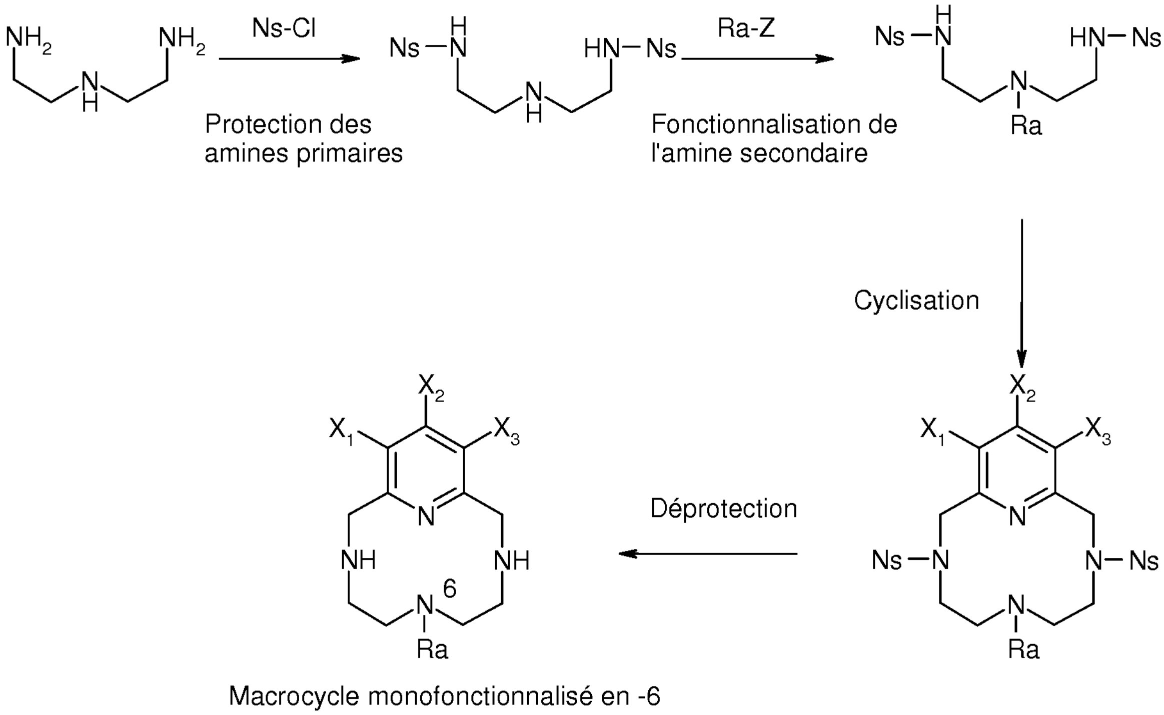


*Schéma 1*

Dans le schéma 1, $X_1$, $X_2$ et $X_3$ sont tels que définis pour les composés de formule générale (I), le groupe Ra est un groupement $-C(R_3)(R_4)-Y_2$, avec $R_3$, $R_4$ et $Y_2$ tels que définis pour les composés de formule générale (I), un ester ou un groupement protecteur orthogonal comme le ter-butoxycarbonyle et Z est un groupe partant tel que Cl, Br, I, tosylate mésylate ou triflate. Ce procédé de préparation est appelé « Voie directe » ou « Voie Boc » dans les exemples.

L'invention concerne également un procédé de préparation pour les composés de formule générale (I) selon l'invention étant dissymétriques. Ce procédé de préparation est avantageusement basé sur la réaction d'un diester de l'acide oxalique sur le pyclène qui permet de bloquer deux atomes d'azote du pyclène (N-6 et N-9) afin de pouvoir intervenir sélectivement sur le troisième atome (N-3) laissé libre. Après fonctionnalisation de l'azote en position -3, la déprotection du groupe oxalamide conduit à un pyclène substitué en position -3 de manière contrôlée, selon le schéma 2 suivant :

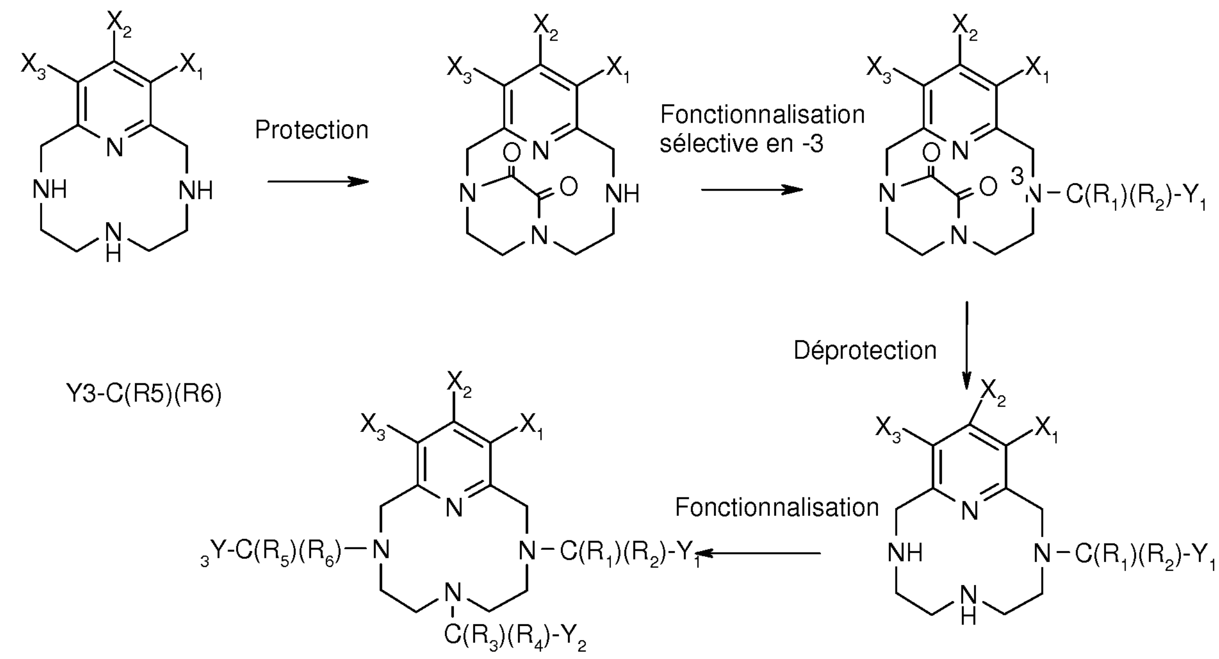


*Schéma 2*

Dans le schéma 2, $X_1$, $X_2$, $X_3$, $R_1$ à $R_6$ et $Y_1$ à $Y_3$ sont tels que définis pour les composés de formule générale (I). Selon un mode de réalisation, l'étape de protection est réalisée en présence de méthanol.

L'invention concerne un procédé de préparation des composés de formule générale (I) pour lesquels les groupements $-C(R_1)(R_2)-Y_1$ et $-C(R_5)(R_6)-Y_3$ sont différents, comprenant une étape de fonctionnalisation d'un composé de formule générale (IX) suivante :

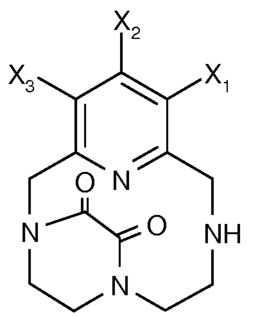

,

pour former un composé de formule générale (X) suivante :

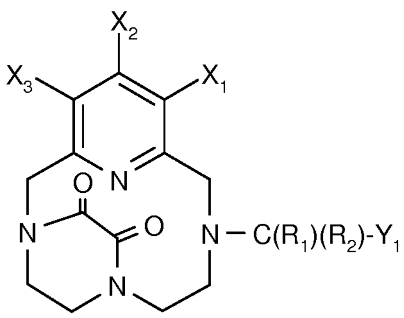

(X),

dans laquelle $X_1$, $X_2$, $X_3$, $R_1$, $R_2$ et $Y_1$ sont tels que définis pour les composés de formule générale (I).

Selon un mode de réalisation particulier, ledit procédé de préparation comprend en outre :

- une étape de déprotection du composé de formule générale (X), pour obtenir un composé de formule générale (XI) suivante :

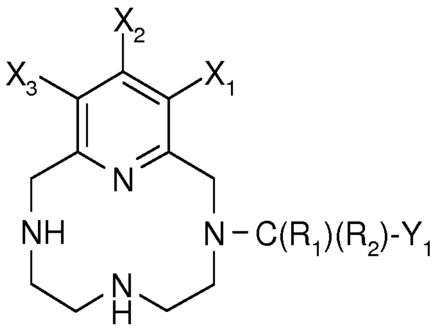

,

dans laquelle $X_1$, $X_2$, $X_3$, $R_1$, $R_2$ et $Y_1$ sont tels que définis pour les composés de formule générale (I) et

- une étape de fonctionnalisation du composé de formule générale (XI), pour obtenir un composé de formule générale (I) tel que défini ci-dessus.

Par fonctionnalisation, on entend l'ajout sur un atome d'azote d'un groupement-$C(R_1)(R_2)$-$Y_1$, -$C(R_3)(R_4)$-$Y_2$ ou -$C(R_5)(R_6)$-$Y_3$.

L'invention concerne également un composé de formule générale (X) suivante :

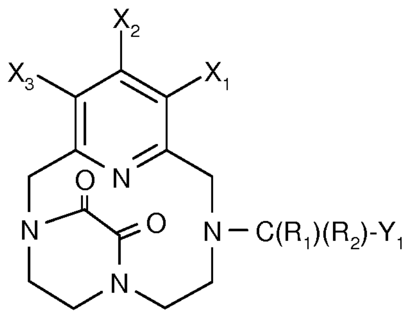

(X),

dans laquelle $X_1$, $X_2$, $X_3$ $R_1$, $R_2$ et $Y_1$ sont tels que définis pour les composés de formule générale (I).

Selon un mode de réalisation particulier du procédé de préparation des composés dissymétriques de formule (I), lorsque l'un au moins des Ri est différent de H, la préparation d'un intermédiaire picolinate substitué est réalisée via un dérivé bromé en position -4, ce qui permet par une réaction de couplage avec un alcyne, catalysée au palladium (réaction dite de Sonogashira, Comprehensive Chirality, Volume 4, Pages18-32, 2012), d'installer le résidu choisi, selon le schéma 3 ci-dessous (exemple avec un alcyne en $C_{12}$) :

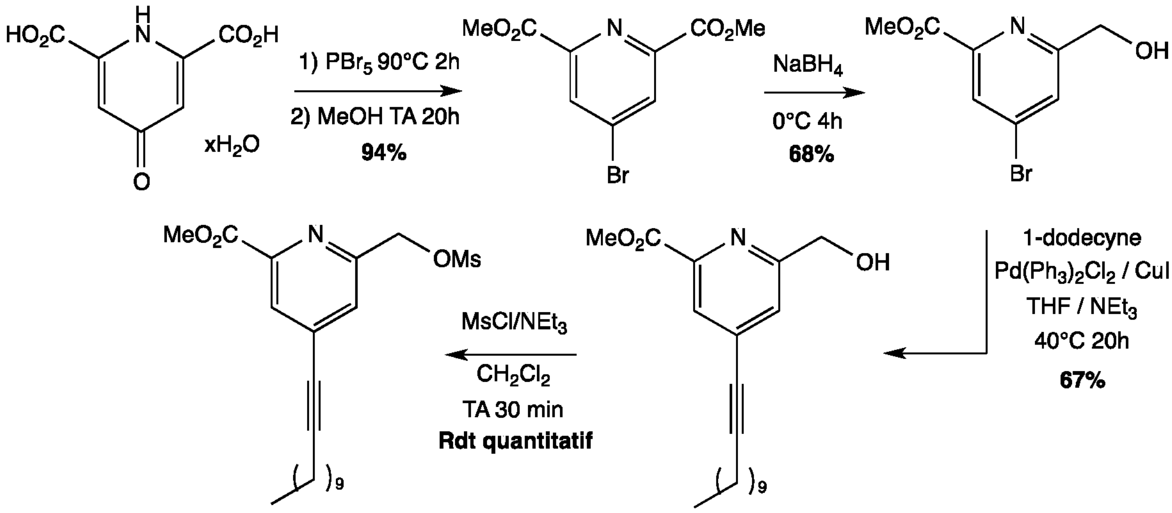


*Schéma 3*

L'invention concerne également un procédé de radiomarquage des composés de formule générale (I). Ledit procédé de radiomarquage étant de préférence réalisé à un pH compris entre 6,5 et 9. Selon un mode de réalisation particulier, ledit radiomarquage est réalisé en présence de tampon acétate. Selon un mode de réalisation, le radiomarquage est réalisé en présence d'eau ou d'alcool tel que l'éthanol, ou leurs mélanges.

Selon un autre mode de réalisation, le radiomarquage est réalisé à une température comprise entre 80°C et 100°C.

**Description des figures**

Figure 1 : Spectres RMN $^1H$ du ligand **P04213** et de son complexe d'yttrium **P04183** (300 MHz, 298 K, $D_2O$)

Figure 2 : Spectres d'absorption des ligands et de leurs complexes d'yttrium enregistrés dans l'eau à pH 3,8 et 5,5 (tampon acétate). La bande d'absorption correspondant aux transitions $\pi$ - $\pi^*$ de la pyridine s'étend de 240 à 300 nm pour les ligands et les complexes.

Figure 3 : Suivi de la variation de l'absorbance au $\lambda$max du complexe ou du ligand à pH 5,5 et 3,8.

Figure 4 : Spectre RMN $^1H$ du ligand P04330.

Figure 5 : Pourcentage d'extraction du complexe P04283 à l'$^{90}Y$ en fonction du temps, en heure (Re-SSS est un complexe de référence).

Figure 6 : Rapport entre la relaxivité des complexes de gadolinium des ligands P04218 et P04216 mesurée à un temps donné et celle à t = 0 min en fonction du temps de présence dans une solution de Zn et phosphates.

Les exemples suivants sont décrits à titre illustratifs de la présente invention.

**EXEMPLES**

Tableau récapitulatif des différentes appellations des composés spécifiques de formule générale (I) selon l'invention :

| | Acronyme | Ligand | Complexe d'Yttrium | Complexe d'$^{90}$Yttrium |
|---|---|---|---|---|
| Mono S | Pc-2a1pa Sym | P04218 | P04219 | |
| Mono AS | Pc-2a1pa Asym | P04216 | P04217 | |

(suite)

| | Acronyme | Ligand | Complexe d'Yttrium | Complexe d'$^{90}$Yttrium |
|---|---|---|---|---|
| Di Sym | Pc-1a2pa sym | P04213 | P04183 | |
| Di AS | Pc-1a2pa Asym | P04214 | P04215 | P04233 |
| Tri | Pc-3pa | P04221 | P04222 | |
| Di AS | Pc-1a2pa Asym C12 | P04245 | | P04283 |
| Di AS | Pc-1a2pa Asym C8 | P04330 | | |

**A- Matériels et méthodes**

Le chlorure d'yttrium-90 est acheté chez PerkinElmer Life Sciences. Les activités mises en jeu étaient comprises entre 28 $\mu$Ci et 8,51 mCi (1,04 - 314,87 MBq). Les produits (solvants HPLC, tampons,...) sont utilisés tels quels, sans purification supplémentaire. Excepté si précisé autrement, le ligand est solubilisé dans l'éthanol.

Les expériences ont été réalisées dans des flacons en verre borosilicaté sertis. Les flacons ont été chauffés dans un bloc chauffant Bioblock permettant de chauffer jusqu'à 6 flacons. Lorsqu'une agitation était nécessaire, un vortex Lab Dancer S40 (VWR) était utilisé. Les centrifugations ont été réalisées avec une centrifugeuse MF 20-R (Awel). Les activités étaient mesurées dans un activimètre CRC-127R (Capintec), dont la calibration était réalisée chaque matin.

Les contrôles qualité ont été réalisés par CCM sur papier Whatman 1, avec comme éluant un mélange MeOH/$NEt_3$ 0.1 %. Les Puretés Radio-Chimiques sont déterminées à l'aide d'un phosphoimageur Cyclone (Perkin Elmer), à l'aide du logiciel Optiquant.

Des analyses HPLC ont également été réalisées, sur une chaîne HPLC Dionex Ultimate 3000 équipée d'un détecteur à barrette de diodes et d'un détecteur radiochromatographique fLumo (Berthold), pilotée par le logiciel Chromeleon.

Les analyses ont été réalisées sur colonne Accucore C18 100 x 3 mm, 2,6 $\mu$ avec le programme suivant : 0,4 mL/min ; A= $_{H2O}$; B= ACN ; 0-3 min : 100 % A; 3-20 min : 0-90 % B ; 20-25 min : 10 % A / 90 % B ; 25-26 min : 90-0 % B ; 26-30 min : 100 % A.

<u>Etudes spectroscopiques</u>

Les spectres UV-Visible des ligands et des complexes d'Yttrium (III) ont été mesurés en solution aqueuse de tampon acétate (pH = 5,5 ou 3,8 sans contrôle de la force ionique) à 298 K à l'aide d'un spectrophotomètre Jasco V-650.

Les expériences RMN (COSY, HMBC and HMQC) ont été enregistrées pour les ligands et leurs complexes avec un spectromètre Bruker Advance 500 (500 MHz) dans $D_2O$.

<u>Etudes cinétiques</u>

La formation des complexes d'Yttrium (III) de do2pa sym, do2pa asym et do1pa sym a été étudiée dans une solution aqueuse de tampon acétate (C = 0,150 M) à 25°C sous des conditions de pseudo ordre 1. L'augmentation de l'intensité de la bande d'absorption dans la région UV a été suivie à pH = 3,8 et 5,5 avec $C_L = C_M = 4 \times 10^{-5}$ M et sans contrôle de la force ionique.

La dissociation en milieu acide des complexes d'Yttrium (III) a été étudiée dans des conditions de pseudo ordre 1 sans contrôle de la force ionique et par addition de solutions aqueuses de HCl (0,5, 1, 2, 4 et 5 M) à une solution de complexe (C = $4.10^{-5}$ M)

La dissociation a été suivie par diminution de l'intensité de la bande d'absorption du complexe ou augmentation de la bande d'absorption du ligand dans la région UV. $t_{1/2}$ a été calculé en ajustant la courbe $A_{max}$= f (t) ($A_{max}$ = absorbance à $\lambda_{max}$ du complexe ou du ligand) avec l'équation exponentionnelle de pseudo ordre 1 suivante : Abs(t) = Abs(eq)+(Abs(0) - Abs(eq)) x exp (-x/t1).

<u>Etudes potentiométriques</u>

*Equipement :* Les expériences ont été réalisées sous atmosphère inerte en solutions aqueuses thermostatées à 25.0 $\pm$ 0.1 °C. Les titrages de protonation et de complexation ont été réalisés dans une cellule de titrage en verre à double paroi en utilisant une burette automatique Metrohm 702 SM Titrino connectée à une électrode combinée en verre Metrohm 6.0233.100. Les titrages ont été contrôlés automatiquement par un logiciel après sélection des paramètres

appropriés évitant la surveillance lors des mesures longues.

Le titrant est une solution de KOH à ca. 0,1 M préparée à partir d'une ampoule commerciale de qualité analytique et sa concentration exacte est obtenue par application de la méthode de Gran par titrage avec une solution standard de $HNO_3$.

Les solutions de ligand ont été préparées à ca. $2.0 \times 10^{-3}$ M et les solutions de $Cu^{2+}$, $Pb^{2+}$ et $Y^{3+}$ à ca. 0,04 M à partir des sels de chlorure de qualité analytique et standardisées par titrage complexométrique avec $H_4$edta (acide éthylènediaminetétraacétique).[1] La solution à titrer contient approximativement 0,05 mmol de ligand dans un volume de 30,00 mL dont la force ionique a été maintenue à 0,10 M en utilisant $KNO_3$ comme électrolyte. 1,2 équivalents de cation métallique ($Cu^{2+}$ou $Pb^{2+}$) ont été ajoutés au ligand (0,05 mmol) lors des titrages de standardisation de la solution de ligand.

0,9 équivalents de cation métallique ($Y^{3+}$) ont été ajoutés au ligand lors des méthodes de titrages de complexation.

*Mesures.* La force électromotrice de la solution a été mesurée après calibration de l'électrode par titrage d'une solution standard de $HNO_3$ à $2.10^{-3}$ M. La $[H^+]$ des solutions a été déterminée par mesure de la force électromotrice de la cellule, $E = E°' + Q\log[H^+] + Ej$. Le terme pH est définit par $-\log [H^+]$. E°' et Q sont déterminés par la région acide des courbes de calibration. Le potentiel de jonction liquide, Ej, est négligeable dans les conditions expérimentales utilisées. La valeur de $K_e = [H^+][OH^-]$ est de $10^{-13.78}$.

*Calculs.* Les données potentiométriques ont été affinées avec le logiciel Hyperquad[2] et les diagrammes de spéciations ont été tracés à l'aide du logiciel HySS.[3]

Les constantes d'équilibre global $\beta_l H$ et $\beta M_mH_hL_l$ sont définit par $\beta M_mH_hL_l = [M_mH_hL_l] / [M]_m[H]_h[L]_l$ ($\beta_l H = [H_hL_l] /[H]_h[L]_l$ et $\beta MH_{-1}L = \beta ML(OH) \times Ke$). Les différences, en unité de log, entre les valeurs de protonation (ou hydrolyse) et les constantes de non-protonation donnent les constantes de réaction intermédiaires (log K) (avec $KM_mH_hL_l = [M_mH_hL_l] / [M_mH_{h-1}L_l][H]$). Les erreurs indiquées sont les écarts-types calculés par le programme d'ajustement à partir de l'ensemble des données expérimentales pour chaque système.

## B- Synthèse des composés de formule générale (I)

Tous les réactifs commerciaux ont été utilisés tels que reçus des fournisseurs sauf indication contraire. Les solvants ont été distillés avant utilisation d'après les procédures décrites dans la littérature. Les purifications par HPLC semi-prep (High Performance Liquid Chromatography) ont été réalisées avec un appareil Prominence Shimadzu HPLC/LCMS-2020 équipé d'un détecteur UV SPD-20 A. Le système chromatographique HPLC utilise une colonne (VisionHT C18 HL 5μ 250 × 10 mm) éluée avec un gradient isocratique $H_2O$ (avec 0.1 % TFA ou HCl)-MeCN

Les spectres RMN $^1H$ et $^{13}C$ NMR ont été enregistrés sur un spectromètre Bruker AMX3-300 MHz opérant à 300.17 et 75.47 MHz, respectivement pour $^1H$ et $^{13}C$. Toutes les mesures ont été réalisées à 25°C. Les signaux sont indiqués comme tels : δ déplacement chimique (ppm), multiplicité (s, singulet; d, doublet; t, triplet; m, multiplet, q, quadruplet), intégration, constantes de couplage J en hertz (Hz).

La spectrométrie de masse haute résolution (HRMS-ESI) a été réalisée en mode ionisation positive par électrospray (ESI+) par le service Spectrométrie de Masse de l'Institut de Chimie Organique et Analytique (ICOA), Orléans, France.

### 1) Synthèse des ligands Pc1a2pa sym P04213 de formule (I) par la voie « directe »

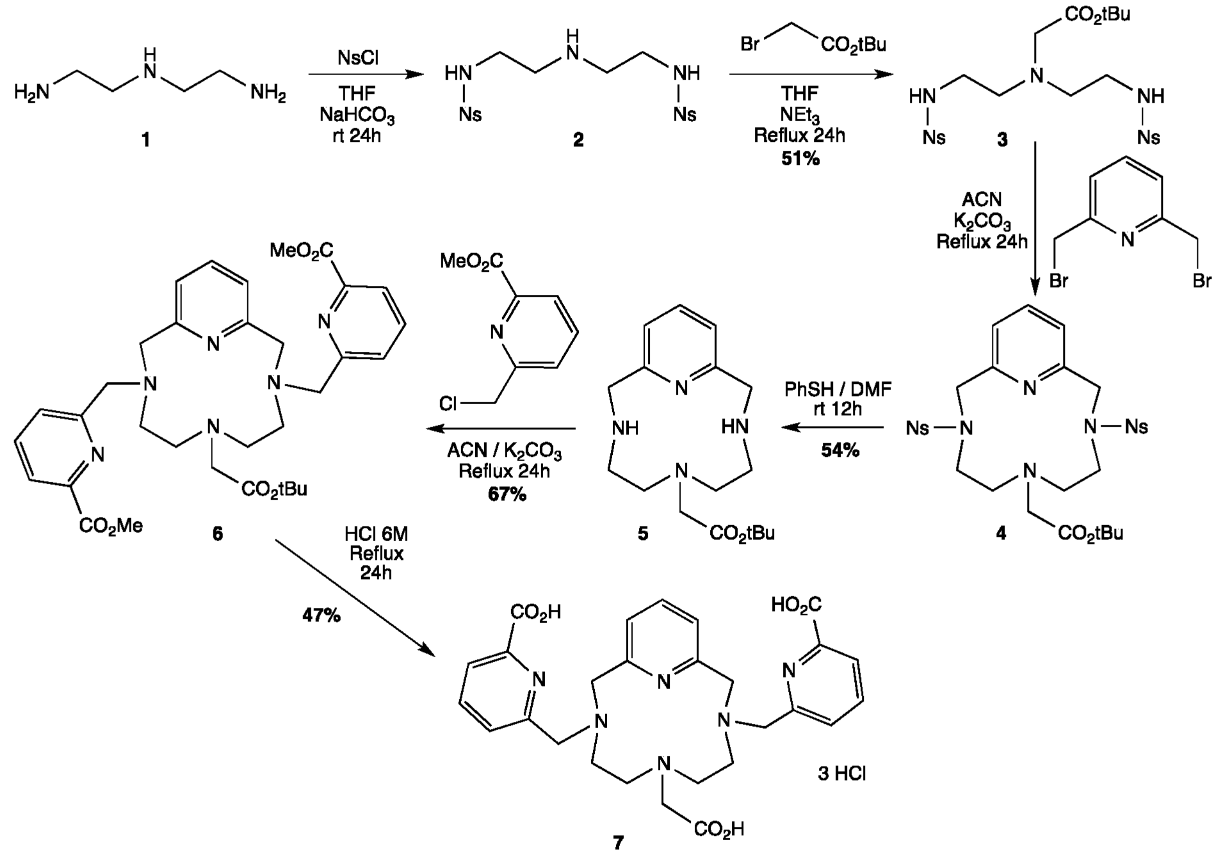


*Schéma de synthèse de l'exemple 1*

**Exemple 1-int.2**

Une solution de chlorure de l'acide 2-nitrobenzène sulfonyle (4.3 g, 19.4 mmol) dans le THF fraîchement distillé est ajoutée à 0°c à un mélange de diéthylènetriamine (1.0g, 9.69mmol) et de $NaHCO_3$ (3.26g, 38.8 mmol) dans le THF (200ml). Le milieu est agité à température ambiante durant 20h puis le solide est filtré. Le filtrat est concentré à sec pour obtenir un solide blanc. Ce composé est utilisé dans la réaction suivante sans purification.
$^1$H RMN (300 MHz, DMSO-$d_6$) : $\delta$8.03-7.82 (m, 8H), 2.87 (t, 4H, $^3J$= 6.0 Hz), 2.47 (t, 4H, $^3J$= 6.0 Hz).
$^{13}$C RMN (75.47 MHz, DMSO-$d_6$) : $\delta$ 147.73, 133.99, 132.68, 132.60, 129.49, 124.39, 47.80, 42.65.

**Exemple 1-int.3**

Une solution de *tert*-butylbromoacétate (6.09 g, 31.2 mmol) dans le THF (50ml) est ajoutée à une solution du composé précédemment préparé (4.93g, 10.4 mmol) et de triéthyle amine (6.31g, 62.4 mmol) dans le THF (75 ml). Le mélange est porté au reflux durant 24h. Après refroidissement du milieu, 50 ml d'une solution saturée de $NH_4Cl$ sont ajoutés et le solvant est éliminé par évaporation sous pression réduite. La phase aqueuse ainsi obtenue est extraite trois fois avec 50 ml de $CH_2Cl_2$. Les fractions chlorométhyléniques sont réunies et séchées sur $MgSO_4$ puis filtrées. Après évaporation du solvant le solide blanc obtenu est chromatographié sur gel de silice (acétate d'éthyle / Pentane : 5/5 à 8/2) pour conduire à un produit blanc après évaporation du solvant (2.9 g, 51 % calc. au départ de **1**).
$^1$H RMN (300 MHz, $CDCl_3$) : $\delta$8.09 (m, 2H), 7.82 (m, 2H), 7.72 (m, 4H), 5.94 (t, 2H, $^3J$ = 5.7 Hz), 3.17 (s, 2H), 3.07 (m, 4H), 2.76 (t, 4H, $^3J$ = 5.7 Hz), 1.41 (s, 9H).
$^{13}$C RMN (75.47 MHz, $CDCl_3$) : $\delta$170.78, 148.27, 133.71, 133.45, 132.78, 130.97, 125.52, 82.13, 55.99, 54.65, 41.90, 28.16.

**Exemple 1-int.4**

A une solution dans l'acétonitrile (20ml) du composé précédemment préparé (2.86 g, 4.87 mmol) sont ajoutés 4g de $K_2CO_3$ et le mélange est porté à reflux. La dibromomethyl pyridine (1.55 g, 5.84 mmol) en solution dans 10 ml

d'acétonitrile est alors ajoutée. Le milieu est chauffé au reflux une nuit et le solide est filtré après refroidissement. Le solvant est évaporé sous pression réduite. Le composé obtenu est engagé dans la réaction suivante sans purification supplémentaire.
$^1$H RMN (300 MHz, $CDCl_3$) : $\delta$8.1-7.6 (m, 9H), 7.42 (d, 2H, $^3J$ = 7.8 Hz), 4.56 (s, 4H), 3.30 (m, 4H), 3.17 (s, 2H), 2.57 (m, 4H), 1.37 (s, 9H).
$^{13}$C RMN (75.47 MHz, $CDCl_3$) : $\delta$171.09, 154.75, 148.33, 139.17, 133.81, 132.74, 131.91, 130.86, 124.39, 124.27, 81.25, 57.51, 54.33, 51.18, 44.93, 28.18.

**Exemple 1-int.5**

Le composé précédemment préparé (2.9 g, 4.29 mmol) est dissous dans le DMF en présence de $Na_2CO_3$ (3.64 g, 34.3 mmol). Le thiophénol (1.88 g, 17.2 mmol) est ensuite ajouté et le milieu est agité à température ambiante durant une nuit. Après évaporation du solvant, le résidu est repris au $CH_2Cl_2$ (100ml) et la solution obtenue est lavée avec 3 x 40 ml d'une solution de NaOH 0.5M. Après séchage sur $MgSO_4$ et filtration, la solution organique est concentrée et le produit obtenu est chromatographié sur alumine neutre ($CH_2Cl_2$/MeOH : 99/1) pour donner un solide blanc (0.835 g, 54 % calc. au départ de 3).
$^1$H RMN (300 MHz, $CDCl_3$) : $\delta$7.4 (t, 1H, $^3J$ = 7.5 Hz), 6.86 (d, 2H, $^3J$ = 7.5 Hz), 3.83 (s, 4H), 3.22 (s, 2H), 2.48 (m, 4H), 2.40 (m, 4H), 1.28 (s, 9H).
$^{13}$C RMN (75.47 MHz, $CDCl_3$) : $\delta$ 171.06, 157.49, 136.65, 120.03, 80.98, 59.10, 56.00, 52.67, 47.41, 27.95.

**Exemple 1-int.6**

L'ester méthylique de l'acide chlorométhyle-6 pyridine-2 carboxylique (0.872 g, 4.70 mmol) est ajouté à une solution du composé précédemment préparé (0.835 g, 2.61 mmol) dans l'acétonitrile (35 ml) en présence de $K_2CO_3$ (1.4 g, 10.4 mmol). Le milieu est porté à reflux durant une nuit puis filtré et concentré. Le résidu est purifié par chromatographie sur alumine neutre ($CH_2Cl_2$/MeOH : 98/2) pour donner 1.09g d'une huile jaune (67%).
$^{13}$C RMN (75.47 MHz, $CDCl_3$) : $\delta$169.95, 164.98, 158.48, 145.96, 138.30, 137.54, 126.69, 123.11, 119.93, 81.07, 62.45, 61.81, 54.44, 53.13, 52.18, 51.19, 27.51.

**Exemple 1-int.7**

Le composé précédemment préparé (1.09 g, 1.76 mmol) est dissous dans une solution d'acide chlorhydrique 6M et le milieu est porté à reflux une nuit. Après concentration le produit est purifié par HPLC sur phase C18 ($H_2O$/acétonitrile 100/0 à 10/90) pour conduire à l'intermédiaire 7 sous forme de chlorhydrate (0.690 g, 57 % calc pour 3 HCl).
$^1$H RMN (500.25 MHz, $D_2O$): $\delta$8.21 (t, 2H, $^3J$ = 7.8 Hz), 8.07 (d, 2H, $^3J$ = 7.8 Hz), 7.88 (d, 2H, $^3J$ = 7.8 Hz), 7.68 (t, 1H, $^3J$ = 7.8 Hz), 7.07 (d, 2H, $^3J$ = 7.8 Hz), 4.63 (s, 4H), 4.45 (s, br, 4H), 3.78 (s, 2H), 3.58 (m, 4H), 3.46 (s, br, 4H).
$^{13}$C RMN (125.79 MHz, $D_2O$) : $\delta$175.11, 170.44, 157.33, 153.78, 152.13, 145.76, 142.12, 131.02, 127.79, 124.63, 62.55, 60.72, 57.63, 56.20, 54.67.

**2) Synthèse Pc2a1 pa sym P04218 de formule (I) par la voie « directe »**

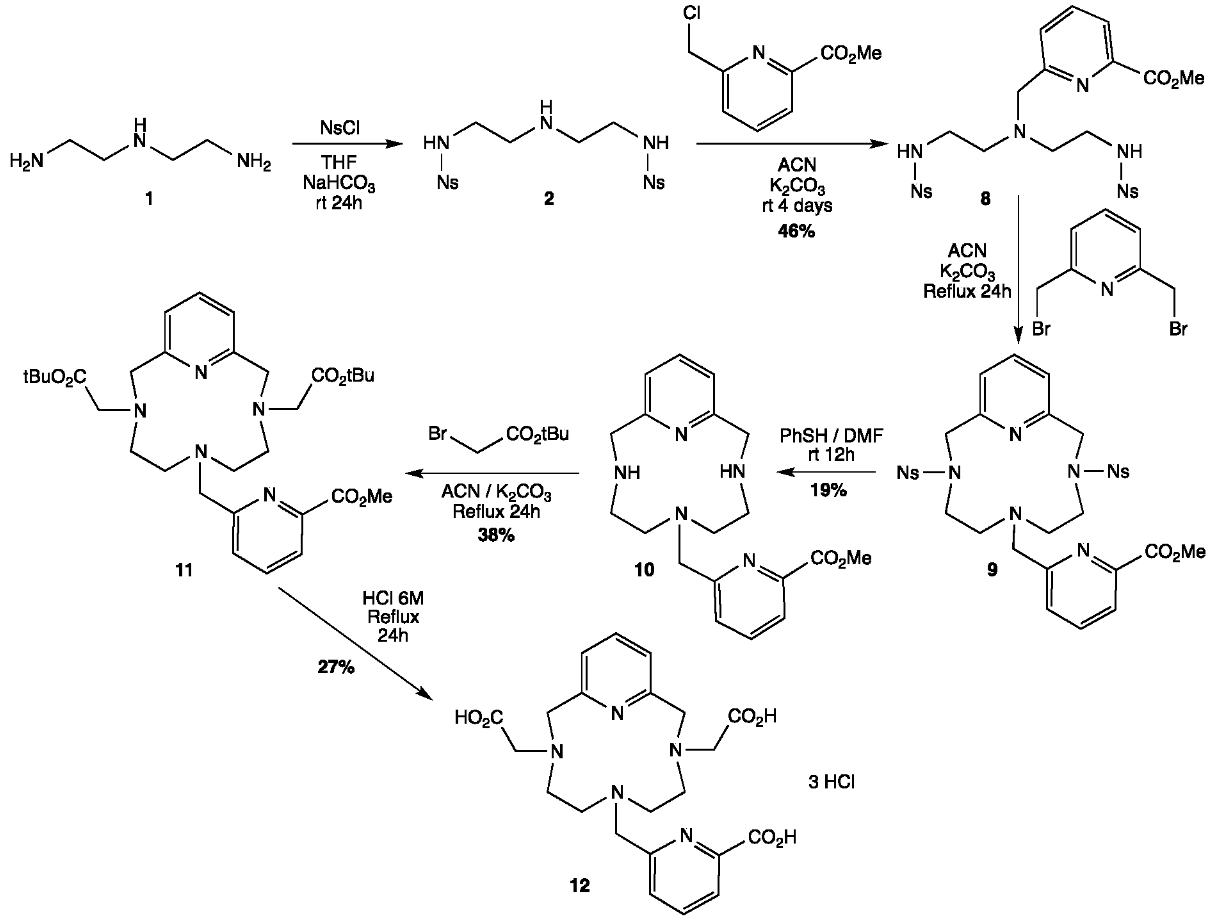


*Schéma de synthèse de l'exemple 2*

**Exemple 2-int.8**

L'ester méthylique de l'acide chlorométhyle-6 pyridine-2 carboxylique (1.93 g, 10.42 mmol) est ajouté à une solution du composé 2 (Exemple 1-int.2) (4.935 g, 10.42 mmol) dans l'acétonitrile (60 ml) en présence de $K_2CO_3$ (4.3 g, 31.26 mmol) et le mélange est agité à température ambiante durant 4 jours. Le solvant est évaporé et le résidu repris au $CH_2Cl_2$, filtré, concentré et purifié par chromatographie sur gel de silice (acétate d'éthyle / pentane : 5/5 to 8/2). Le produit est récupéré sous forme d'une huile jaune (2.78 g, 46 % calc. au départ de 1).
$^1$H NMR (300 MHz, $CDCl_3$) : $\delta$8.08-7.99 (m, 2H), 7.95 (d, 1H, $^3J$ = 7.9 Hz), 7.82-7.61 (m, 7H), 7.47 (d, 1H, $^3J$ = 7.9 Hz), 6.21 (m, 2H), 3.94 (s, 3H), 3.78 (s, 2H), 3.10 (m, 4H), 2.68 (t, 4H, $^3J$ = 5.5 Hz).
$^{13}$C NMR (75.47 MHz, $CDCl_3$) : $\delta$165.64, 159.19, 148.11, 147.80, 138.01, 133.58, 132.71, 130.71, 126.16, 125.18, 123.99, 59.45, 54.83, 53.03, 41.58.

**Exemple 2-int.9**

Le composé précédemment préparé (2.78 g, 4.46 mmol) et 3.7g de $K_2CO_3$ dans 30 ml d'acétonitrile sont portés à reflux puis une solution de dibromométhyle pyridine (1.42 g, 5.36 mmol) dans 10 ml d'acétonitrile est ajoutée. Le milieu est agité à reflux de l'acétonitrile pendant une nuit puis le solide est filtré et le filtrat est concentré sous pression réduite. Le composé obtenu est utilisé à l'étape suivante sans purification supplémentaire.
$^1$H NMR (300 MHz, $CDCl_3$) : $\delta$7.88-7.50 (m, 12H), 7.35 (d, 2H, $^3J$ = 7.5 Hz), 4.52 (s, 4H), 3.89 (s, 3H), 3.77 (s, 2H), 3.27 (m, 4H), 2.52 (m, 4H).
$^{13}$C NMR (75.47 MHz, $CDCl_3$) : $\delta$165.23, 159.52, 153.98, 147.55, 146.63, 138.66, 137.10, 133.39, 131.81, 131.45, 130.08, 125.18, 123.78, 123.57, 123.25, 120.99, 120.56, 59.82, 53.62, 52.33, 48.61, 43.35.

**Exemple 2-int.10**

Le composé précédemment préparé (3.9 g, 5.5 mmol) est dissous dans le DMF en présence de $Na_2CO_3$ (4.6

g, 43.9 mmol) puis le thiophénol (2.42 g, 21.9 mmol) est ajouté et le milieu est agité à température ambiante 1 nuit. Le solvant est ensuite éliminé par distillation sous pression réduite et le résidu repris avec 100 ml de $CH_2Cl_2$. Après trois lavages (3x 40 ml) de la phase organique avec une solution de soude 0.5M, séchage sur $MgSO_4$ et évaporation du solvant, le résidu obtenu est purifié par chromatographie sur alumine neutre ($CH_2Cl_2$/MeOH : 99/1) huile jaune (0.297 g, 19 % calc. au départ de 8).

$^1$H NMR (300 MHz, $CDCl_3$) : $\delta$7.93 (d, 1H, $^3J$ = 7.5 Hz), 7.76 (t, 1H, $^3J$ = 7.5 Hz), 7.61 (t, 1H, $^3J$ = 7.5 Hz), 7.40 (d, 1H, $^3J$ = 7.5 Hz), 7.07 (d, 2H, $^3J$ = 7.5 Hz), 4.13 (s, 4H), 4.00 (s, 2H), 3.79 (s, 3H), 2.73 (m, 8H).

$^{13}$C NMR (75.47 MHz, $CDCl_3$) : $\delta$165.32, 159.60, 155.26, 147.50, 137.96, 137.52, 125.85, 123.96, 120.60, 61.47, 55.96, 52.67, 51.96, 47.09.

**Exemple 2-int.11**

A un mélange de *tert*-butylbromoacétate (0.294 g, 1.50 mmol) et de $K_2CO_3$ (0.464 g, 3.4 mmol).dans 10 ml d'acétonitrile est ajouté le composé précédemment préparé (0.297 g, 0.84 mmol). Le milieu est porté à reflux durant une nuit puis le solide est filtré et la solution obtenue est concentrée. Le résidu obtenu est purifié par chromatographie sur alumine neutre ($CH_2Cl_2$/MeOH : 100/0 à 95/5) pour conduire au composé 11 sous forme d'une huile jaune (0.185 g, 38 %).

**Exemple 2-int.12**

Le composé précédemment préparé (0.185 g, 0.32 mmol) est dissous dans 20 ml d'HCl 6M et le milieu est porté à reflux une nuit. Après évaporation du solvant, le produit est purifié par HPLC sur phase C18 ($H_2O$/acétonitrile 100/0 à 10/90) pour conduire au produit attendu sous forme de chlorhydrate (0.050 g, 27% calc. pour 3 HCl).

$^1$H NMR (500.25 MHz, $D_2O$): $\delta$ 8.33 (t, 1H, $^3J$ = 7.8 Hz), 8.25 (d, 1H, $^3J$ = 7.8 Hz), 8.07 (d, 1H, $^3J$ = 7.8 Hz), 8.00 (t, 1H, $^3J$ = 7.8 Hz), 7.49 (d, 2H, $^3J$ = 7.8 Hz), 4.81 (s, 4H), 4.20 (s, 2H), 3.76 (s, 4H), 3.63 (m, 4H), 2.99 (s, br, 4H).

$^{13}$C NMR (125.79 MHz, $D_2O$): $\delta$172.17, 168.64, 157.78, 152.89, 150.25, 146.36, 142.80, 131.30,

**3) Synthèse d'un composé intermédiaire de préparation**

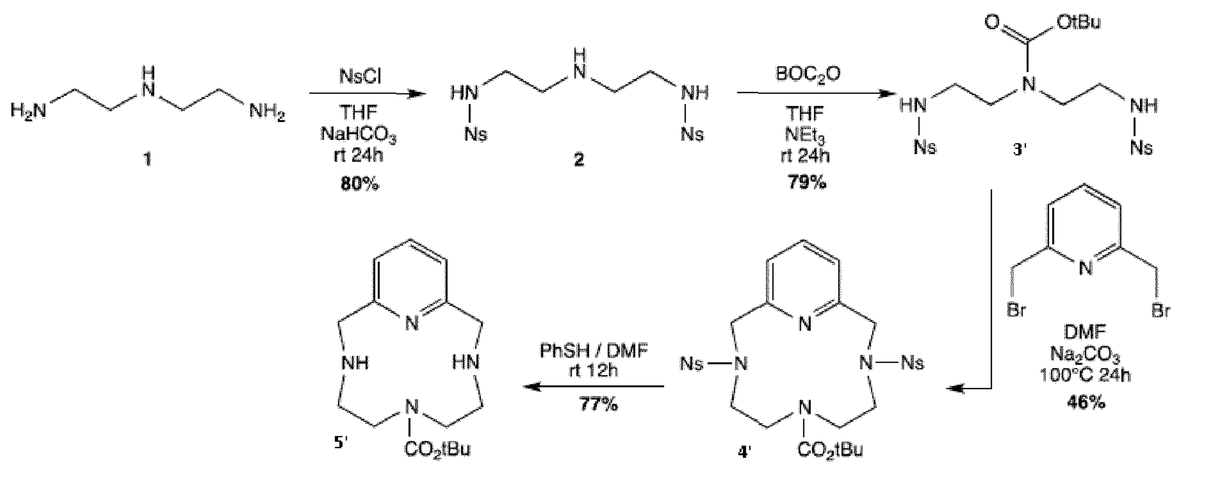


*Schéma de synthèse de l'exemple 3*

**Exemple 3-int.3'**

A une solution du composé 2 (Exemple 1-int.2) et de triéthyle amine (3.9 g, 38.8 mmol) dans le THF fraîchement distillé (150 ml) est ajouté, à 0°c une solution de di-tert-butyl dicarbonate (5.07 g, 23.3 mmol) dans le THF fraîchement distillé (50 ml). Le milieu est agité à température ambiante durant 24h puis traité avec une solution saturée de $NH_4Cl$. Le solvant est évaporé sous pression réduite et la phase aqueuse lavée au dichlorométhane (3 x 80 ml). Après séchage sur $MgSO_4$ la solution organique est filtrée et concentrée sous vide. Le résidu est chromatographié sur gel de silice (acétate d'éthyle/heptane : 3/7 à 7/3) pour conduire au composé attendu sous forme d'une huile jaune (7.0 g, 79 %).

$^1H$ RMN (300 MHz, $CDCl_3$) : $\delta$8.05-7.63 (m, 2H), 7.79-7.63 (m, 6H), 5.99 (s, br, 1H), 5.77 (s, br, 1H), 3.3 (m, 4H), 3.19 (m, 4H), 1.37 (s, 9H).
$^{13}C$ RMN (75.47 MHz, $CDCl_3$) : $\delta$155.78, 147.75, 133.77, 133.09, 132.95, 130.71, 125.20, 80.85, 42.34, 28.16.

**Exemple 3-int.4'**

A un mélange composé du produit précédemment préparé (7.0g, 12.2 mmol), de $Na_2CO_3$ et de DMF (200 ml) chauffé à 100°c est ajouté sous atmosphère d'azote une solution de 2,6-bis(bromométhyl)pyridine (3.23 g, 12.2 mmol) dans 100ml de DMF sec. Le milieu est agité à 100°c durant 24h puis refroidi. Le solvant est évaporé sous pression réduite et le résidu ainsi obtenu est repris avec du $CH_2Cl_2$. La phase organique est lavée avec une solution de NaOH 1M et séchée avec $MgSO_4$. Après filtration et concentration, le produit est précipité dans l'acétone pour conduire à un solide blanc (3.76 g, 46 %).
$^1H$ RMN (300 MHz, DMSO-$d_6$) : $\delta$8.10-7.80 (m, 9H), 7.35 (m, 2H), 4.60 (s, 4H), 3.53 (s, 8H), 1.38 (s, 9H).
$^{13}C$ RMN (75.47 MHz, DMSO-$d_6$) : $\delta$ 155.83, 155.75, 154.59, 147.95, 147.89, 138.40, 135.63, 134.57, 132.75, 132.62, 131.17, 130.97, 129.52, 129.19, 124.62, 124.63, 122.49, 122.44, 78.81, 55.17, 50.02, 49.79, 45.42, 44.72, 44.66, 43.09, 27.97.

**Exemple 3-int.5'**

A une suspension de $Na_2CO_3$ dans 250 ml de DMF sont ajoutés le composé précédemment préparé (3.59 g, 5.43 mmol) puis le thiophénol (2.35 g, 21.3 mmol). Le mélange est agité à température ambiante durant 12h puis le solvant est évaporé sous pression réduite et le résidu obtenu repris avec $CH_2Cl_2$. La phase organique est lavée à l'eau et séchée avec $MgSO_4$ puis filtrée et concentrée. Le résidu ainsi obtenu est purifié par chromatographie sur gel de silice (MeOH/ $NH_3$ aq 32% : 100/0 to 95/5) pour donner le composé attendu sous forme d'une huile jaune (1.06 g, 76%).
$^1H$ RMN (300 MHz, $CDCl_3$) : $\delta$7.51 (t, 1H, $^3J$ = 7.5 Hz), 6.94 (d, 2H, $^3J$ = 7.5 Hz), 3.94 (s, 4H), 3.52 (t, 4H, $^3J$ = 5.1 Hz), 3.04 (s, 2H), 2 .61 (t, 4H, $^3J$ = 5.65 Hz), 1.49 (s, 9H).
$^{13}C$ RMN (75.47 MHz, $CDCl_3$) : $\delta$ 158.22, 157.53, 136.65, 120.34, 80.27, 52.06, 50.78, 48.88, 28.59.

**4) Synthèse du ligand Pc2a1pa sym P04218 de formule (I) par la voie « Boc »**

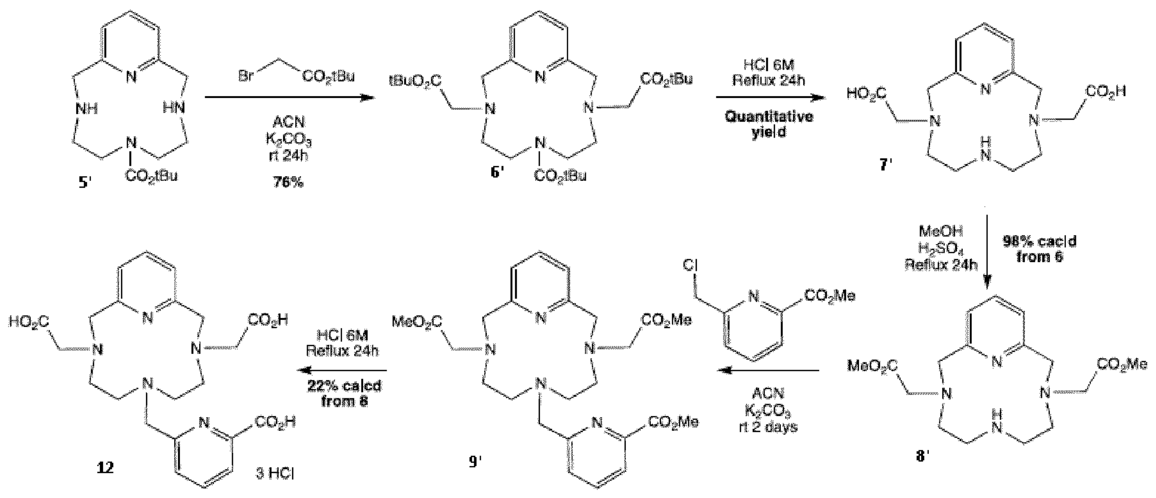


*Schéma de synthèse de l'exemple 4*

**Exemple 4-int.6'**

A un mélange composé du produit précédemment obtenu (Exemple 3-int.5') (0.803 g, 2.62 mmol) et de $K_2CO_3$ dans 150 ml d'acétonitrile est ajouté une solution de *tert*-butylbromoacétate (1.022 g, 5.24 mmol) dans 50 ml d'acétonitrile et le mélange est agité à température ambiante durant 24h. Le solvant est évaporé et le résidu est repris dans le $CH_2Cl_2$ puis la solution obtenue est filtrée et concentrée. Le produit est purifié par chromatographie sur gel de silice ($CH_2Cl_2$/MeOH : 100/0 à 98/2) pour conduire à une huile jaune (1.06 g, 76 %).

$^1H$ RMN (300 MHz, $CDCl_3$) : $\delta$7.5 (t, 1H, $^3J$ = 7.5 Hz), 7.08 (d, 2H, $^3J$ = 7.5 Hz), 3.86 (s, br, 4H), 3.27 (d, 4H, $^3J$ = 9.4 Hz), 3.01 (m, 4H), 2.75-2.55 (m, 4H), 1.34 (s, 18H), 1.24 (s, 9H).
$^{13}C$ RMN (75.47 MHz, $CDCl_3$) : $\delta$170.45, 170.27, 157.44, 156.97, 155.26, 137.18, 122.67, 122.61, 80.75, 78.71, 59.99, 59.60, 59.02, 58.67, 51.77, 51.27, 45.04, 44.81, 28.21, 28.03.

**Exemple 4-int.7'**

Le composé précédemment préparé (1.06 g, 9.5 mmol) est dissous dans 20 ml d'acide chlorhydrique 6M et le mélange est porté à reflux durant 1 nuit. Après refroidissement, le solvant est évaporé et le produit attendu est obtenu sous forme d'un solide brun (100%).
$^1H$ RMN (300 MHz, $D_2O$): $\delta$7.91 (t, 1H, $^3J$ = 7.9 Hz), 7.36 (d, 2H, $^3J$ = 7.9 Hz), 4.20 (s, 4H), 3.65 (s, 4H), 2.96 (m, 4H), 2.78 (m, 4H).
$^{13}C$ RMN (75.47 MHz, $D_2O$) : $\delta$ 175.61, 154.59, 147.87, 127.29, 60.26, 59.52, 54.14, 46.69.

**Exemple 4-int.8'**

A une solution du composé précédemment obtenu dans 30 ml de méthanol est ajouté 5 ml de $H_2SO_4$ concentré puis le mélange est agité et porté à reflux une nuit. Après refroidissement, le solvant est évaporé, repris par 10 ml d'eau et le pH ajusté à 7 par ajout de $K_2CO_3$. L'eau est évaporée et le résidu repris au dichlorométhane. La phase organique est ensuite séchée sur $MgSO_4$, filtrée et concentrée. Le produit attendu est obtenu sous forme d'huile jaune (0.67 g, 98 % calc au départ de **6**').
$^1H$ RMN (300 MHz, $CDCl_3$) : $\delta$7.34 (t, 1H, $^3J$ = 7.5 Hz), 6.84 (d, 2H, $^3J$ = 7.5 Hz), 3.86 (s, 4H), 3.51 (s, 10H), 2.69 (m, 4H), 2.02 (m, 4H).
$^{13}C$ RMN (75.47 MHz, $CDCl_3$) : $\delta$ 172.12, 159.24, 136.56, 120.66, 59.54, 57.73, 52.59, 50.95, 46.99.

**Exemple 4-int.9'**

A un mélange composé du produit précédemment obtenu (0.67 mg, 1.9 mmol) et de $K_2CO_3$ (0.524 g, 3.8 mmol) dans 50 ml d'acétonitrile est ajouté 0.353 g (1.9 mmol) de l'ester méthylique de l'acide chlorométhyle-6 pyridine-2 carboxylique et le milieu est agité deux jours à température ambiante. Le solvant est évaporé et le résidu repris avec $CH_2Cl_2$ puis filtré. La solution obtenue est concentrée et le produit est directement utilisé à l'étape suivante sans purification supplémentaire.

**Exemple 4-int.12**

Au composé précédemment préparé est ajouté 20 ml d'acide chlorhydrique 6M et le mélange est porté au reflux une nuit. Le solvant est évaporé et le résidu obtenu est purifié par HPLC sur phase C18 ($H_2O$ 0.1% HCl /acétonitrile : 100/0 à 10/90) pour conduire au produit attendu sous forme d'une huile incolore (0.237 g, 22% calc. au départ de 8' pour 3 HCl).
$^1H$ RMN (500.25 MHz, $D_2O$): $\delta$ 8.33 (t, 1H, $^3J$ = 7.8 Hz), 8.25 (d, 1H, $^3J$ = 7.8 Hz), 8.07 (d, 1H, $^3J$ = 7.8 Hz), 8.00 (t, 1H, $^3J$ = 7.8 Hz), 7.49 (d, 2H, $^3J$ = 7.8 Hz), 4.81 (s, 4H), 4.20 (s, 2H), 3.76 (s, 4H), 3.63 (m, 4H), 2.99 (s, br, 4H).
$^{13}C$ RMN (125.79 MHz, $D_2O$) : $\delta$172.17, 168.64, 157.78, 152.89, 150.25, 146.36, 142.80, 131.30, 128.33, 125.60, 62.35, 60.08, 59.47, 56.09, 52.88.

**5) Synthèse du ligand Pc1a2pa sym P04213 de formule (I) par la voie « Boc »**

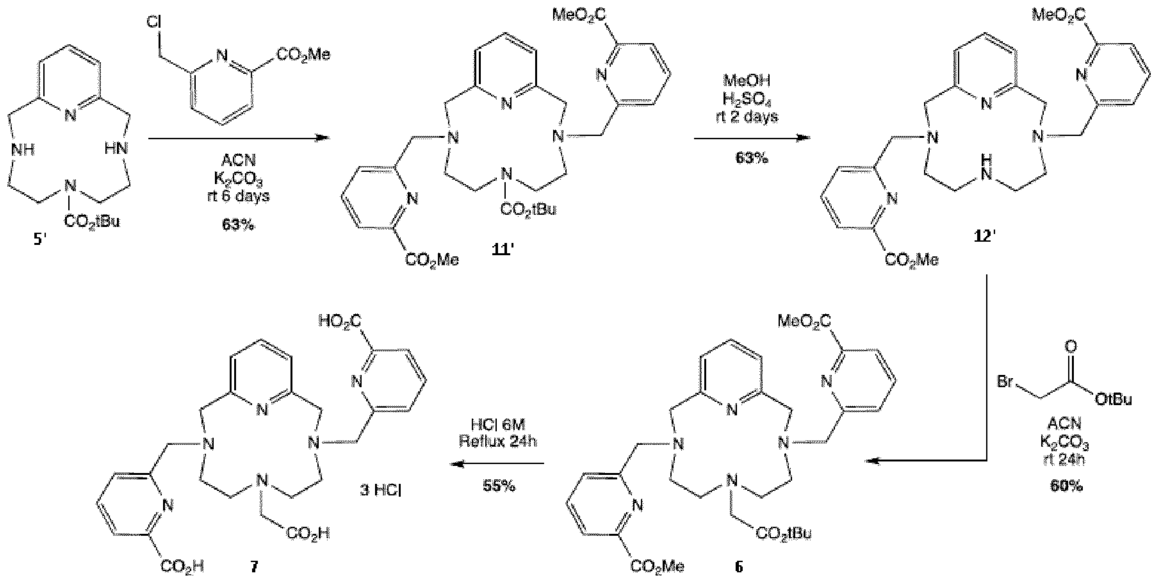

5'
11'
12'
6
7

*Schéma de synthèse de l'exemple 5*

**Exemple 5-int.11'**

A un mélange composé du produit précédemment obtenu (Exemple 3-int.5') (0.326 g, 1.06 mmol) et de $K_2CO_3$ (0.587 g, 4.3 mmol) dans 50 ml d'acétonitrile est ajouté une solution de l'ester méthylique de l'acide chlorométhyle-6 pyridine-2 carboxylique (0.395 g, 2.13 mmol) dans 20 ml d'acétonitrile. Le mélange est agité à température ambiante durant 5 jours et le solvant est évaporé. Le résidu est repris avec du dichlorométhane et la suspension est filtrée. La solution chlorométhylénique est concentrée et le résidu purifié par chromatographie sur alumine neutre ($CH_2Cl_2$/MeOH : 100/0 à 98/2) pour donner une huile jaune (0.407 g, 63 %).

$^1H$ RMN (300 MHz, $CDCl_3$) : $\delta$8.05-7.95 (m, 2H), 7.87-7.73 (m, 4H), 7.66 (t, 1H, $^3J$ = 7.2 Hz), 7.2 (m, 2H), 4.10-3.80 (m, 14H), 3.46-3.31 (m, 4H), 2.75-2.50 (m, 4H), 1.17 (s, 9H).

$^{13}C$ RMN (75.47 MHz, $CDCl_3$) : $\delta$ 165.91, 160.82, 160.70, 156.80, 156.54, 155.48, 147.44, 137.66, 137.55, 137.38, 126.14, 126.07, 123.83, 23.14, 122.96, 79.03, 62.90, 62.71, 59.96, 58.78, 53.00, 51.59, 51.27, 45.14, 44.75, 28.30.

**Exemple 5-int.12'**

A une solution du composé précédemment préparé (0.407 g, 0.67 mmol) dans 20 ml de méthanol est ajouté 1 ml d'acide sulfurique concentré. Le mélange est agité et porté à reflux pendant 2 jours. Après refroidissement le solvant est évaporé, le résidu repris à l'eau (10 ml) et le pH du milieu est ajusté à 7 par ajout de $K_2CO_3$. L'eau est évaporée et le résidu est repris avec du dichlorométhane. La phase organique est séchée sur sulfate de magnésium, filtrée et concentrée. Le produit est purifié par chromatographie sur alumine neutre ($CH_2Cl_2$/MeOH : 100/0 à 98/2) pour donner une huile jaune (0.214 g, 63 %).

$^{13}C$ RMN (75.47 MHz, $CDCl_3$) : $\delta$ 165.60, 159.30, 159.24, 146.64, 137.17, 127.10, 123.58, 119.79, 61.92, 57.51, 52.72, 52.56, 46.12.

**Exemple 5-int.6**

A un mélange du composé précédemment préparé (0.214 g, 0.423 mmol) et de $K_2CO_3$ (0.117 g, 0.85 mmol) dans 20 ml d'acétonitrile est ajouté une solution de *tert*-butylbromoacétate (0.083 g, 0.423 mmol) dans 10 ml d'acétonitrile. Le mélange est agité à Température ambiante durant 24h puis concentré. Le résidu est repris dans $CH_2Cl_2$ et les sels sont filtrés. Après évaporation du solvant, le résidu est purifié par chromatographie sur alumine neutre ($CH_2Cl_2$/MeOH : 100/0 to 98/2) pour donner le produit attendu sous forme d'une huile jaune (0.155 g, 60 %).

**Exemple 5-int.7**

Le composé précédemment obtenu est dissous dans 20 ml d'acide chlorhydrique 6M et le mélange est porté à reflux une nuit. Après évaporation de l'eau, le résidu est purifié par HPLC sur phase C18 ($H_2O$/ACN : 100/0 à 90/10)

pour donner une huile incolore (0.089 g, 55 % calc. pour 3 HCl).
$^{1}$H RMN (500.25 MHz, $D_2O$): $\delta$8.21 (t, 2H, $^{3}J$ = 7.8 Hz), 8.07 (d, 2H, $^{3}J$ = 7.8 Hz), 7.88 (d, 2H, $^{3}J$ = 7.8 Hz), 7.68 (t, 1H, $^{3}J$ = 7.8 Hz), 7.07 (d, 2H, $^{3}J$ = 7.8 Hz), 4.63 (s, 4H), 4.45 (s, br, 4H), 3.78 (s, 2H), 3.58 (m, 4H), 3.46 (s, br, 4H).
$^{13}$C RMN (125.79 MHz, $D_2O$) : $\delta$175.11, 170.44, 157.33, 153.78, 152.13, 145.76, 142.12, 131.02, 127.79, 124.63, 62.55, 60.72, 57.63, 56.20, 54.67.

Références

1. Schwarzenbach, G.; Flaschka, W. Complexometric Titrations; Methuen & Co.: London, 1969.
2. Gans, P.; Sabatini, A.; Vacca, A. Talanta 1996, 43, 1739-1753.
3. Alderighi, L.; Gans, P.; Ienco, A.; Peters, D.; Sabatini, A.; Vacca, A. Coord. Chem. Rev. 1999, 184,311-318.


**6) Synthèse du ligand Pc1a2pa asym P04214 de formule (I) par la voie « oxalate »**

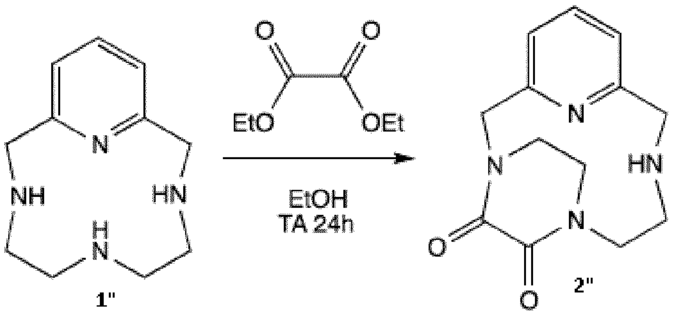


1"

2"

Une solution de diethyloxalate (2.02 g, 13.8 mmol) dans EtOH (100 mL) a été ajoutée à une solution de pyclène (2.37 g, 11.5 mmol) dans EtOH (200 mL). Le mélange a été agité à température ambiante pendant toute la nuit puis concentré. Le résidu obtenu a été purifié par chromatographie sur colonne d'alumine ($CH_2Cl_2$/MeOH 98/2). Le produit final a été obtenu sous forme de solide blanc (0.548 g, 19 %).
$^{1}$H RMN (300 MHz, $CDCl_3$) : $\delta$7.52 (t, 1H, $^{3}J$ = 7.7 Hz), 7.02 (d, 1H, $^{3}J$ = 7.9 Hz), 6.93 (d, 1H, $^{3}J$ = 7.5 Hz), 5.59 (d, 1H, $^{2}J$ = 16.2 Hz), 4.62 (ddd, 1H, $^{2}J$ = 13.9 Hz, $^{3}J$ = 11.1 Hz, $^{3}J$ = 2.5 Hz), 4.08 (d, 1H, $^{2}J$ = 16.6 Hz), 3.95 (d, 1H, $^{2}J$ = 17.3 Hz), 3.77 (ddd, 1H, $^{2}J$ = 13.9 Hz, $^{3}J$ = 10.6 Hz, $^{3}J$ = 4.52 Hz), 3.70 (d, 1H, $^{2}J$ = 17.3 Hz), 3.5 (ddd, 1H, $^{2}J$ = 12.4 Hz, $^{3}J$ = 10.6 Hz, $^{3}J$ = 4.5 Hz), 3.24 (dt, 1H, $^{2}J$ = 13.9 Hz, $^{3}J$ = 4.4 Hz), 3.13 (dt, 1H, $^{2}J$ = 12.4 Hz, $^{3}J$ = 4.1 Hz), 3.01 (dt, 1H, $^{2}J$ = 12.2 Hz, $^{3}J$ = 3.2 Hz), 2.83 (dt, 1H, $^{2}J$ = 13.9 Hz, $^{3}J$ = 3.0 Hz), 2.74 (td, 1H, $^{2}J$ = 11.7 Hz, $^{3}J$ = 2.3 Hz).
$^{13}$C RMN (75.47 MHz, $CDCl_3$) : $\delta$ 162.96, 161.23, 159.10, 153.42, 136.83, 120.58, 119.44, 55.40, 52.53, 47.89, 47.66, 44.61, 44.20

Synthèse de l'intermédiaire Pvclen-Oxalate 2" :

Différents essais ont été réalisés pour obtenir l'intermédiaire « pyclen oxalate » 2", comme indiqué ci-dessous.

Voie 1
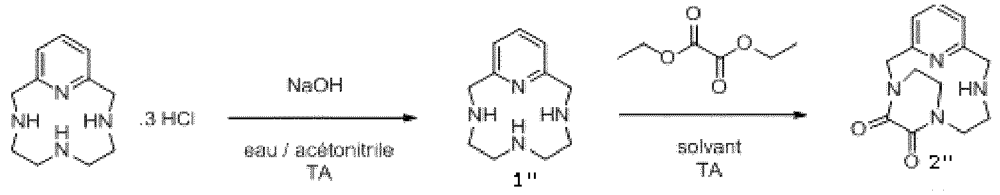


Voie 2
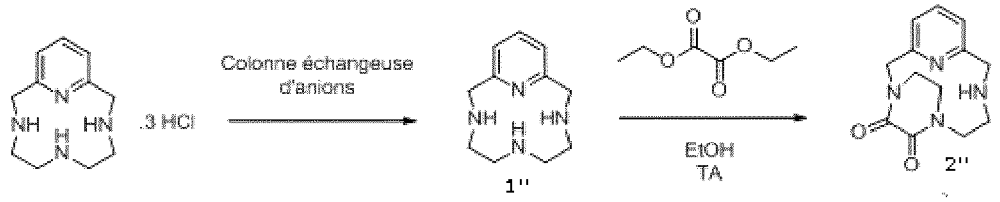


Voie 3
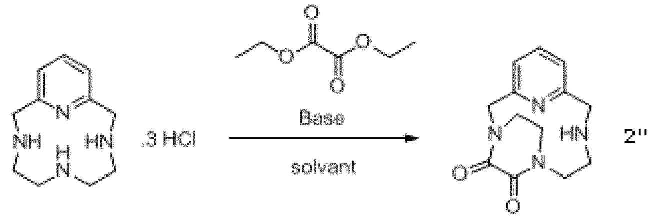


| Essai | Voie utilisée | Conditions | Purification | Rendement |
|---|---|---|---|---|
| **1** | 3 | DIPEA, EtOH, 2 j, TA | précipitation | 22 % |
| **2** | 1 | EtOH, 1,5 j, TA | Chromatographie sur alumine | 33 % |
| **3** | 1 | EtOH, 2 j, TA | Chromatographie sur alumine | 36 % |
| **4** | 2 | EtOH, 41 h, TA | Chromatographie sur alumine | 37 % |
| **5** | 1 | MeOH, 48 h, TA | Précipitation | 54 % |
| **6** | 1 | MeOH, 23 h, TA | Précipitation | **93 %** |

*Résumé des essais sur la synthèse de l'intermédiaire Pyclen-Oxalate 2"*

On constate que l'intermédiaire « Pyclen-oxalate » 2" est obtenu selon les différentes conditions opératoires et voies testées. En particulier, on observe un très bon rendement, supérieur à 90%, en présence de méthanol.

Suite de la synthèse :

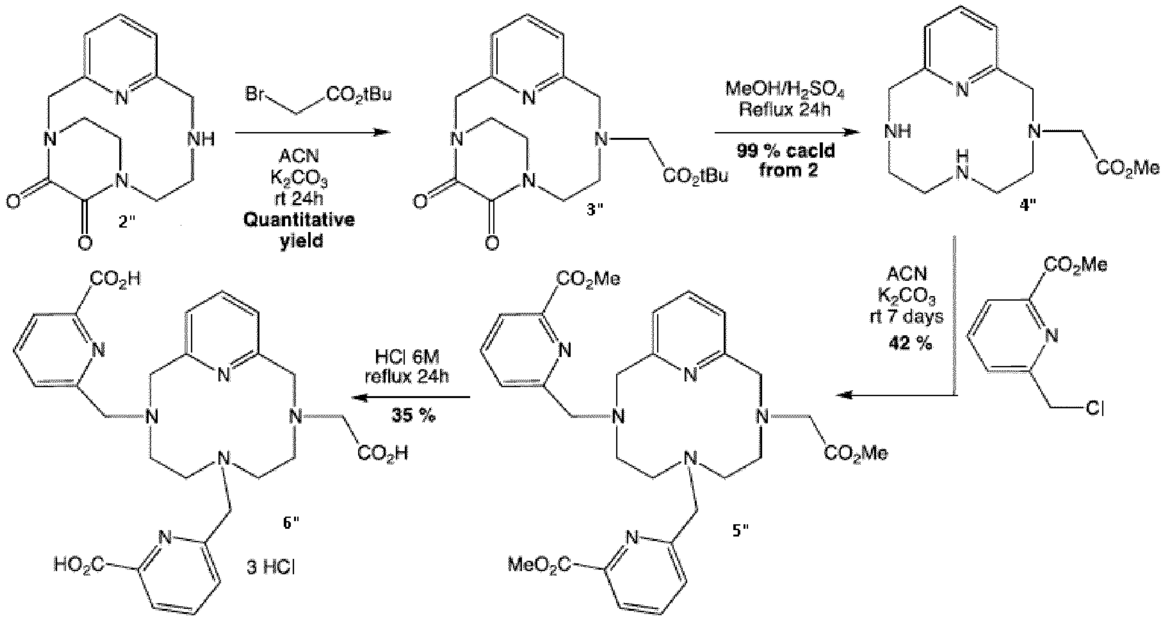


Une solution de *tert*-butylbromoacetate (0.668 g, 3.42 mmol) dans de l'acetonitrile (100 mL) a été ajoutée à une solution de 2" (0.890 g, 3.42 mmol) et $K_2CO_3$ (1.42 g, 10.3 mmol) dans de l'acetonitrile (150 mL). Le mélange a été agité à température ambiante pendant 24h. Le solvant a été évaporé et le résidu a été repris dans du dichlorométhane, puis filtré et concentré. Le produit souhaité a été obtenu sous forme d'huile jaune et utilisé dans les étapes suivantes sans purification supplémentaire (1.25 g).

$^1$H RMN (300 MHz, $CDCl_3$) : $\delta$ 7.28 (t, 1H, $^3J$ = 7.7 Hz), 6.8 (d, 1H, $^3J$ = 7.5 Hz), 6.63 (d, 1H, $^3J$ = 7.5 Hz), 5.26 (d, 1H, $^2J$ = 16.6 Hz), 4.08 (m, 1H), 3.89 (d, 1H, $^2J$ = 16.6 Hz), 3.68 (m, 4H), 3.0 (m, 4H), 2.77 (m, 2H), 2.52 (m, 1H), 1.18 (m, 9H).

$^{13}$C NMR (75.47 MHz, $CDCl_3$) : $\delta$ 170.65, 162.42, 159.73, 158.43, 153.60, 136.48, 119.67, 119.11, 80.37, 61.08, 56.45, 52.59, 52.07, 46.64, 46.07, 44.76, 27.61.

Le composé **3"** a été solubilisé dans MeOH (100 mL) et de l'acide sulfurique concentré a été ajouté lentement (10 mL). Le mélange a été mis sous reflux pendant 24h. Après refroidissement à température ambiante, le solvant a été évaporé. 20 mL d'eau ont été ajoutés et le pH a été ajusté à 7 par $K_2CO_3$. L'eau a été évaporée et le résidu repris dans du dichlorométhane. Du sulfate de Magnesium a été ajouté et la phase organique as été filtrée puis concentrée. Le produit brut a été purifié par chromatographie sur colonne d'alumine ($CH_2Cl_2$/MeOH : 98/2 to 95/5). Le composé 4" est sous forme de solide blanc (0.939 g, 99 % calculé à partir de **2**").

$^1$H RMN (300 MHz, $CDCl_3$) : $\delta$ 7.52 (t, 1H, $^3J$ = 7.5 Hz), 6.96 (m, 2H), 3.96 (d, 4H, $^3J$ = 9.8 Hz), 3.63 (m, 5H), 3.28 (m, 2H), 3.18 (m, 2H), 2.86 (dt, 4H, $^2J$ = 11.3, $^3J$ = 5.7 Hz).

$^{13}$C RMN (75.47 MHz, $CDCl_3$) : $\delta$ 172.17, 161.02, 159.09, 137.57, 120.07, 119.97, 57.69, 57.04, 52.35, 51.72, 51.54, 46.80, 46.29, 46.23.

Le methylester d'acide chloromethylpyridine-2-carboxylique a été ajouté à une solution du composé **4"** (0.939 g, 3.38 mmol) dans l'acétonitrile (150 mL) en présence de $K_2CO_3$ (1.8 g, 13.5 mmol). Le mélange a été agité à température ambiante pendant une semaine, puis filtré et concentré. Le produit brut a été purifié par chromatographie sur colonne d'alumine ($CH_2Cl_2$/MeOH 98/2) permettant d'obtenir le composé **5"** sous forme d'huile de couleur jaune (0.822 g, 42 %).

L'acide chlorhydrique (20 mL, 6 M) a été lentement ajouté au composé 5". Le mélange a été mis sous reflux pendant 24h puis concentré. Le produit brut a été purifié en utilisant une HPLC-C18 ($H_2O$ 0.1% HCl/acétonitrile : 90/10 to 5/95) et le ligand 6" a été obtenu sous forme d'huile incolore (0.310 g, 35 % calculé pour 3 HCl).

$^1$H RMN (500 MHz, $D_2O$) : $\delta$ 7.98-7.87 (m, 5H), 7.65 (d, 1H, $^3J$ = 7.3 Hz), 7.47 (m, 1H), 7.43 (d, 1H, $^3J$ = 7.9 Hz), 7.31 (d, 1H, $^3J$ = 7.9 Hz), 4.78 (s, 2H), 4.74 (s, br, 2H), 4.54 (s, 2H), 4.20 (s, 2H), 3.78 (s, br, 2H), 3.63 (s, br, 2H), 3.55 (s, 2H), 3.12 (m, 4H).

$^{13}$C RMN (125.77 MHz, $D_2O$) : 172.25, 171.82, 170.66, 158.41, 153.72, 153.30, 152.78, 152.14, 151.95, 143.56, 142.61, 142.36, 130.33, 129.50, 127.63, 127.24, 125.33, 125.16, 61.91, 61.78, 61.72, 60.08, 56.17, 56.12, 53.57, 53.37

**7) Synthèse du ligand Pc2a1pa asym P04216 de formule (I) par la voie « oxalate »**

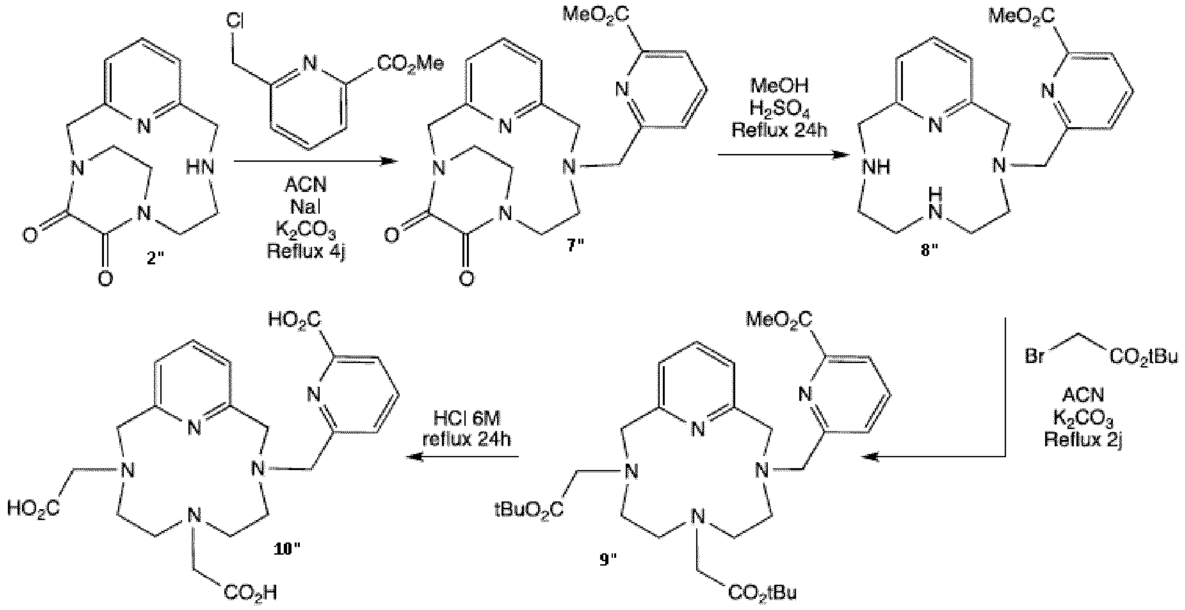


L'ester méthylique de l'acide chlorométhyle-6 pyridine-2 carboxylique (711 mg, 3.85 mmol) a été ajouté à une solution du composé 2" (1.0 g, 3.85 mmol) dans de l'acétonitrile (300 mL) en présence de $K_2CO_3$ (1.5 g, 12 mmol). Le mélange a été mis sous reflux pendant 4 jours puis filtré et concentré. Le produit brut a été purifié par chromatographie sur colonne d'alumine ($CH_2Cl_2$/MeOH 98/2) donnant le composé 7" sous forme d'huile de couleur jaune (1.56 g, 99 %).

Le composé 7" (1.56 g, 3.81 mmol) a été solubilisé dans MeOH (40 mL) et de l'acide sulfurique concentré a été lentement ajouté (1 mL). Le mélange a été mis sous reflux pendant 24h. Après refroidissement à température ambiante, le solvant a été évaporé. 20 mL d'eau ont été ajoutés et le pH a été ajusté à 7 par $K_2CO_3$. L'eau a été évaporée et le résidu repris dans le dichlorométhane. Du sulfate de magnésium a été ajouté et la phase organique a été filtrée puis concentrée. Le produit brut a été purifié par chromatographie sur colonne d'alumine ($CH_2Cl_2$/MeOH : 98/2 to 95/5) donnant **8"** sous forme d'huile de couleur jaune (1.24 g, 92 %).

Une solution de *tert*-butylbromoacétate (1.36 g, 6.98 mmol) dans l'acétonitrile (150 mL) a été ajoutée à une solution de **8"** (1.24 g, 3.49 mmol) et $K_2CO_3$ (1.93 g, 14mmol) dans l'acétonitrile (150 mL). Le mélange a été mis sous reflux pendant deux jours. Le solvant a été évaporé et le résidu repris dans du dichlorométhane filtré et concentré. Le composé **9"** a été obtenu sous forme d'huile de couleur jaune et utilisé dans l'étape suivante sans purification supplémentaire.

De l'acide chlorhydrique (20 mL, 6 M) a été lentement ajouté au composé **9".** Le mélange a été mis sous reflux pendant 24h puis concentré. Le produit brut a été purifié en utilisant une HPLC-C18 ($H_2O$ 0.1% HCl/acétonitrile : 90/10 to 5/95) et le ligand **10"** a été obtenu sous forme d'huile incolore.

**8) Synthèse du ligand Pc3pa P04221 de formule (I) :**

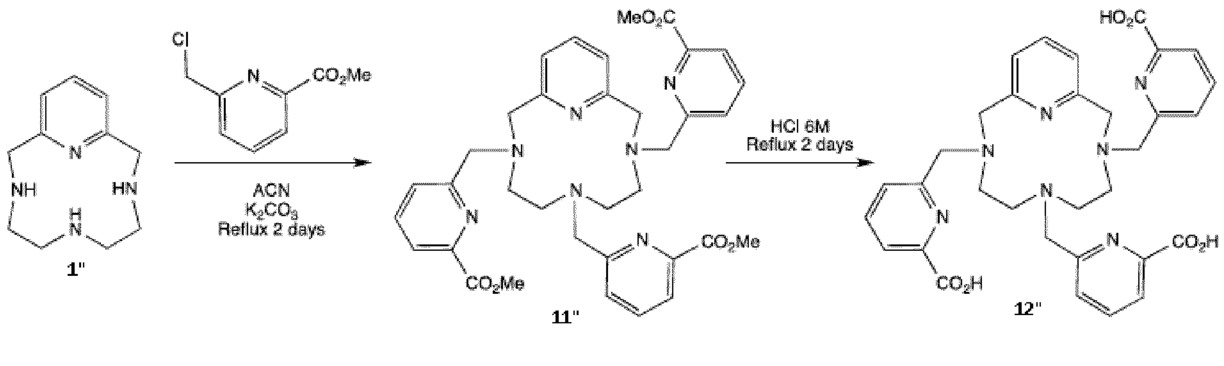


L'ester méthylique d'acide chloromethylpyridine-2-carboxylique (1.35 g, 7.28 mmol) a été ajouté à une solution du composé **1"** (0.50 g, 2.43 mmol) dans de l'acétonitrile (350 mL) en présence de $K_2CO_3$ (1 g, 7.28 mmol). Le mélange

a été mis sous reflux pendant deux jours, puis filtré et concentré. Le produit brut a été purifié par chromatographie sur colonne d'alumine ($CH_2Cl_2$/MeOH 98/2). Le composé **11"** est obtenu sous forme d'une huile jaune (862 mg, 54 %).

De l'acide chlorhydrique (20 mL, 6 M) a été ajouté au composé **11"** (862 mg, 1.32 mmol). Le mélange a été mis sous reflux 48h puis concentré. Le produit brut a été purifié par précipitation dans l'acétone. Le composé **12"** a été obtenu sous forme salifiée chlorhydrate (0.574 g, 57% calculé pour 4 HCl).
$^1$H RMN (300 MHz, $D_2O$) : $\delta$7.5-7.25 (m, 8H), 7.12-7.09 (m, 2H), 6.75 (d, 2H), 4.17 (s, 4H), 4.09 (s, 4H), 3.86 (s, 2H), 3.29 (m, 4H), 2.83 (m, 4H).
$^{13}$C NMR (75.47 MHz, $D_2O$) : $\delta$170.11, 168.95, 158.31, 154.51, 153.79, 150.12, 149.48, 145.95, 144.50, 143.67, 132.57, 132.24, 129.55, 126.76, 62.60, 62.03, 60.78, 57.15, 54.14.

**9-1) Synthèse d'un dérivé du bromure de picolinate de formule (II)**

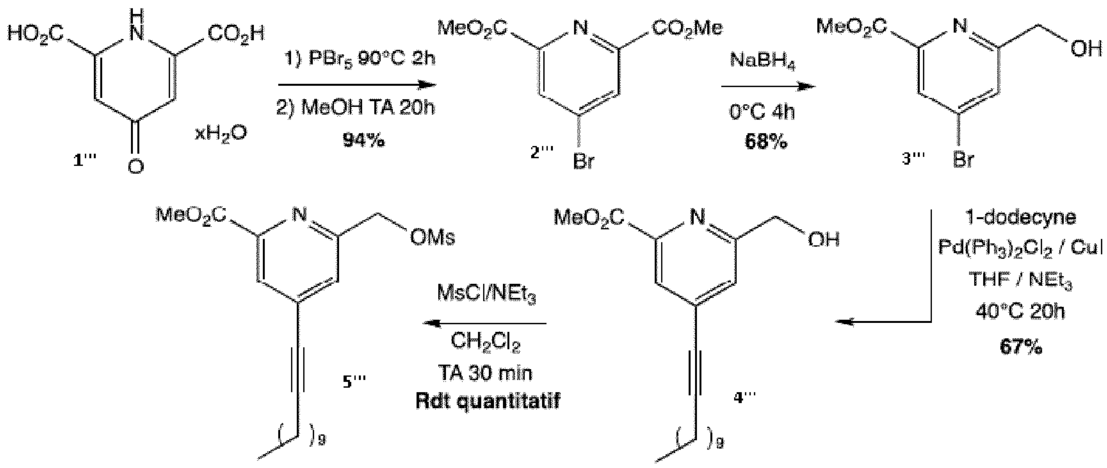


Le monohydrate d'acide chelidamique **1'''** (5 g, 24.9 mmol) et le pentabromure de phosphore (34 g, 79.0 mmol) ont été chauffés à 90°C. Une fois un mélange liquide obtenu, le chauffage est poursuivi pendant 2h à 90°C. Après refroidissement du mélange avec de la glace, du chloroforme (100 mL) et du MeOH (100 mL) ont été ajoutés. La solution est mélangée pendant 20h à température ambiante et le pH ajusté à 7 avec une solution saturée de $NaHCO_3$. Les solvants ont été évaporés et la phase aqueuse extraite à l'aide de dichlorométhane (3 x 100 mL). La phase organique a été séchée par du $MgSO_4$, filtrée et concentrée pour donner le composé **2'''** sous forme de solide blanc (6.43 g, 94 %)
$^1$H RMN (300 MHz, $CDCl_3$) : $\delta$ 8.42 (s, 2H), 3.99 (s, 6H).
$^{13}$C RMN (300 MHz, $CDCl_3$) : $\delta$ 164.02, 149.12, 135.13, 131.33, 53.52.

Le composé **2'''** (6.43 g, 23.5 mmol) a été solubilisé dans du dichlorométhane (50 mL) et du méthanol (70 mL). A 0°C, sous azote, $NaBH_4$ (1.02 g, 28.2 mmol) a été ajouté au mélange en petites quantités. Après 4h de mélange, l'acide chlorhydrique a été ajouté pour ajuster le pH à 5. Les solvants ont été évaporés, le pH de la phase aqueuse ajusté à 12 grâce à $Na_2CO_3$. La phase aqueuse a été extraite avec du dichlorométhane (3 x 100 mL), la phase organique a été séchée avec du $MgSO_4$, filtrée et concentrée sous vide. Après purification sur alumine, le composé **3'''** a été obtenu sous forme de solide blanc (3.92 g, 68 %).
$^1$H RMN (300 MHz, $CDCl_3$) : $\delta$ 8.12 (s, 1H), 7.76 (s, 1H), 4.82 (s, 2H), 3.95 (s, 3H).
$^{13}$C RMN (300 MHz, $CDCl_3$) : $\delta$ 164.55, 162.33, 147.96, 134.66, 127.31, 127.21, 64.49, 53.29.

Sous atmosphère inerte, $Pd(Ph_3)_2Cl_2$ (232 mg, 0.33 mmol) et CuI (124.2, 0.65 mmol) ont été ajoutés à une solution dégazée de 1-dodecyne (651 mg, 3.92 mmol) dans de la triéthylamine (10 mL) et **3'''** (800 mg, 3.26 mmol) dans du THF fraîchement distillé. Le mélange a été agité à 40°C pendant 20h. Après refroidissement à température ambiante, la suspension a été filtrée et triturée avec $Et_2O$ (40 mL). Le filtrat a été lavé avec une solution saturée de $NH_4Cl$ (2 x 50 mL) et de saumure (40 mL). Ensuite, la phase organique a été séchée sur $MgSO_4$, filtrée et concentrée sous vide. Le produit brut a été purifié sur gel de silice (hexane/acetate d'éthyle : 7/3 to 4/6) obtenant le composé **4'''** sous forme de solide blanc (727 mg, 67 %).
$^1$H RMN (300 MHz, $CDCl_3$) : $\delta$7.93 (s, 1H), 7.48 (s, 1H), 4.80 (s, 2H), 3.95 (s, 3H), 2.41 (t, 2H), 1.58 (m, 2H), 1.5-1.1 (m, 14H), 0.85 (t, 3H).
$^{13}$C RMN (300 MHz, $CDCl_3$) : $\delta$ 165.37, 160.62, 147.05, 134.54, 126.15, 125.98, 97.83, 78.05, 64.62, 53.02, 31.99, 29.67, 29.59, 29.40, 29.21, 29.01, 28.39, 22.77, 19.60, 14.20.

Le composé **4'''** (727 mg, 2,15 mmol) a été solubilisé dans du dichlorométhane (80 mL) avec de la triéthylamine

(653 mg, 6.45 mmol). Le chlorure de mésyl (369 mg, 3.23 mmol) a été ajouté et le mélange agité à température ambiante pendant 30 minutes. La phase organique a été lavée avec une solution aqueuse saturée d'hydrogénocarbonate de sodium (100 mL), puis séchée sur $MgSO_4$, filtrée et concentrée. Le composé **5'''** sous forme d'un solide blanc (896 mg, rendement quantitatif).

**9-2) Synthèse du ligand Pc1a2pa asym C12 P04245 de formule (I) :**

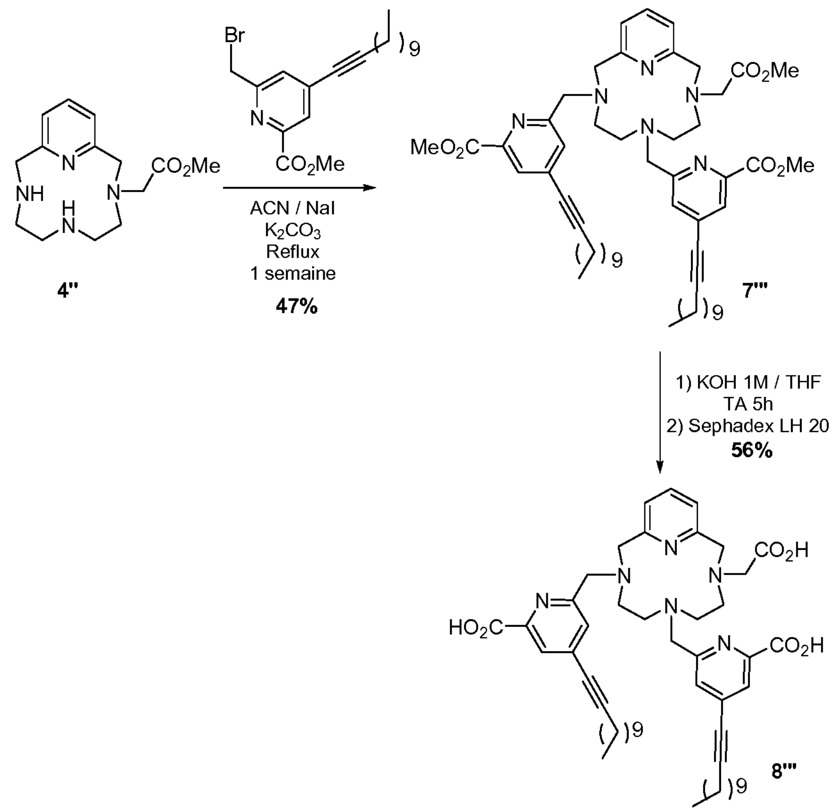


4''

7'''

8'''

Une solution du composé **5'''** (712 mg, 1.75 mmol) dans de l'acétonitrile (50 mL) a été ajoutée à une solution du composé **4"** mise sous reflux (243 mg, 0.87 mmol) dans l'acétonitrile (100 mL) en présence de $K_2CO_3$ (361 mg, 2.6 mmol). Le mélange a été mis sous reflux pendant une semaine. Après refroidissement à température ambiante, la suspension a été filtrée et le solvant évaporé. Le produit brut a été purifié par chromatographie sur colonne d'alumine pour obtenir le composé **7'''** sous forme d'huile de couleur jaune.

Obtention et purification du ligand Pc1a2pa asym C12 P04245 : Etape de saponification

Une solution de KOH (5 mL, 1M) a été ajoutée à une solution du composé **7'''** (91 mg, 0,10 mmol) dans du THF (6 mL). Le mélange a été agité vigoureusement pendant 5 heures à température ambiante. La phase organique a été évaporée puis le résidu a été purifié par chromatographie d'exclusion (Sephadex LH20, $CH_2Cl_2$/ MeOH de 100/0 à 90/10). Le produit **8'''** a été obtenu sous forme de solide incolore (48 mg, 56%).

**10) Synthèse de l'analogue Pc1a2pa asym C8 P04330 :**

**10-1) Synthèse d'un dérivé du Bromure de picolinate en C8**

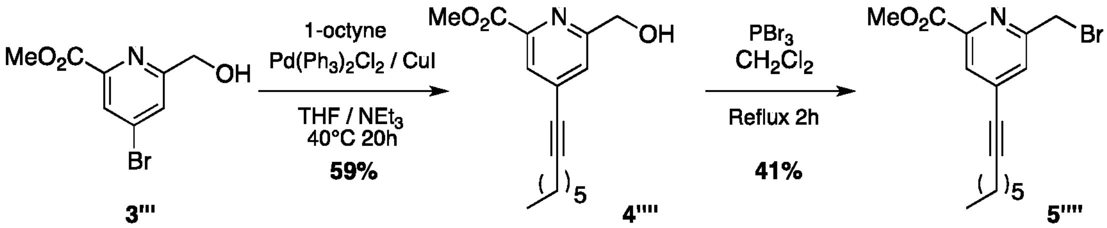


3''' 4'''' 5''''

Sous atmosphère inerte, $Pd(Ph_3)_2Cl_2$ (246 mg, 0,35 mmol) et CuI (134, 0,70 mmol) ont été ajoutés à une solution dégazée de 1-octyne (464 mg, 4,21 mmol) dans de la triéthylamine (10 mL) et **3'''** (863 mg, 3,51 mmol) dans du THF fraîchement distillé (20 mL). Le mélange a été agité à 40°C pendant 20h. Après refroidissement à température ambiante, la suspension a été filtrée et triturée avec $Et_2O$ (40 mL). Le filtrat a été lavé avec une solution saturée de $NH_4Cl$ (2 x 20 mL) et de saumure (20 mL). Ensuite, la phase organique a été séchée sur $MgSO_4$, filtrée et concentrée sous vide. Le produit brut a été purifié sur gel de silice (hexane/acetate d'éthyle : 7/3 to 4/6) obtenant le composé **4''''** sous forme de solide blanc (573 mg, 59 %).

$^1H$ NMR (300 MHz, $CDCl_3$): $\delta$ 7.69 (s, 1H), 7.39 (s, 1H), 4.63 (s, 2H), 3.74 (s, 3H), 2.22 (t, 2H), 1.40 (m, 2H), 1.24 (m, 2H), 1.35-1.15 (m, 4H), 0.69 (t, 3H).

$^{13}C$ NMR (300 MHz, $CDCl_3$): $\delta$ 164.88, 161.27, 146.43, 133.96, 125.53, 125.35, 97.05, 77.74, 64.26, 52.45, 30.96, 28.25, 27.94, 22.18, 19.12, 13.66.

Sous atmosphère inerte, une solution du composé **4''''** (573 mg, 2,08 mmol) dans du $CH_2Cl_2$ anhydre (50 mL) a été refroidie à 0°C. $PBr_3$ (676 mg, 2,5 mmol) a été ajouté puis le mélange a été chauffé au reflux pendant 2 heures. Après refroidissement à température ambiante, le milieu réactionnel a été neutralisé par 50 mL d'eau et $K_2CO_3$ jusqu'à pH 7. La phase organique a été séchée sur $MgSO_4$, filtrée puis concentrée sous vide. Après purification sur gel de silice (hexane, acétate d'éthyle de 9/1 à 4/6), le produit **5''''** a été obtenu sous forme de solide blanc (289 mg, 41 %).

$^1H$ NMR (300 MHz, $CDCl_3$): $\delta$ 7.91 (s, 1H), 7.54 (s, 1H), 4.52 (s, 2H), 3.93 (s, 3H), 2.37 (t, 2H), 1.54 (m, 2H), 1.37 (m, 2H), 1.3-1.2 (m, 4H), 0.85 (t, 3H).

$^{13}C$ NMR (300 MHz, $CDCl_3$): $\delta$ 165.00, 157.39, 147.63, 134.85, 128.82, 126.58, 98.23, 77.60, 53.04, 32.80, 31.25, 28.55, 28.18, 22.48, 19.48, 14.01.

**10-2) Synthèse du ligand Pc1a2pa asym C8 P04330**

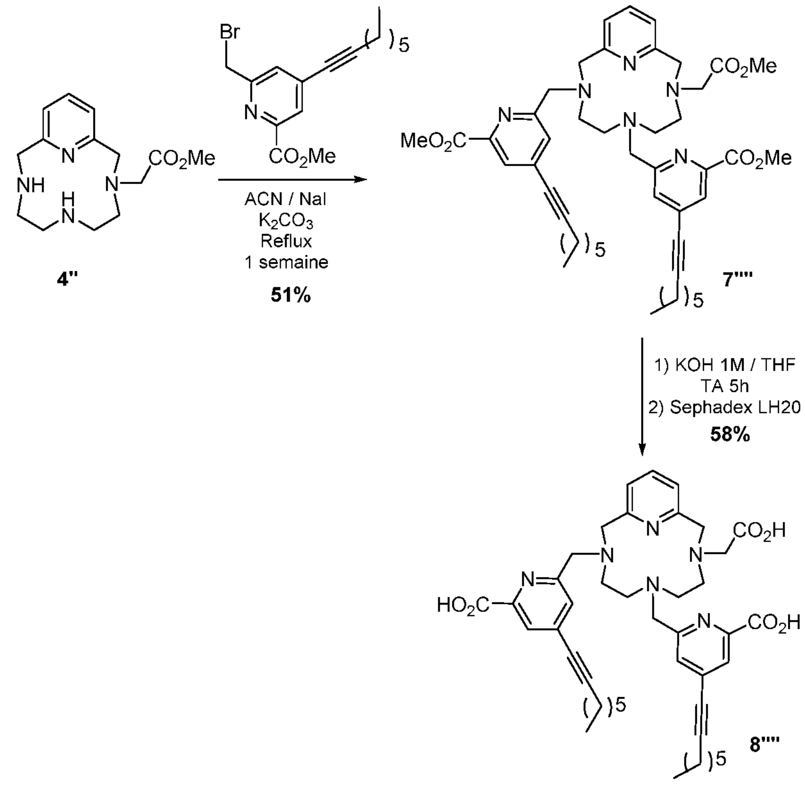


4"

7""

8""

Le composé **5""** (289 mg, 0,85 mmol) a été ajouté à une solution du composé **4"** (106 mg, 0,38 mmol) dans de l'acétonitrile anhydre (30 mL) en présence de $K_2CO_3$ (158 mg, 1,1 mmol). Le mélange a été mis sous reflux pendant une semaine. Après refroidissement à température ambiante, la suspension a été filtrée et le solvant évaporé. Le produit brut a été solubilisé dans un minimum d'acétate d'éthyle puis du pentane a été ajouté jusqu'à que ce la solution se trouble. L'huile formée a été rincée avec du pentane et précipitée une nouvelle fois. Le composé **7""** a été obtenu sous forme d'une huile de couleur jaune (155 mg, 51 %).

Une solution de KOH (2 mL, 1M) a été ajoutée à une solution du composé **7""** (55 mg, 0,069 mmol) dans du THF (5 mL). Le mélange a été agité vigoureusement pendant 5 heures à température ambiante. La phase organique a été évaporée puis le résidu a été purifié par chromatographie d'exclusion (Sephadex LH20, $CH_2Cl_2$/ MeOH de 100/0 à 90/10). Le produit **8""** a été obtenu sous forme de solide incolore (30 mg, 58%).

**C- Etude des composés de formule (I) et des complexes selon l'invention**

**C-1 Synthèse des complexes d'yttrium**

***1)*** *La procédure de synthèse du complexe Y-Pc1a2pa sym* ***P04183*** *est décrite ci-dessous et est applicable à l'ensemble des ligands de formule générale (I) :*

Le ligand **P04213** est solubilisé dans de l'eau ultra pure, le pH est ajusté à 5 avec une solution d'hydroxyde de sodium 1M. Le sel $YCl_3.6H_2O$ (1.5 eq) est dissous dans de l'eau ultra pure. Sous agitation, la solution d'yttrium est ajoutée à la solution de ligand. Après avoir ajusté le pH à 5, la solution est chauffée au reflux pendant une nuit. Le complexe est ensuite purifié par HPLC sur C18 ($H_2O$/ACN : de100/0 à 10/90) selon le schéma ci-dessous :

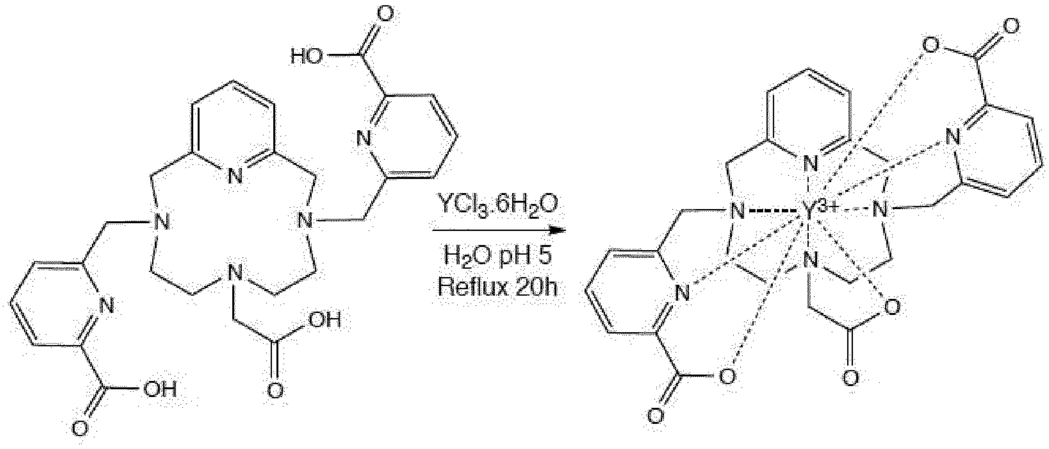


*__2)__ Synthèse d'un complexe d'yttrium 90, P04233 :*

Le ligand **P04214** a été engagé dans une réaction de complexation avec de l'yttrium 90 afin de confirmer les résultats de complexation obtenus avec l'yttrium naturel non radioactif. Une étude de radiomarquage a été réalisée.

Les paramètres étudiés sont les suivants :

| Paramètres | Plage d'étude | Optimum |
|---|---|---|
| pH | 1-9 | 6.5-9 |
| Température | 20-100°c | 80°c |
| Concentration en ligand Mol/L | $10^{-5}$-$10^{-2}$ mol/L | $10^{-4}$-$10^{-2}$ mol/L |
| Durée Min. | 5-60 min. | 15 min |

En résumé, les conditions de marquage optimales du **P04214** sont l'yttrium-90 en milieu acétate pH = 6,5-9, le ligand P04214 entre $10^{-4}$ et $10^{-2}$ M dans l'EtOH ; 15 min à 80°C. Le rendement de radiomarquage obtenu est > 90% (**P04233**).

*__3)__ Procédure et résultat du marquage du ligand __P04245__ avec l'Yttrium 90, obtention du complexe __P04283__ :*

Les paramètres étudiés sont les suivants :

| Paramètres | Plage d'étude | Optimum |
|---|---|---|
| pH | 4.65-9 | 6.5-9 |
| Température | 20-90°c | 50°c |
| Concentration en ligand Mol/L | $10^{-5}$-$10^{-3}$ mol/L | $10^{-3}$mol/L |
| Durée Min. | 5-60 min. | 15 min |

Les conditions de marquage optimales sont :

- Yttrium-90 en milieu acétate ;
- pH = 4,65-9 ;
- ligand P04245 à $10^{-3}$ M dans l'EtOH ;
- pendant 15 min à 50°C.

*__4)__ Procédure et résultat de l'extraction du complexe __P04283__ par le Lipiodol, obtention du __P04284__ :*

La solution contenant le complexe P04283 a été complétée à 2 mL par 1 mL de sérum, et un volume équivalent

de Lipiodol (2mL) a été ajouté à la solution contenant le complexe. Après agitation et centrifugation, les phases sont séparées et comptées. Le rendement d'extraction dans le Lipiodol est de 89,8 ± 5,0 % (n=3).

***5)*** *Procédure et résultats des tests de stabilité dans le sérum physiologique humain :* Procédure de préparation du radiotraceur **P04284**

1 mL d'acétate d'yttrium-90 à pH = 7 est ajouté à 1 mL de ligand P04245 en solution dans l'éthanol à une concentration de $10^{-3}$ mol/L pour former le complexe P04283. La solution est chauffée 30 min à 90°C. 2 mL de Lipiodol sont ajoutés et le mélange est agité vigoureusement. Les phases sont séparées par centrifugation (3500 tours/min, 15 min). La phase lipiodolée est collectée et complétée par 2 mL de Lipiodol pour donner le radiotraceur attendu P04284.
1 mL de radiotraceur fraichement préparé est prélevé puis déposé dans un flacon en verre à fond plat de 12 mL. L'activité est mesurée à l'activimètre, et l'heure notée. 10 mL de solution saline à 0,9% (sérum physiologique) sont ajoutés et le mélange est agité. Le flacon est ensuite déposé dans l'incubateur placé à 37°C, muni d'un agitateur réglé à 30 rpm (tours par minute).
On laisse l'agitation pendant plusieurs jours. La phase aqueuse est prélevée à différents temps pour doser l'Yttrium-90 relargué. Chaque échantillon a été réalisé en triplicat.
Les résultats sont donnés à la Figure 5. Les complexes formés selon l'invention et vectorisés par le Lipiodol sont stables dans le sérum physiologique.

**C-2 Synthèse des complexes de lanthanides :**

**1)** Les réactions de complexation du gadolinium par les ligands P04218 et P04216 ainsi que par le ligand P04213 sont réalisées dans l'eau en présence d'un équivalent de $GdCl_3$ à un pH 5-6 pendant une nuit à reflux.

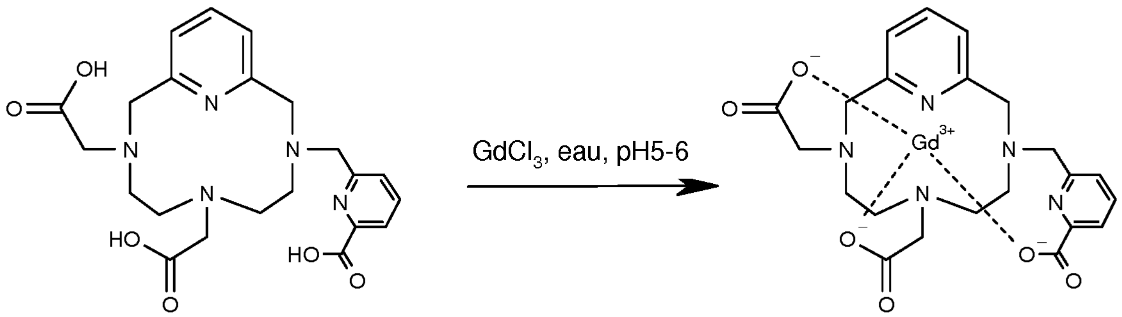


*Exemple: Complexation du ligand P04216 par le gadolinium*

La purification des complexes est réalisée par HPLC préparative afin de pouvoir éliminer les sels restants.

**2)** *Etude de relaxivité des complexes de gadolinium* :
Les études de relaxivité ont été réalisées sur les complexes de gadolinium des ligands P04218, P04216 et P04213, sur les appareils Minispec Mq-20 et Minispec Mq-60 (Bruker, Karlsruhe, Allemagne) à 20 MHz (0,47 T) et 60 MHz (1,4 T) dans l'eau à 37°C.

Pour chaque complexe préparé précédemment, une gamme de concentration en [Gd] allant de 0,5 à 5 mM a été réalisée puis les valeurs T1 et T2 de chacune de ces solutions ont été mesurées pour déterminer les valeurs de relaxivité r1 et r2 à l'aide de l'équation 1. Pour chacun des ligands, une droite de tendance dont le coefficient de corrélation était égal ou très proche de 1 a été obtenue, ce qui a permis de vérifier l'équation 1 et de valider la qualité des mesures réalisées. Les courbes tracées permettent de déterminer la valeur de relaxivité « r » qui correspond au coefficient « a » de l'équation de la droite « ax + b ».

$$r = \frac{1}{[Gd^{3+}]}\left(\frac{1}{T_{obs}} - \frac{1}{T_{H2O}}\right)$$

| Relaxivité ($mmol^{-1}s^{-1}$) | 20MHz | 60MHz |
|---|---|---|
| Pc2a1 pa sym P04218 | r1 = 3,9 r2 = 4,5 | r1 = 3,2 r2 = 3,9 |
| Pc2a1 pa asym P04216 | r1 = 3,7 r2 = 4,1 | r1 = 3,2 r2 = 3,7 |
| Pc1a2pa sym P04213 | r1 = 1,9 r2 = 2,1 | r1 = 1,6 r2 = 1,8 |

On constate que les relaxivités observées sont du même ordre de grandeur que celles obtenues avec les agents de contraste gadolinés utilisés en clinique, par exemple Dotarem®.

*3) Stabilité des complexes de Gd en milieu compétiteur :*

A une solution comprenant le complexe de gadolinium des ligands P04218 et P04216 à 2,5 mM dans un tampon phosphate à 333 mM est ajoutée une solution de $ZnCl_2$ à 2,5 mM. La valeur de la relaxivité de ces solutions est mesurée régulièrement. Le rapport entre la relaxivité mesuré à un temps donné et celle à t = 0 min en fonction du temps de présence dans la solution de Zn est donné en Figure 6. Les complexes selon l'invention sont stables au cours du temps.

*4) Synthèse et caractérisation des complexes*

Procédure générale de préparation des complexes de lanthanide (Ln= $Y^{3+}$, $Gd^{3+}$, $Eu^{3+}$, $Tb^{3+}$, $Yb^{3+}$, $Lu^{3+}$).
Le ligand est dissout dans l'eau et le pH est ajusté à 5 avec une solution de KOH 1M puis une solution du chlorure métallique (M= $Y^3$+, $Gd^{3+}$, $Eu^{3+}$, $Tb^{3+}$, $Yb^{3+}$, $Lu^{3+}$) est ajoutée (1.2 équivalents). Le mélange est porté à reflux une nuit et la solution obtenue est concentrée. Le complexe est purifié par chromatographie préparative sur une colonne de silice greffée C-18 et en éluant avec un mélange eau /acétonitrile.

| | **Acronyme** | **Ligand** | |
|---|---|---|---|
| Mono S | Pc-2a1 pa Sym | P04218 | L1 |
| Mono AS | Pc-2a1 pa Asym | P04216 | L3 |
| Di Sym | Pc-1 a2pa sym | P04213 | L2 |
| Di AS | Pc-1a2pa Asym | P04214 | L4 |

Synthèse de [ML1($H_2O$)]

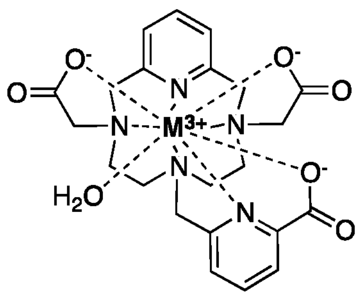

M3+: Y 3+, Gd3+, Eu3+, Tb3+, Yb3+, Lu3+ [YL1($H_2O$)]
L1.3HCl (27.2 mg, 0.048 mmol), $YCl_3.6H_2O$ (25.0 mg, 0.082 mmol)
Rendement: 24.5 mg, 91%
ESI-HR-MS (positive, $H_2O$) m/z calc. $[C_{22}H_{25}YN_5O_6]^+$, 544.0858; mesuré 544.0858 $[M + H]^+$, calc. $[C_{22}H_{26}YN_5O_6]^{2+}$, 272.5465; mesuré 272.5469 $[M + 2H]^{2+}$.

[$GdL1(H_2O)$]

L1.3HCl (36.5 mg, 0.064 mmol), $GdCl_3.6H_2O$ (27.5 mg, 0.074 mmol)
Rendement: 39.8 mg, 98%
ESI-HR-MS (positive, $H_2O$) m/z calc. $[C_{22}H_{25}GdN_5O_6]^+$, 613.1040; mesuré 613.1031 $[M + H]^+$, calc. $[C_{22}H_{26}GdN_5O_6]^{2+}$, 307.0557; mesuré 307.0560 $[M + 2H]^{2+}$.

[$EuL1(H_2O)$]

L1.3HCl (22.0 mg, 0.039 mmol), $EuCl_3.6H_2O$ (17.1 mg, 0.047 mmol)
Rendement: 22.1 mg, 91%
ESI-HR-MS (positive, $H_2O$) m/z calc. $[C_{22}H_{25}EuN_5O_6]^+$, 608.1012; mesuré 608.1004 $[M + H]^+$, calc. $[C_{22}H_{26}EuN_5O_6]^{2+}$, 304.5542; mesuré 304.5544 $[M + 2H]^{2+}$.

[$TbL1(H_2O)$]

L1.3HCl (22.0 mg, 0.039 mmol), $TbCl_3$.6H2O (17.4 mg, 0.047 mmol)
Rendement: 22.6 mg, 95%
ESI-HR-MS (positive, $H_2O$) m/z calc. $[C_{22}H_{25}TbN_5O_6]^+$, 614.1053; mesuré 614.1048 $[M + H]^+$, calc. $[C_{22}H_{26}TbN_5O_6]^{2+}$, 307.5563; mesuré 307.5565 $[M + 2H]^{2+}$.

[$YbL1(H_2O)$]

L1.3HCl (25.0 mg, 0.044 mmol), $YbCl_3$.6H2O (20.5 mg, 0.053 mmol)
Rendement: 27.3 mg, 96%
ESI-HR-MS (positive, $H_2O$) m/z calc. $[C_{22}H_{25}YbN_5O_6]^+$, 629.1188; mesuré 629.1187 $[M + H]^+$, calc. $[C_{22}H_{26}YbN_5O_6]^{2+}$, 315.0630; mesuré 315.0635 $[M + 2H]^{2+}$.

[$LuL1(H_2O)$]

L1.3HCl (25.0 mg, 0.044 mmol), $LuCl_3.6H_2O$ (20.6 mg, 0.053 mmol)
Rendement: 26 mg, 91%
ESI-HR-MS (positive, $H_2O$) m/z calc. $[C_{22}H_25 5LuN_5O_6]^+$, 630.1207; found 630.1196 $[M + H]^+$, calc. $[C_{22}H_{26}LuN_5O_6]^{2+}$, 315.5640; found 315.5641 $[M + 2H]^{2+}$.

Synthèse de [ML2]

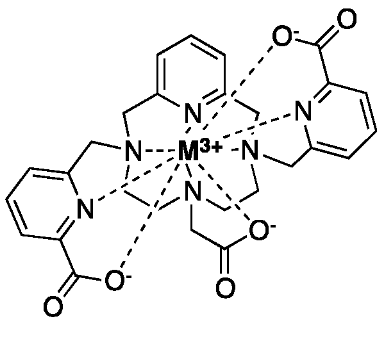

M3+: Y 3+, Gd3+, Eu3+, Tb3+, Yb3+, Lu3+ [YL2]
L2.3HCl (100.0 mg, 0.155 mmol), $YCl_3$.6H2O (89.0 mg, 0.293 mmol)
Rendement: 84.8 mg, 88%
ESI-HR-MS (positive, $H_2O$) m/z calc. $[C_{27}H_{28}YN_6O_6]^+$, 621.1123; mesuré 621.1116 $[M + H]^+$, calc. $[C_{27}H_{29}YN_6O_6]^{2+}$, 311.0598; mesuré 311.0603 $[M + 2H]^{2+}$.

[GdL2]

L2.3HCl (39.0 mg, 0.061 mmol), $GdCl_3$.6H2O (27.0 mg, 0.073 mmol)
Rendement: 41.1 mg, 99%
ESI-HR-MS (positive, $H_2O$) m/z calc. $[C_{27}H_{28}GdN_6O_6]^+$, 690.1306; mesuré 690.1313 $[M + H]^+$, calcd. for $[C_{27}H_{29}GdN_6O_6]^{2+}$, 345.5689; mesuré 345.5690 $[M + 2H]^{2+}$.

[EuL2]

L2.3HCl (25.0 mg, 0.039 mmol), $EuCl_3.6H_2O$ (17.1 mg, 0.047 mmol)
Rendement: 25.3 mg, 96%
ESI-HR-MS (positive, $H_2O$) m/z calc. $[C_{27}H_{28}EuN_6O_6]^+$, 685.1277; mesuré 685.1279 $[M + H]^+$, calc. $[C_{27}H_{29}EuN_6O_6]^{2+}$, 343.0675; mesuré 343.0680 $[M + 2H]^{2+}$.

[TbL2]

L2.3HCl (20.0 mg, 0.031 mmol), $TbCl_3.6H_2O$ (13.9 mg, 0.037 mmol)
Rendement: 19.6 mg, 92%
ESI-HR-MS (positive, $H_2O$) m/z calc. $[C_{27}H_{28}TbN_6O_6]^+$, 691.1318; mesuré 691.1314 $[M + H]^+$, calc. $[C_{27}H_{29}TbN_6O_6]^{2+}$, 346.0696; mesuré 346.0697 $[M + 2H]^{2+}$.

[YbL2]

L2.3HCl (22.0 mg, 0.034 mmol), $YbCl_3.6H_2O$ (15.9 mg, 0.041 mmol)
Rendement: 22.1 mg, 92%
ESI-HR-MS (positive, $H_2O$) m/z calc. $[C_{27}H_{28}YbN_6O_6]^+$, 706.1453; mesuré 706.1454 $[M + H]^+$, calc. $[C_{27}H_{29}YbN_6O_6]^{2+}$, 353.5763; mesuré 353.5764 $[M + 2H]^{2+}$.

[LuL2]

L2.3HCl (22.0 mg, 0.034 mmol), $LuCl_3.6H_2O$ (16.0 mg, 0.041 mmol)
Rendement: 22.8 mg, 95%
ESI-HR-MS (positive, $H_2O$) m/z calc. $[C_{27}H_{28}LuN_6O_6]^+$, 707.1473; mesuré 707.1476 $[M + H]^+$, calc. $[C_{27}H_{29}LuN_6O_6]^{2+}$, 354.0773; mesuré 354.0776 $[M + 2H]^{2+}$.

Synthèse de $[ML3(H_2O)]$

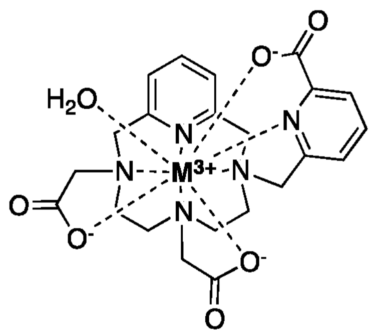

M3+: Y 3+, Gd3+, Eu3+, Tb3+, Yb3+, Lu3+ $[YL3(H_2O)]$
L3.3HCl (30.0 mg, 0.053 mmol), $YCl_3.6H_2O$ (24.0 mg, 0.079 mmol)
Rendement: 28.0 mg, 94%
ESI-HR-MS (positive, $H_2O$) m/z calc. $[C_{22}H_{25}YN_5O_6]^+$, 544.0858; mesuré 544.0859 $[M + H]^+$, calc. $[C_{22}H_{26}YN_5O_6]^{2+}$, 272.5465; mesuré 272.5469 $[M + 2H]^{2+}$.

[$GdL3(H_2O)$]

L3.3HCl (53.0 mg, 0.093 mmol), $GdCl_3$.6H2O (41.3 mg, 0.111 mmol)
Rendement: 58.7 mg, 99%
ESI-HR-MS (positive, $H_2O$) m/z calc. $[C_{22}H_{25}GdN_5O_6]^+$, 613.1040; mesuré 613.1030 $[M + H]^+$, calc. $[C_{22}H_{26}GdN_5O_6]^{2+}$, 307.0557; mesuré 307.0568 $[M + 2H]^{2+}$.

[$EuL3(H_2O)$]

L3.3HCl (28.5 mg, 0.050 mmol), $EuCl_3.6H_2O$ (22.1 mg, 0.060 mmol)
Rendement: 29.0 mg, 92%
ESI-HR-MS (positive, $H_2O$) m/z calc. $[C_{22}H_{25}EuN_5O_6]^+$, 608.1012; mesuré 608.1007 $[M + H]^+$, calc. $[C_{22}H_{26}EuN_5O_6]^{2+}$, 304.5542; mesuré 304.5544 $[M + 2H]^{2+}$.

[$TbL3(H_2O)$]

L3.3HCl (24.0 mg, 0.042 mmol), $TbCl_3$.6H2O (19.0 mg, 0.051 mmol)
Rendement: 23.2 mg, 89%
ESI-HR-MS (positive, $H_2O$) m/z calc. $[C_{22}H_{25}TbN_5O_6]^+$, 614.1053; mesuré 614.1049 $[M + H]^+$, calc. $[C_{22}H_{26}TbN_5O_6]^{2+}$, 307.5563; mesuré 307.5563 $[M + 2H]^{2+}$.

[$YbL3(H_2O)$]

L3.3HCl (25.0 mg, 0.044 mmol), $YbCl_3$.6H2O (20.5 mg, 0.053 mmol)
Rendement: 27.8 mg, 98%
ESI-HR-MS (positive, $H_2O$) m/z calc. $[C_{22}H_{25}YbN_5O_6]^+$, 629.1188; mesuré 629.1182 $[M + H]^+$, calc. $[C_{22}H_{26}YbN_5O_6]^{2+}$, 315.0630; mesuré 315.0634 $[M + 2H]^{2+}$.

[$LuL3(H_2O)$]

L3.3HCl (28.0 mg, 0.049 mmol), $LuCl_3.6H_2O$ (23.1 mg, 0.059 mmol)
Rendement: 29 mg, 91%
ESI-HR-MS (positive, $H_2O$) m/z calc. $[C_{22}H_{25}LuN_5O_6]^+$, 630.1207; mesuré 630.1204 $[M + H]^+$, calc. $[C22H26LuN5O6]^{2+}$, 315.5640; mesuré 315.5642 $[M + 2H]^{2+}$.

Synthèse de [$ML4(H_2O)$]

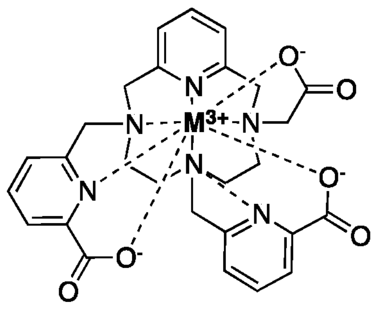

M3+: Y 3+, Gd3+, Eu3+, Tb3+, Yb3+, Lu3+ [YL4]
L4.3HCl (30.0 mg, 0.047 mmol), $YCl_3.6H_2O$ (24.0 mg, 0.079 mmol)
Rendement: 24.8 mg, 92%
ESI-HR-MS (positive, $H_2O$) m/z calc. $[C_{27}H_{28}YN_6O_6]^+$, 621.1123; mesuré 621.1121 $[M + H]^+$, calc. $[C_{27}H_{29}YN_6O_6]^{2+}$, 311.0598; mesuré 311.0601 $[M + 2H]^{2+}$.

[GdL4]

L4.3HCl (36.2 mg, 0.056 mmol), $GdCl_3.6H_2O$ (25.1 mg, 0.068 mmol)
Rendement: 38.1 mg, 98%
ESI-HR-MS (positive, $H_2O$) m/z calc. $[C_{27}H_{28}GdN_6O_6]^+$, 690.1306; mesuré 690.1321 $[M + H]^+$, calc. $[C_{27}H_{29}GdN_6O_6]^{2+}$, 345.5698; mesuré 345.5690 $[M + 2H]^{2+}$.

[EuL4]

L4.3HCl (23.5 mg, 0.036 mmol), $EuCl_3.6H_2O$ (16.0 mg, 0.044 mmol)
Rendement: 21.8 mg, 87%
ESI-HR-MS (positive, $H_2O$) m/z calc. $[C_{27}H_{28}EuN_6O_6]^+$, 685.1277; mesuré 685.1277 $[M + H]^+$, calc. $[C_{27}H_{29}EuN_6O_6]^{2+}$, 343.0675; mesuré 343.0680 $[M + 2H]^{2+}$.

[TbL4]

L4.3HCl (24.0 mg, 0.037 mmol), $TbCl_3.6H_2O$ (16.4 mg, 0.044 mmol)
Rendement: 25.3 mg, 98%
ESI-HR-MS (positive, $H_2O$) m/z calc. $[C_{27}H_{28}TbN_6O_6]^+$, 691.1318; mesuré 691.1316 $[M + H]^+$, calc. $[C_{27}H_{29}TbN_6O_6]^{2+}$, 346.0696; mesuré 346.0699 $[M + 2H]^{2+}$.

[YbL4]

L4.3HCl (30.0 mg, 0.047 mmol), $YbCl_3.6H_2O$ (21.7 mg, 0.056 mmol)
Rendement: 30.4 mg, 93%
ESI-HR-MS (positive, $H_2O$) m/z calc. $[C_{27}H_{28}YbN_6O_6]^+$, 706.1453; mesuré 706.1454 $[M + H]^+$, calc. $[C_{27}H_{29}YbN_6O_6]^{2+}$, 353.5763; mesuré 353.5768 $[M + 2H]^{2+}$.

[LuL4]

L4.3HCl (30.0 mg, 0.047 mmol), $LuCl_3.6H_2O$ (21.8 mg, 0.056 mmol)
Rendement: 30.4 mg, 92%
ESI-HR-MS (positive, $H_2O$) m/z calc. $[C_{27}H_{28}LuN_6O_6]^+$, 707.1473; mesuré 707.1470 $[M + H]^+$, calc. $[C_{27}H_{29}LuN_6O_6]^{2+}$, 354.0773; mesuré 354.0776 $[M + 2H]^{2+}$.

## C-3 Étude en solution

***1)** Étude par Résonance Magnétique Nucléaire :*

A titre d'exemple, les spectres RMN $^1H$ du ligand **P04213** et de son complexe d'yttrium **P04183** enregistrés dans $D_2O$ sont représentés en Figure 1. Par rapport au spectre du ligand, la présence du cation métallique engendre une dissymétrie et donc un nombre de signaux plus important (cf. Figure 1).

***2)** Étude par spectroscopie UV-visible :*

Les spectres d'absorption des ligands et de leurs complexes d'yttrium ont été enregistrés dans l'eau à pH 3,8 et 5,5 (tampon acétate). La bande d'absorption correspondant aux transitions $\pi$ - $\pi^*$ de la pyridine s'étend de 240 à 300 nm pour les ligands et les complexes (cf. Figure 2)

## C-4 Cinétique de complexation

Les cinétiques de complexation des ligands Pc1a2pa sym **P04213,** Pc1a2pa asym **P04214** et Pc2a1pa sym **P04218** avec l'yttrium ont été étudiées à pH 3,8 et pH 5,5 en milieu tampon acétate par spectroscopie UV-Visible. Placée au maximum d'absorption du complexe, l'augmentation de l'intensité d'absorbance est mesurée toutes les deux secondes jusqu'à atteindre l'absorbance maximale. La diminution de l'intensité de l'absorbance au maximum d'absorption du ligand est suivie lorsque la bande d'absorption du complexe est masquée par celle du ligand. Pour cette étude, la concentration des ligands Pc1a2pa sym et Pc1a2pa asym est de $4.10^{-5}$ M et de $8.10^{-5}$ M pour le ligand Pc2a1pa sym. A pH 5,5 et 3,8, le ligand Pc1a2pa sym présente la cinétique de complexation la plus rapide avec une complexation

totale en respectivement 30 et 400 secondes. Pour les ligands Pc1a2pa asym et Pc2a1pa sym, la complexation est totale en 1100 secondes à pH 3,8 et 100 secondes à pH 5,5.

La complexation est donc rapide pour l'ensemble des ligands dans les conditions étudiées. (cf. Figure 3).

## C-5 Inertie cinétique en milieu compétiteur

La cinétique de dissociation des complexes en milieu acide concentré permet de connaître le comportement des complexes en milieu très compétiteur. La vitesse de décomplexation est suivie par spectroscopie UV-visible, avec $C_{YL}$ = 4.10$^{-5}$ M pour les complexes Y-Pc1a2pa sym **P04183,** Y-Pc1a2pa asym **P04215** et Y-Pc2a1pa sym **P04219,** en milieu HCl 0,5, 1, 2, 4 et 5 M. La bande d'absorption du complexe disparaît plus ou moins rapidement pour faire apparaître la bande d'absorption du ligand à des longueurs d'onde plus basses. Le tracé de l'augmentation de l'intensité de l'absorbance au maximum d'absorption du ligand en fonction du temps (A = f(t)) permet de connaître les temps de demi-vie $t_{½}$. Les valeurs des $t_{½}$ des différents complexes sont répertoriées dans le Tableau ci-dessous. Les complexes peuvent être classés de la façon suivante du plus inerte au moins inerte : Y-Pc1a2pa asym >> Y-Pc1a2pa sym > Y-PCTA > Y-PCTMB > Y-Pc2a1pa sym. La présence de deux bras picolinate sur le macrocycle pyclène augmente l'inertie du complexe d'yttrium en milieu acide. De plus, l'inertie du complexe Y-Pc1a2pa asym est supérieure à celle de son analogue symétrique avec respectivement un $t_{½}$ de 433 minutes en milieu HCl 5 M contre 8,5 minutes.

| Ligands | PCTMB | Pc1a2pa sym | Pc1a2pa asym | Pc2a1pa sym | PCTA |
|---|---|---|---|---|---|
| $C_{HCl}$ | $t_{1/2}$ (min) | | | | |
| 0,5 M | 37 | 347 | > 1 sem (en cours) | 55 | 95 |
| 1 M | 20 | 140 | (en cours) | 27 | 39 |
| 2 M | 9 | 51 | 2745 | 10,6 | 17 |
| 4 M | 3,2 | 13 | 907 | 2,7 | 6,7 |
| 5 M | 2.6 | 8,5 | 433 | 0.8 | 3,1 |

## C-6 Études de stabilité thermodynamiques par potentiométrie

### 1) Constantes de protonation des ligands

Quatre constantes de protonation ont été déterminées pour les ligands Pc1a2pa sym **P04213,** Pc1a2pa asym **P04214,** Pc2a1pa sym **P04216** et Pc3pa **P04221.** Ces valeurs sont cohérentes avec celles déterminées pour le PCTMB (Phosphonic acid, P,P',P''-[3,6,9,15-tetraazabicyclo[9.3.1]pentadeca-1(15),11,13-triene-3,6,9-triyltris(methylene)]tris-, P,P',P''-tributyl ester) ainsi qu'avec celles décrites dans la littérature notamment pour le PCTA (3,6,9,15-tetraazabicyclo[9.3.1]pentadeca-1(15),11,13-triene-3,6,9-triacetic acid), EDTA (Ethylenediaminetetraacetic acid) et DOTA (1,4,7,10-Tetraazacyclododecane-1,4,7,10-tetraacetic acid.

| | Ligands | PCTMB | EDTA [2] | PCTA [3] | DOTA [4] | Pc1a2pa sym | Pc1a2pa asym | Pc2a1pa sym |
|---|---|---|---|---|---|---|---|---|
| | *I* | *0,1 $KNO_3$* | *0,1 $KNO_3$* | *1,0 KCl* | *0,1 $Me_4NNO_3$* | *0,1 $KNO_3$* | *0,1 $KNO_3$* | *0,1 $KNO_3$* |
| $\log K_i^H$ | [HL]/[L][H] | 11,16 | 10,22 | 11,36 | 12,09 | 11,30 | 10,50 | 10,43 |
| | $[H_2L]/[HL][H]$ | 5,28 | 6,16 | 7,35 | 9,76 | 5,58 | 6,73 | 7,38 |
| | $[H_3L]/[H_2L][H]$ | 1,72 | 2,71 | 3,83 | 4,56 | 4,23 | 3,86 | 3,95 |
| | $[H_4L]/[H_3L][H]$ | - | 2,0 | 2,12 | 4,09 | 3,01 | 2,98 | 2,15 |
| | $[H_5L]/[H_4L][H]$ | - | - | 1,29 | - | - | - | - |
| | $\Sigma\log K_i$ | 18,15 | 21,09 | 25,95 | 30,50 | 24,12 | 24,06 | 23,90 |

*Tableau 13*

Pour le dérivé Pc3pa **P04221** les valeurs obtenues sont les suivantes :

*Tableau 14*

| Pc3pa **P04221** | | |
|---|---|---|
| $logK^{H}_{i}$ | $[HL]/[L][H]$ | 10.03 |
| | $[H_2L]/[HL][H]$ | 5.95 |
| | $[H_3L]/[H_2L][H]$ | 3.82 |
| | $[H_4L]/[H_3L][H]$ | 2.98 |

**2)** Constantes de stabilité des complexes

Les constantes thermodynamiques de protonation et de stabilité des complexes ont été déterminées par potentiométrie à 25°C avec contrôle de la force ionique (I = 0,1 M $KNO_3$). L'affinement des courbes de titrage avec le logiciel HyperQuad permet de déterminer les constantes globales (log β), à partir desquelles les constantes partielles (log K) sont calculées.

Les constantes de stabilité des ligands Pc1a2pa sym **P04213,** Pc1a2pa asym **P04214** et Pc2a1pa sym **P04218** et **P04221** avec l'yttrium, ont été déterminées par titrage potentiométrique direct. Les valeurs des constantes log $K_{YL}$ des ligands Pc1a2pa sym, Pc1a2pa asym et Pc2a1pa sym sont respectivement de 19.78, 19.49, 19.28 et celles des constantes log $K_{YLH}{}^{-1}$ sont de 11.84, 11.79 et 10.60.

| | | Equilibre de réaction [a] | PCTMB [b] | EDTA [c] | PCTA [f] | DOTA [g] | Pc1a2pa sym | Pc1a2pa asym | Pc2a1pa sym |
|---|---|---|---|---|---|---|---|---|---|
| $\log K_{MHiL}$ | $Y^{3+}$ | [ML]/[M][L] | 19,49 | 18,5 [d] | 20,28 | 24,9 [h] | 19,78 | 19,49 | 19,28 |
| | | [MHL]/[ML][H] | 3,45 | - | 1,81 | - | - | - | - |
| | | [ML]/[MLOH][H] | 9,10 | - | 11,10 | - | 11,84 | 11,79 | 10,60 |

[a] Pour des raisons de clareté, les charges ne sont pas indiquées. [b] Valeurs déterminées par compétition avec l'EDTA, 0,1 M $KNO_3$. [c] Ref **2,** 0,1 M $KNO_3$. [d] Ref [5], 0,1 M $NMe_4Cl$. [e] Ref [6], 0,1M $KNO_3$. [f] Ref **3**, 1,0 M KCl. [g] Ref **4**, 0,1 M $NMe_4Cl$. [h] Ref [7], 0,1 M $NMe_4NO_3$.

*Tableau 15*

*Tableau 16*

| Pc3pa + $Y^{3+}$**P04222** | | |
|---|---|---|
| $logK_{MHiL}$ | **ML]/[M][L]** | 16.42 |
| | [MHL]/[ML][H] | 3.11 |
| | [ML]/[MLOH][H] | 11.02 |

Ces constantes de stabilité ne sont pas comparables telles quelles, il faut prendre en compte la basicité des ligands. La constante pM = -log[M] est utilisée à cet effet. Elle est calculée à partir des constantes de protonation des ligands et de stabilité des complexes avec CL = 10 x CM = $10^{-5}$ M à pH 7,4. Le ligand Pc1a2pa asym**P04214** présente un p(Y) de 17.3, supérieur à celui du PCTA (p(Y) =17.0), du Pc1a2pa sym **P04213** (p(Y) = 16.8) et du Pc2a1pa sym **P04218** (p(Y) = 16.9). Le p(Y) le plus élevé reste néanmoins celui du DOTA avec une valeur de 18,8.

pM= -log[M] [a]

| Ligands | PCTMB | EDTA | PCTA | DOTA | Pc1a2pa sym | Pc1a2pa asym | Pc2a1pa sym |
|---|---|---|---|---|---|---|---|
| pY | 16,7 | 16,6 | 17,0 | 18,8 | 16,8 | 17,3 | 16,9 |

[a] Valeurs calculées à partir des constantes des tableaux précédents avec $C_L = 10 \times C_M = 10^{-5}$ M à pH 7,4.

*Tableau 17*

Pour le Pc3pa **P04222,** le pM calculé est de 14.7.

Les diagrammes de spéciation, tracés à partir des constantes de stabilité thermodynamique des complexes d'yttrium, indiquent que les complexes existent exclusivement sous la forme YL sur une large plage de pH, y compris à pH 7,4.

Références :

1 Aime, S.; Botta, M.; Geninatti Crich, S.; Giovenzana, G. B.; Jommi, G.; Pagliarin, R.; Sisti, M. Inorg. Chem.1997, 36, 2992-3000.

2 Delgado, R.; Figueira, C.; Quintino, S. Talanta 1997, 45, 451.

3 Tircsó, G.; Kovacs, Z.; Dean Sherry, A. Inorg. Chem. 2006, 45, 9269.

4 Chaves, S.; Delgado, R.; Frausto da Silva, J. J. R. Talanta 1992, 39, 249.

5 Kumar, K. ; Chang C. A. ; Francesconi, L. C. ; Dischino, D. D. ; Malley, M. F. ; Gougoutas, J. Z. ; Tweedle,M.F. Inorg. Chem. 1994, 33, 3567.

6 Delgado, R.; Frausto da Silva, J. J. R. Talanta 1982, 29, 815.

7 Cox, J. P. L.; Jankowski, K. J.; Kataky, R.; Parker, D.; Beeley, N. R. A.; Boyce, B. A.; Eaton, M. A. W.; Millar,K.; Millican, A. T.; Harrison, A.; Walkerc, C. J. Chem. Soc. Chem. Commun. 1989, 797.


## C-7 Étude à l'état solide

Le complexe d'yttrium **P04183** cristallise dans l'eau. La structure obtenue par diffraction des rayons X est présentée ci-dessous. Le métal est coordiné par les quatre atomes d'azote du macrocycle, les deux atomes d'azote des bras picolinate et les trois atomes d'oxygène des acides carboxyliques. La sphère de coordination du métal est N6O3, soit 9 atomes coordinants. Les hélicités $\Delta$ et $\Lambda$ issues de l'orientation des bras picolinate et acétate sont toutes les deux présentes, le complexe cristallise donc en mélange racémique.

## Revendications

1. Composé de formule générale (I) suivante :

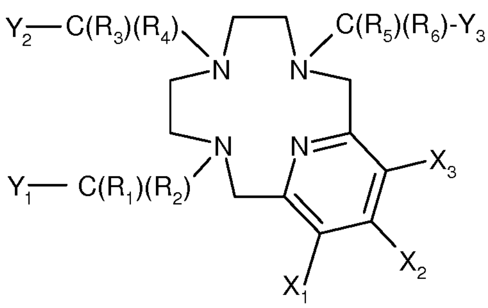

(I)

dans laquelle :

- $R_1$, $R_2$, $R_3$, $R_4$, $R_5$ et $R_6$ représentent indépendamment les uns des autres H, un groupement $(C_1\text{-}C_{20})$alkyle ou un groupement $(C_1\text{-}C_{20})$alkylène-$(C_6\text{-}C_{10})$aryle ; lesdits groupements alkyle, alkylène et aryle pouvant éventuellement être substitués par un ou plusieurs substituant(s) choisi(s) parmi les fonctions acides organiques ;
- $X_1$, $X_2$ et $X_3$ sont choisis indépendamment les uns des autres parmi le groupe constitué de : H, -C(O)N(Re)(Rd), $(C_1\text{-}C_{20})$alkyle, $(C_2\text{-}C_{20})$alcényle, $(C_2\text{-}C_{20})$alcynyle et $(C_6\text{-}C_{10})$aryle,
avec Re et Rd étant indépendamment l'un de l'autre H ou un groupement $(C_1\text{-}C_{20})$alkyle,
lesdits groupements alkyle, alcényle et alcynyle pouvant éventuellement comprendre un ou plusieurs hétéroatome(s) et/ou un ou plusieurs $(C_6\text{-}C_{10})$arylène(s) dans leur chaîne et pouvant être éventuellement substitués par un $(C_6\text{-}C_{10})$aryle ;
lesdits groupements alkyle, alcényle, alcynyle et aryle pouvant éventuellement être substitués par un ou plusieurs substituant(s) choisi(s) parmi les fonctions acides organiques ;

- $Y_1$, $Y_2$ et $Y_3$ représentent indépendamment les uns des autres un groupement C(O)OH ou un groupement de formule (II) suivante :

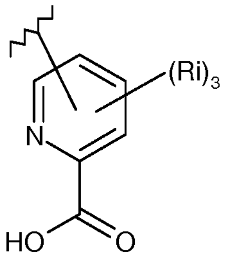

(II)

dans lequel :

- les radicaux Ri sont choisis indépendamment les uns des autres parmi le groupe constitué de : H, halogène, $N_3$, $(C_1\text{-}C_{20})$alkyle, $(C_2\text{-}C_{20})$alcényle, $(C_2\text{-}C_{20})$alcynyle et $(C_6\text{-}C_{10})$aryle,

lesdits groupements alkyle, alcényle et alcynyle pouvant éventuellement comprendre un ou plusieurs hétéroatome(s) et/ou un ou plusieurs $(C_6\text{-}C_{10})$arylène(s) dans leur chaîne et pouvant être éventuellement substitués par un $(C_6\text{-}C_{10})$aryle ;
lesdits groupements alkyle, alcényle, alcynyle et aryle pouvant éventuellement être substitués par un ou plusieurs substituant(s) choisi(s) parmi les fonctions acides organiques ;
et
l'un au moins des radicaux $Y_1$, $Y_2$ et $Y_3$ étant un groupement de formule (II) ;
ou l'un de ses sels pharmaceutiquement acceptables.

**2.** Composé selon la revendication 1, dans lequel lorsque les radicaux $Y_1$, $Y_2$ ou $Y_3$ représentent un groupement de formule (II), les radicaux correspondants $R_1$ et $R_2$, $R_3$ et $R_4$ ou $R_5$ et $R_6$ représentent H.

**3.** Composé selon la revendication 1 ou 2, dans lequel les radicaux Ri sont choisis indépendamment les uns des autres parmi le groupe constitué de : H, $(C_1\text{-}C_{20})$alkyle, $(C_2\text{-}C_{20})$alcényle et $(C_2\text{-}C_{20})$alcynyle,
lesdits groupements alkyle, alcényle et alcynyle pouvant éventuellement comprendre un ou plusieurs hétéroatome(s) choisi(s) parmi N, O ou S.

**4.** Composé de formule (I) selon la revendication 1, choisi parmi le groupe constitué des composés suivants :

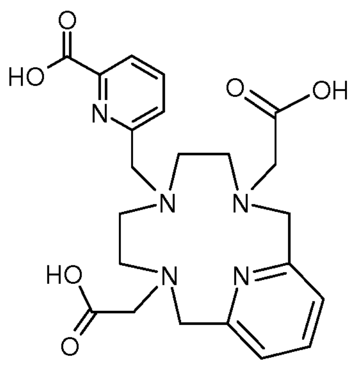

**Pc-2a1pa sym**

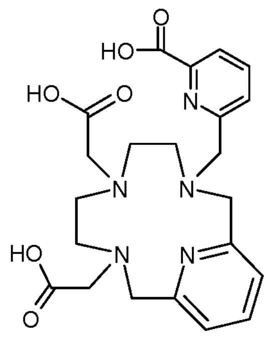

**Pc-2a1pa asym**

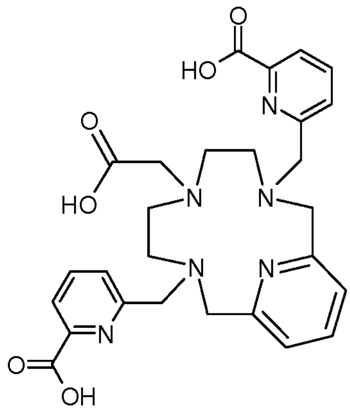

**Pc-1a2pa sym**

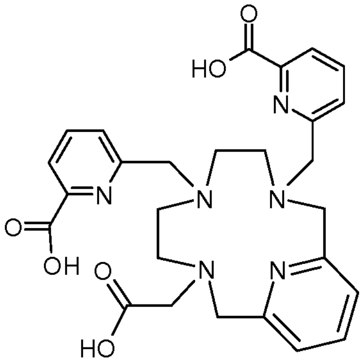

**Pc-1a2pa asym**

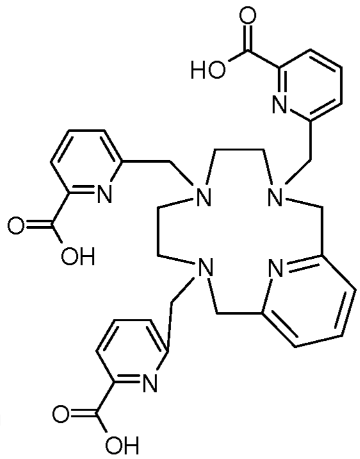

**Pc3pa**

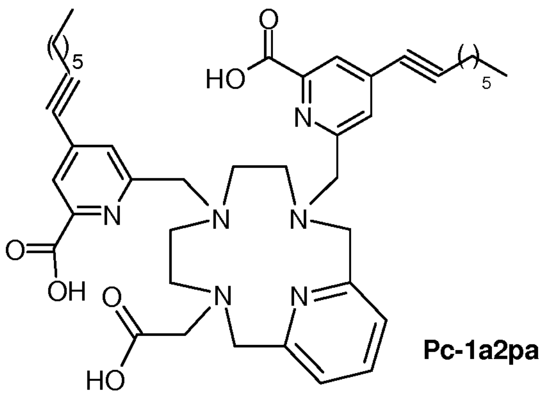

**Pc-1a2pa asym C8**

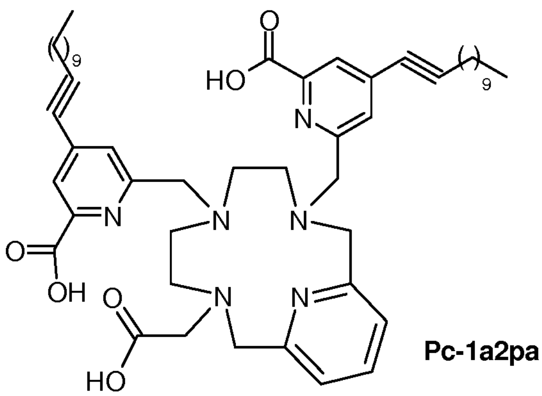

**Pc-1a2pa asym C12**

**5.** Complexe d'un composé de formule (I) ou d'un de ses sels selon l'une quelconque des revendications 1 à 4, avec M ; M étant un métal.

**6.** Complexe selon la revendication 5, pour son utilisation dans le traitement des cancers, en particulier des cancers du foie.

**7.** Utilisation d'un complexe selon la revendication 5 en imagerie médicale.

**8.** Composition pharmaceutique comprenant un composé selon l'une quelconque des revendications 1 à 4 ou un complexe selon l'une quelconque des revendications 5 ou 6, et éventuellement un ou plusieurs excipient(s) pharmaceutiquement acceptable(s).

**9.** Composition pharmaceutique selon la revendication 8, comprenant en outre une huile iodée, notamment une huile iodée comprenant des esters éthyliques d'acides gras iodés de l'huile d'oeillette.

**10.** Procédé de préparation des composés de formule générale (I) suivante :

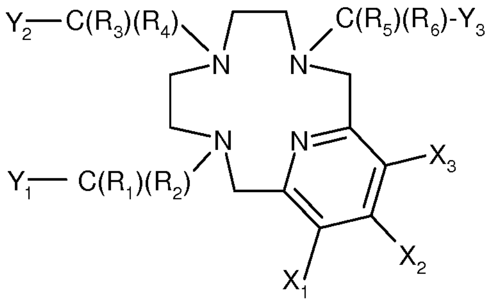

(I)

$X_1$, $X_2$, $X_3$, $Y_1$, $Y_2$, $Y_3$ et $R_1$ à $R_6$ étant tels que définis à la revendication 1 et pour lesquels les groupements $-C(R_1)(R_2)-Y_1$ et $-C(R_5)(R_6)-Y_3$ sont différents, comprenant une étape de fonctionnalisation d'un composé de formule générale (IX) suivante :

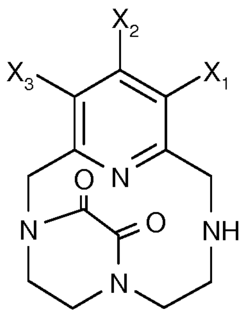

(IX),

pour former un composé de formule générale (X) suivante :

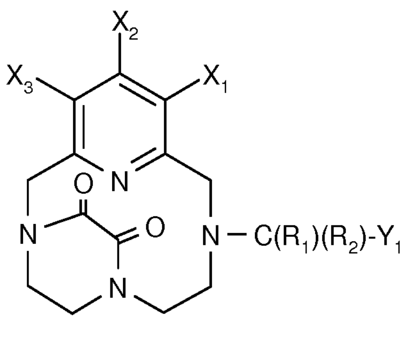

(X),

dans lesquelles $X_1$, $X_2$, $X_3$, $R_1$, $R_2$ et $Y_1$ sont tels que définis à la revendication 1.

**11.** Composé de formule générale (X) suivante :

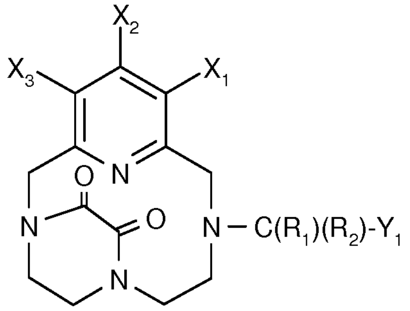

(X),

dans laquelle $X_1$, $X_2$, $X_3$, $R_1$, $R_2$ et $Y_1$ sont tels que définis à la revendication 1.

## Patentansprüche

**1.** Verbindung der folgenden allgemeinen Formel (I):

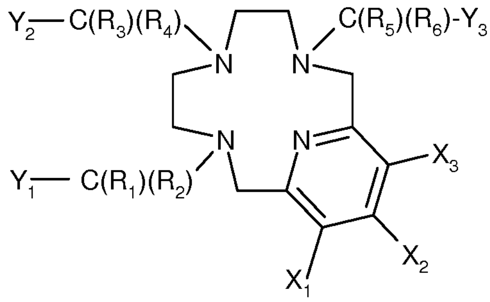

(I)

in der:

- $R_1$, $R_2$, $R_3$, $R_4$, $R_5$ und $R_6$ unabhängig voneinander für H, eine $(C_1-C_{20})$Alkylgruppe oder eine $(C_1-C_{20})$-Alkylen-$(C_6-C_{10})$ arylgruppe stehen;
wobei die Alkyl-, Alkylen- und Arylgruppen gegebenenfalls durch einen oder mehrere Substituenten, die aus organischen Säurefunktionen ausgewählt sind, substituiert sein können;
- $X_1$, $X_2$ und $X_3$ unabhängig voneinander aus der Gruppe bestehend aus H, -C(O)N(Re)(Rd), $(C_1-C_{20})$Alkyl, $(C_2-C_{20})$Alkenyl, $(C_2-C_{20})$Alkinyl und $(C_6-C_{10})$Aryl ausgewählt sind,
wobei Re und Rd unabhängig voneinander für H oder eine $(C_1-C_{20})$Alkylgruppe stehen,
wobei die Alkyl-, Alkenyl- und Alkinylgruppen gegebenenfalls ein oder mehrere Heteroatome und/oder ein oder mehrere $(C_6-C_{10})$Arylene in ihrer Kette umfassen können und gegebenenfalls durch ein $(C_6-C_{10})$Aryl substituiert sein können;
wobei die Alkyl-, Alkenyl-, Alkinyl- und Arylgruppen gegebenenfalls durch einen oder mehrere Substituenten, die aus organischen Säurefunktionen ausgewählt sind, substituiert sein können;
- $Y_1$, $Y_2$ und $Y_3$ unabhängig voneinander für eine C(O)OH-Gruppe oder eine Gruppe der folgenden Formel (II) stehen:

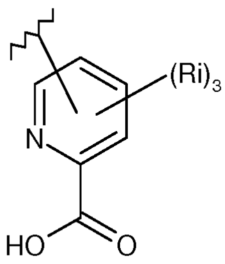

(II)

in der:

- die Reste Ri unabhängig voneinander aus der Gruppe bestehend aus H, Halogen, $N_3$, $(C_1-C_{20})$Alkyl, $(C_2-C_{20})$Alkenyl, $(C_2-C_{20})$Alkinyl und $(C_6-C_{10})$Aryl ausgewählt sind, wobei die Alkyl-, Alkenyl- und Alkinylgruppen gegebenenfalls ein oder mehrere Heteroatome und/oder ein oder mehrere $(C_6-C_{10})$Arylene in ihrer Kette umfassen können und gegebenenfalls durch ein $(C_6-C_{10})$Aryl substituiert sein können;
  wobei die Alkyl-, Alkenyl-, Alkinyl- und Arylgruppen gegebenenfalls durch eine oder mehrere Substituenten, die aus organischen Säurefunktionen ausgewählt sind, substituiert sein können;
  und
  wobei mindestens einer der Reste $Y_1$, $Y_2$ und $Y_3$ für eine Gruppe der Formel (II) steht;

oder ein pharmazeutisch unbedenkliches Salz davon.

**2.** Verbindung nach Anspruch 1, wobei dann, wenn die Reste $Y_1$, $Y_2$ und $Y_3$ für eine Gruppe der Formel (II) stehen, die entsprechenden Reste $R_1$ und $R_2$, $R_3$ und $R_4$ oder $R_5$ und $R_6$ für H stehen.

**3.** Verbindung nach Anspruch 1 oder 2, wobei die Reste Ri unabhängig voneinander aus der Gruppe bestehend aus H, $(C_1-C_{20})$Alkyl, $(C_2-C_{20})$Alkenyl und $(C_2-C_{20})$Alkinyl ausgewählt sind, wobei die Alkyl-, Alkenyl- und Alkinylgruppen gegebenenfalls ein oder mehrere Heteroatome, die aus N, O oder S ausgewählt sind, umfassen können.

**4.** Verbindung der Formel (I) nach Anspruch 1, ausgewählt aus der Gruppe bestehend aus den folgenden Verbindungen:

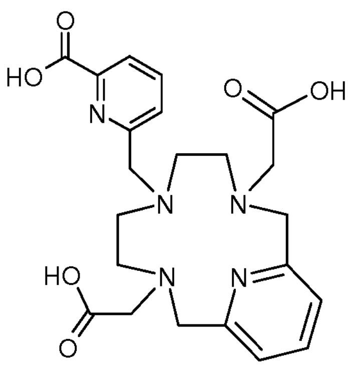

**Pc-2a1pa sym**

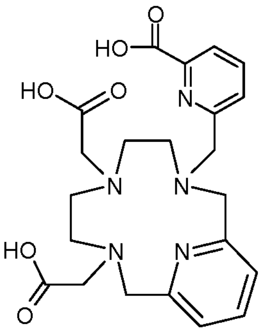

Pc-2a1pa asym

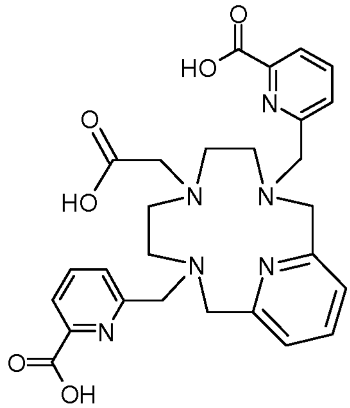

Pc-1a2pa sym

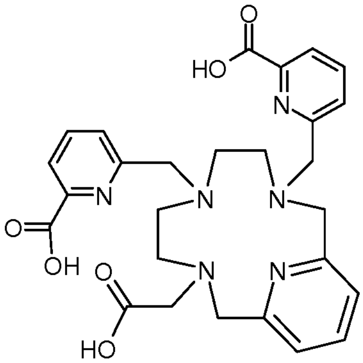

Pc-1a2pa asym

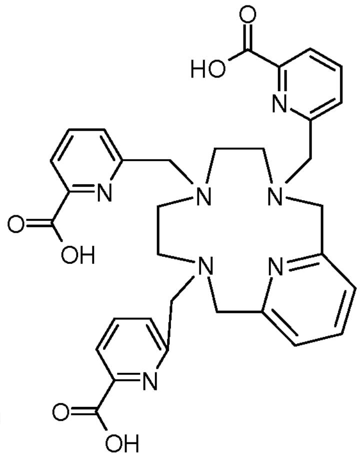

Pc3pa

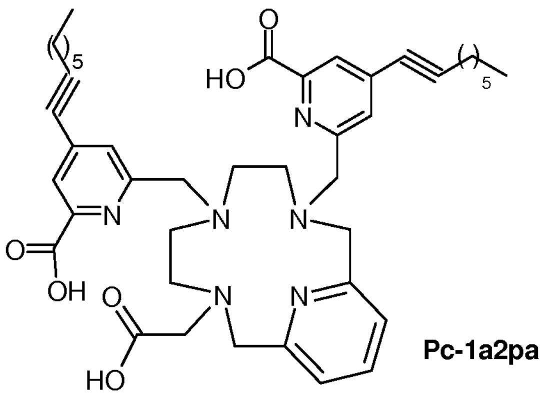

Pc-1a2pa asym C8

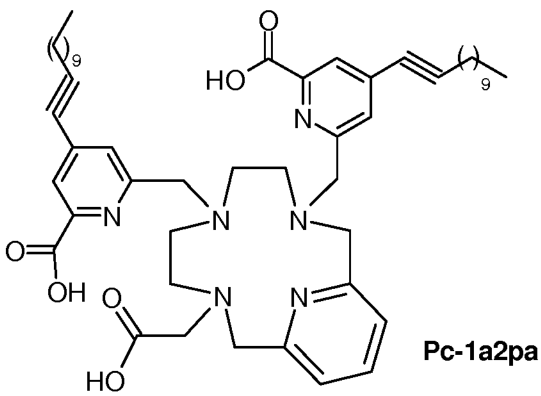

Pc-1a2pa asym C12

5. Komplex einer Verbindung der Formel (I) oder eines Salzes davon nach einem der Ansprüche 1 bis 4 mit M; wobei M für ein Metall steht.

6. Komplex nach Anspruch 5 zur Verwendung bei der Behandlung von Krebserkrankungen, insbesondere Leberkrebserkrankungen.

7. Verwendung eines Komplexes nach Anspruch 5 bei der medizinischen Bildgebung.

8. Pharmazeutische Zusammensetzung, umfassend eine Zusammensetzung nach einem der Ansprüche 1 bis 4 oder einen Komplex nach einem der Ansprüche 5 oder 6 und gegebenenfalls einen oder mehrere pharmazeutisch unbedenkliche Hilfsstoffe.

9. Pharmazeutische Zusammensetzung nach Anspruch 8, außerdem umfassend ein iodiertes Öl, insbesondere ein iodiertes Öl, das Ethylester von iodierten Mohnölfettsäureestern umfasst.

10. Verfahren zur Herstellung der Verbindungen der folgenden allgemeinen Formel (I):

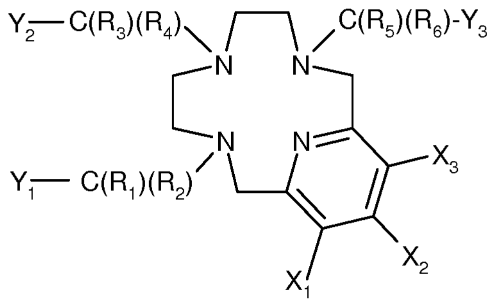

(I)

wobei $X_1$, $X_2$, $X_3$, $Y_1$, $Y_2$, $Y_3$ und $R_1$ bis $R_6$ wie in Anspruch 1 definiert sind und für die die Gruppen $-C(R_1)(R_2)-Y_1$ und $-C(R_5)(R_6)-Y_3$ verschieden sind, umfassend einen Schritt der Funktionalisierung einer Verbindung der folgenden allgemeinen Formel (IX):

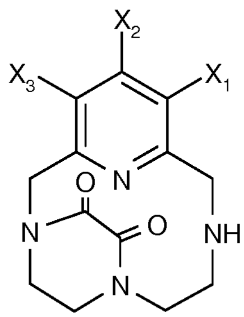

(IX)

zur Bildung einer Verbindung der folgenden allgemeinen Formel (X):

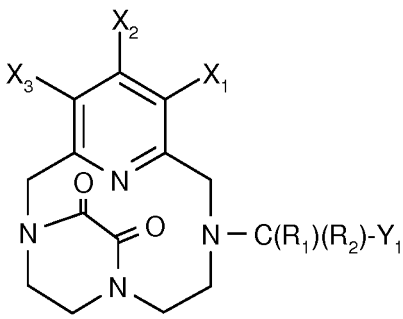

(X),

in der $X_1$, $X_2$, $X_3$, $R_1$, $R_2$ und $Y_1$ wie in Anspruch 1 definiert sind.

**11.** Verbindung der folgenden allgemeinen Formel (X):

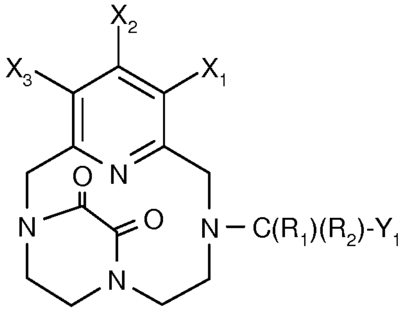

(X),

in der $X_1$, $X_2$, $X_3$, $R_1$, $R_2$ und $Y_1$ wie in Anspruch 1 definiert sind.

## Claims

**1.** Compound of general formula (I) below:

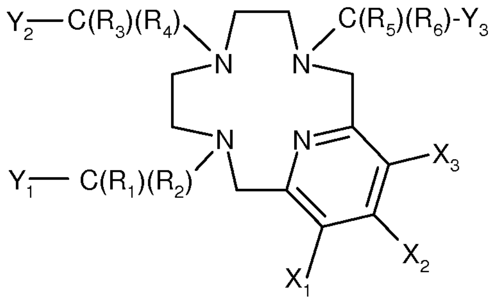

(I)

in which:

- $R_1$, $R_2$, $R_3$, $R_4$, $R_5$ and $R_6$ represent, independently of each other, H, a $(C_1-C_{20})$alkyl group or a $(C_1-C_{20})$alkylene-$(C_6-C_{10})$aryl group;
said alkyl, alkylene and aryl groups possibly being substituted with one or more substituents chosen from organic acid functions;
- $X_1$, $X_2$ and $X_3$ are chosen, independently of each other, from the group constituted by: H,-C(O)N(Re)(Rd), $(C_1-C_{20})$alkyl, $(C_2-C_{20})$alkenyl, $(C_2-C_{20})$alkynyl and $(C_6-C_{10})$aryl,
with Re and Rd being, independently of each other, H or a $(C_1-C_{20})$alkyl group,
said alkyl, alkenyl and alkynyl groups possibly comprising one or more heteroatoms and/or one or more $(C_6-C_{10})$arylenes in their chain and possibly being substituted with a $(C_6-C_{10})$aryl;
said alkyl, alkenyl, alkynyl and aryl groups possibly being substituted with one or more substituents chosen from organic acid functions;
- $Y_1$, $Y_2$ and $Y_3$ represent, independently of each other, a C(O)OH group or a group of formula (II) below:

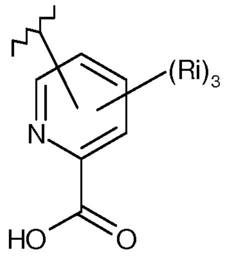

(II)

in which:

- the radicals Ri are chosen, independently of each other, from the group constituted by: H, halogen, $N_3$, $(C_1-C_{20})$alkyl, $(C_2-C_{20})$alkenyl, $(C_2-C_{20})$alkynyl and $(C_6-C_{10})$aryl,

said alkyl, alkenyl and alkynyl groups possibly comprising one or more heteroatoms and/or one or more $(C_6-C_{10})$arylenes in their chain and possibly being substituted with a $(C_6-C_{10})$aryl;
said alkyl, alkenyl, alkynyl and aryl groups possibly being substituted with one or more substituents chosen from organic acid functions; and
at least one of the radicals $Y_1$, $Y_2$ and $Y_3$ being a group of formula (II);
or a pharmaceutically acceptable salt thereof.

**2.** Compound according to Claim 1, in which, when the radicals $Y_1$, $Y_2$ or $Y_3$ represent a group of formula (II), the corresponding radicals $R_1$ and $R_2$, $R_3$ and $R_4$ or $R_5$ and $R_6$ represent H.

**3.** Compound according to Claim 1 or 2, in which the radicals Ri are chosen, independently of each other, from the group constituted by: H, $(C_1-C_{20})$alkyl, $(C_2-C_{20})$alkenyl and $(C_2-C_{20})$alkynyl,
said alkyl, alkenyl and alkynyl groups possibly comprising one or more heteroatoms chosen from N, O and S.

**4.** Compound of formula (I) according to Claim 1, chosen from the group constituted by the following compounds:

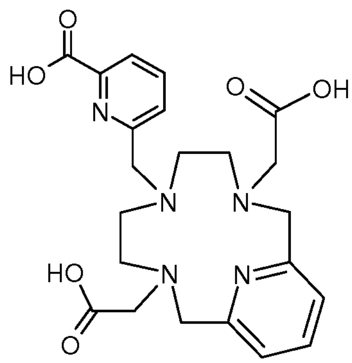

**Pc-2a1pa sym**

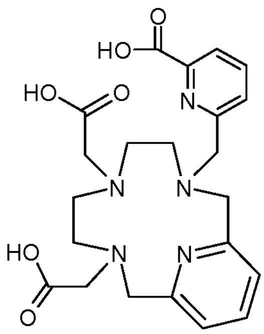

**Pc-2a1pa asym**

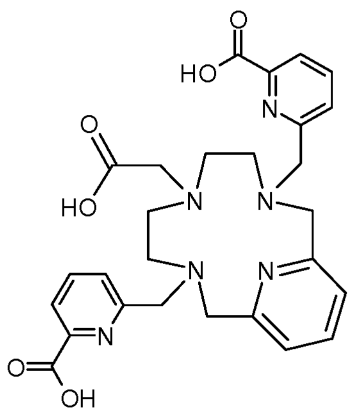

**Pc-1a2pa sym**

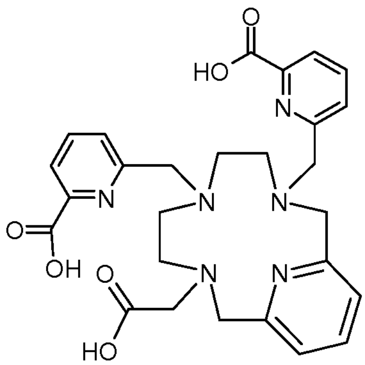

Pc-1a2pa asym

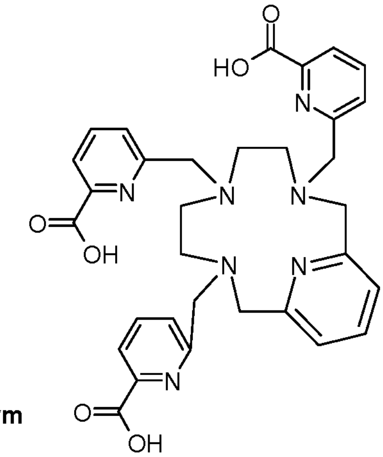

Pc3pa

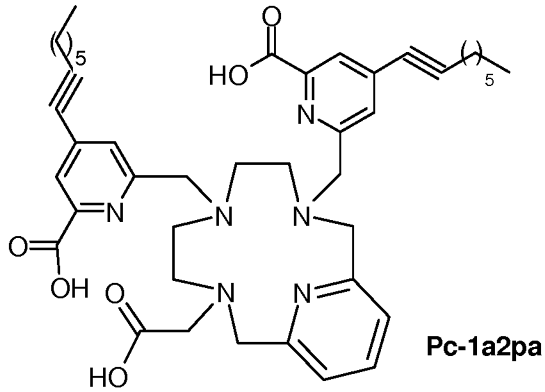

Pc-1a2pa asym C8

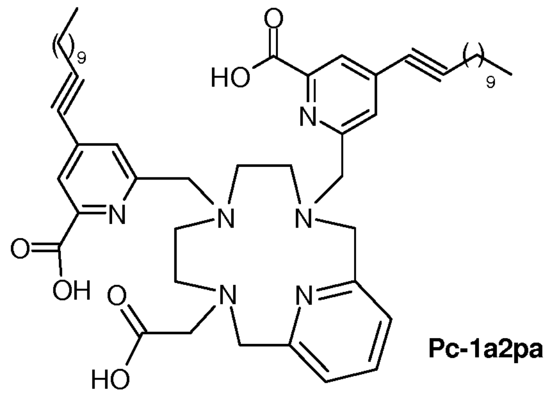

Pc-1a2pa asym C12

5. Complex of a compound of formula (I) or a salt thereof according to any one of Claims 1 to 4, with M; M being a metal.

6. Complex according to Claim 5, for its use in the treatment of cancer, in particular liver cancer.

7. Use of a complex according to Claim 5, in medical imaging.

8. Pharmaceutical composition comprising a compound according to any one of Claims 1 to 4 or a complex according to either of Claims 5 and 6 and optionally one or more pharmaceutically acceptable excipients.

9. Pharmaceutical composition according to Claim 8, also comprising an iodinated oil, especially an iodinated oil comprising ethyl esters of iodinated fatty acids of poppy oil.

10. Process for preparing the compounds of general formula (I) below:

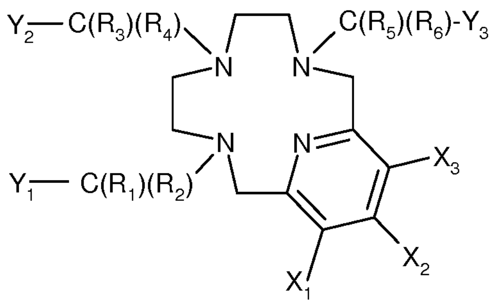

(I)

$X_1$, $X_2$, $X_3$, $Y_1$, $Y_2$, $Y_3$ and $R_1$ to $R_6$ being as defined in Claim 1 and for which the groups $-C(R_1)(R_2)-Y_1$ and $-C(R_5)(R_6)-Y_3$ are different, comprising a step of functionalizing a compound of general formula (IX) below:

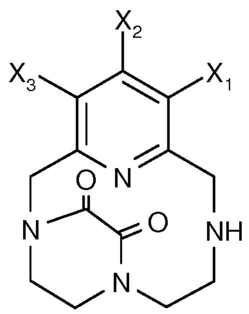

(IX),

to form a compound of general formula (X) below:

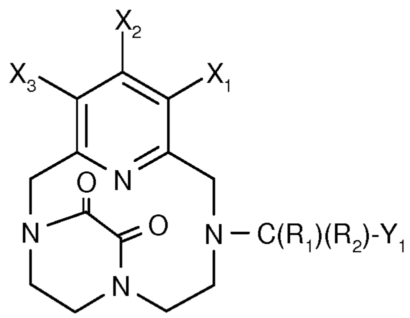

(X),

in which $X_1$, $X_2$, $X_3$, $R_1$, $R_2$ and $Y_1$ are as defined in Claim 1.

**11.** Compound of general formula (X) below:

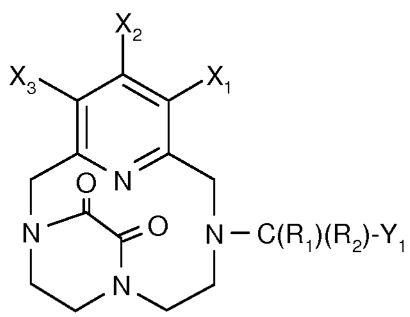

(X),

in which $X_1$, $X_2$, $X_3$, $R_1$, $R_2$ and $Y_1$ are as defined in Claim 1.

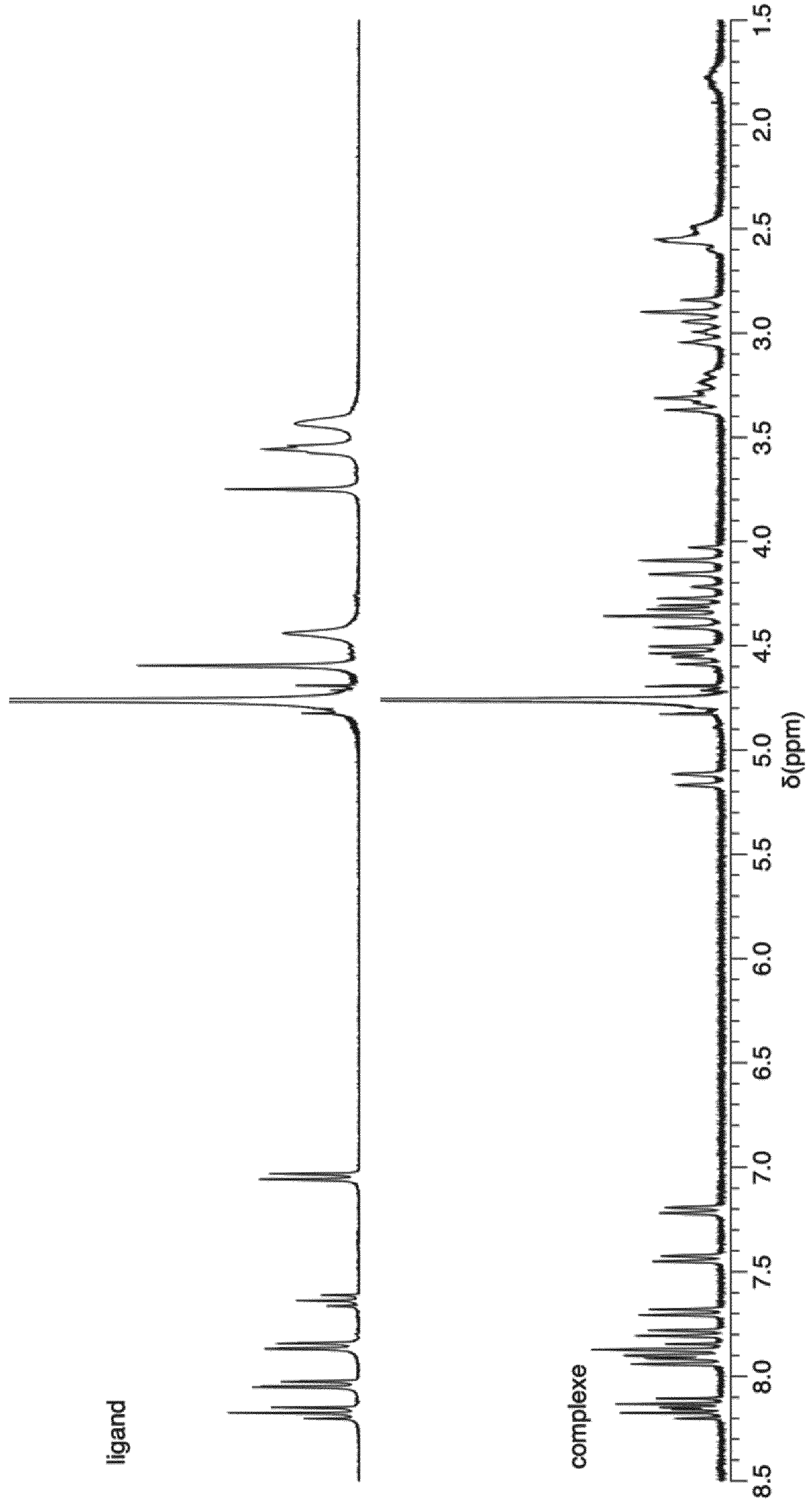


FIG.1

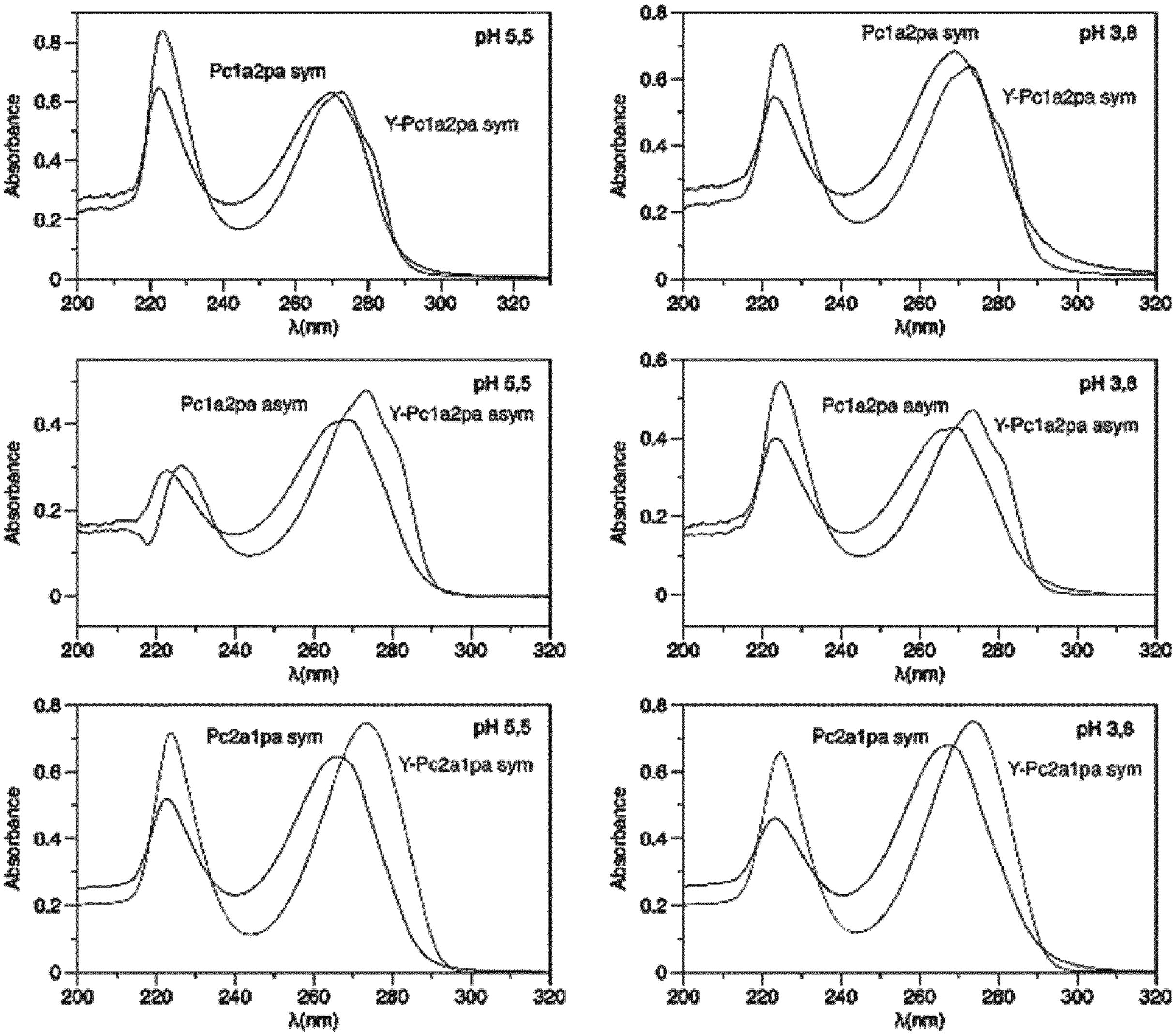
pH 5,5
Pc1a2pa sym
Y-Pc1a2pa sym
pH 3,8
Pc1a2pa sym
Y-Pc1a2pa sym
pH 5,5
Pc1a2pa asym
Y-Pc1a2pa asym
pH 3,8
Pc1a2pa asym
Y-Pc1a2pa asym
pH 5,5
Pc2a1pa sym
Y-Pc2a1pa sym
pH 3,8
Pc2a1pa sym
Y-Pc2a1pa sym
Absorbance
λ(nm)

FIG.2

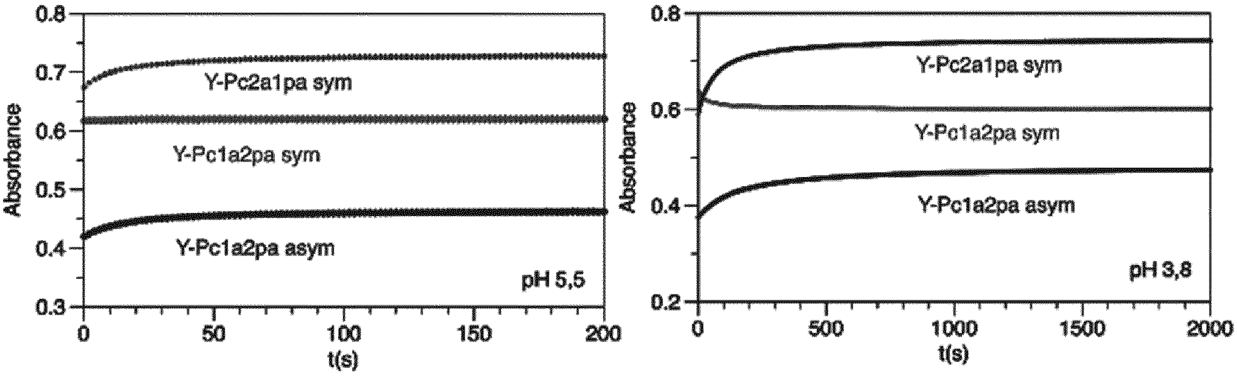
Y-Pc2a1pa sym
Y-Pc1a2pa sym
Y-Pc1a2pa asym
pH 5,5
pH 3,8
Absorbance
t(s)

FIG.3

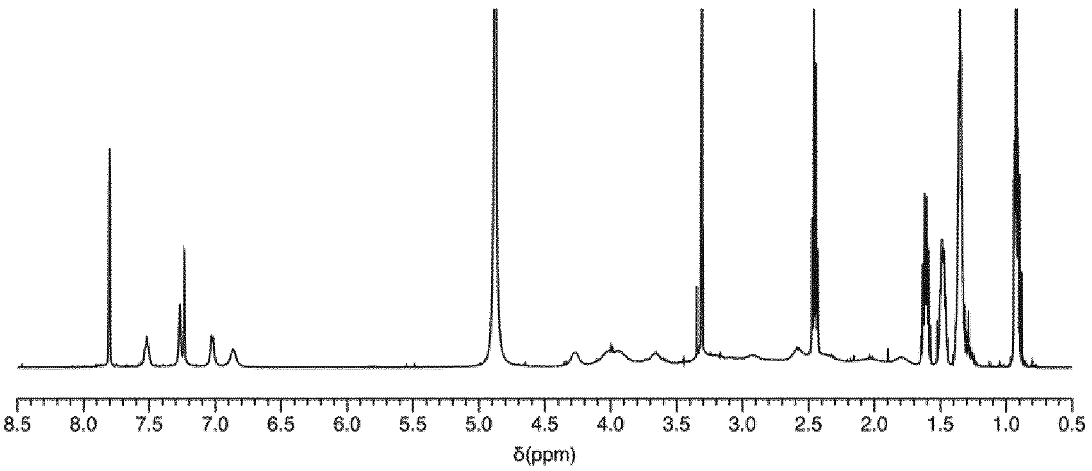
8.5
8.0
7.5
7.0
6.5
6.0
5.5
5.0
4.5
4.0
3.5
3.0
2.5
2.0
1.5
1.0
0.5
δ(ppm)

FIG.4

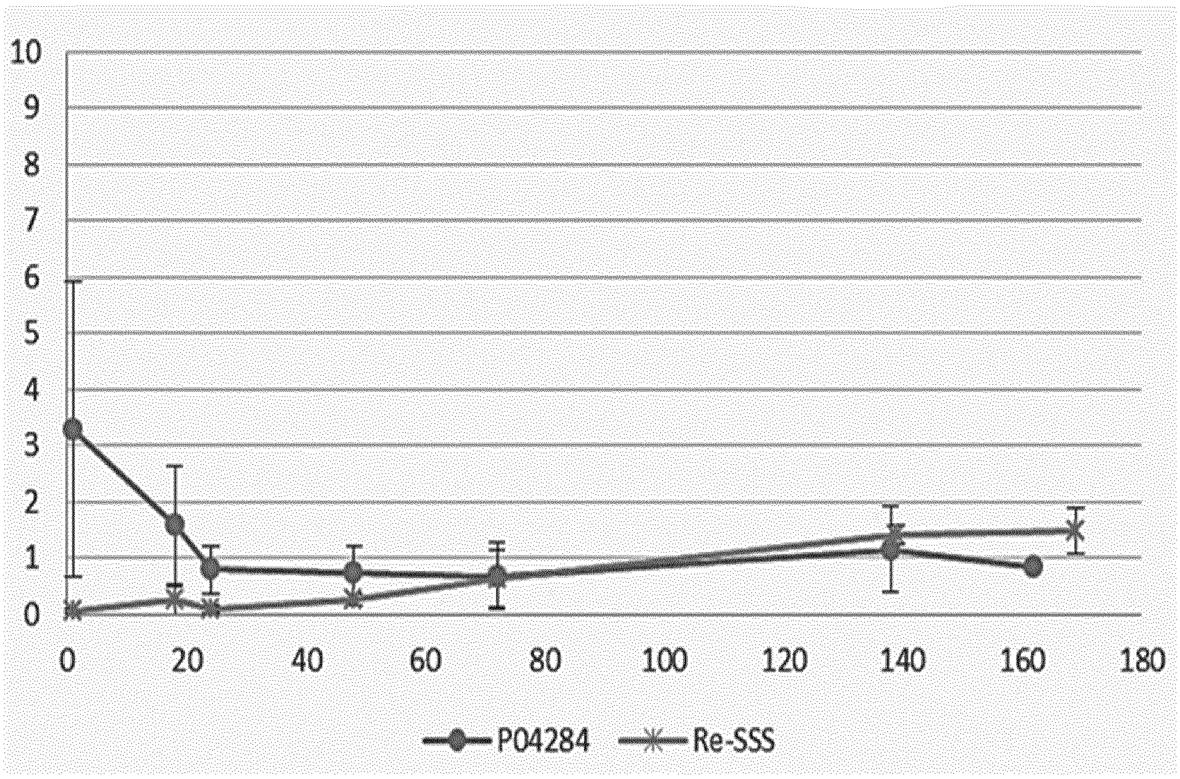
10
9
8
7
6
5
4
3
2
1
0
0
20
40
60
80
100
120
140
160
180
P04284
Re-SSS

FIG.5

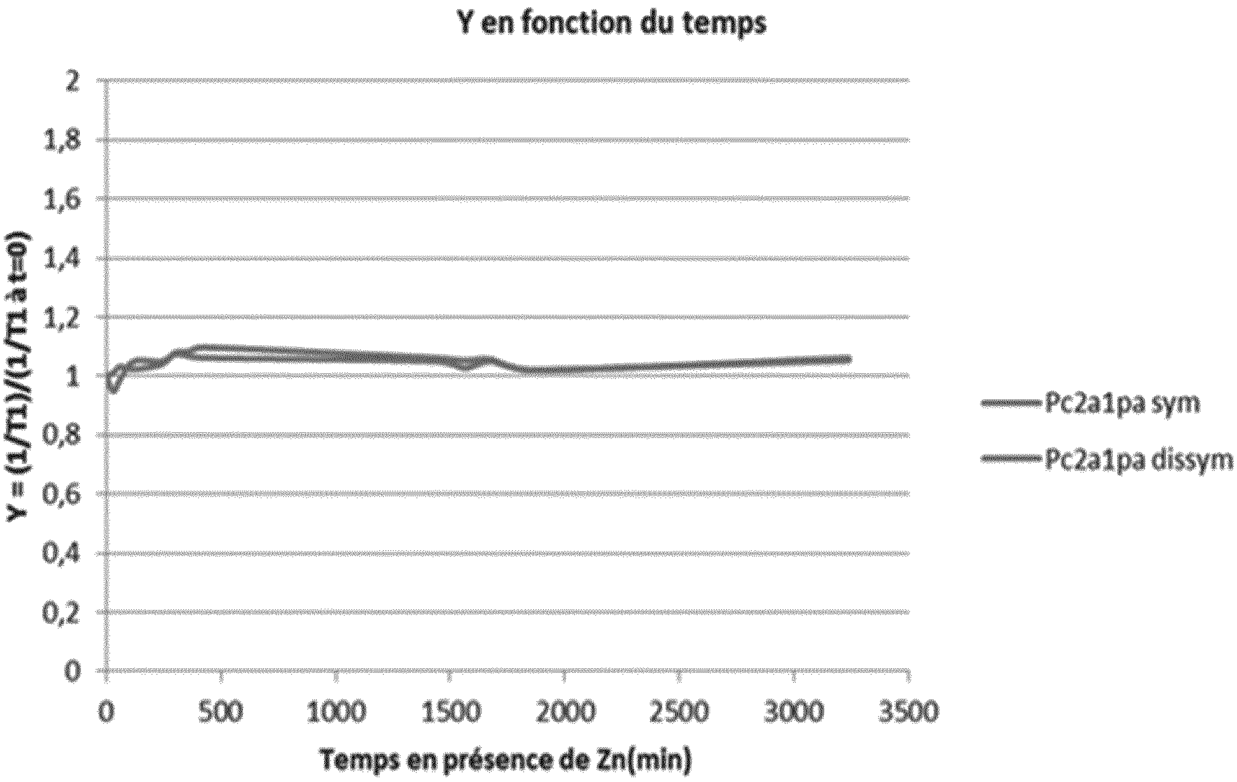
Y en fonction du temps
Y = (1/T1)/(1/T1 à t=0)
2
1,8
1,6
1,4
1,2
1
0,8
0,6
0,4
0,2
0
0
500
1000
1500
2000
2500
3000
3500
Temps en présence de Zn(min)
Pc2a1pa sym
Pc2a1pa dissym

FIG.6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **WOLFF et al.** *Medicine,* 2001, vol. 80, 20-36
- **BERGE et al.** *J. Pharm. Sd,* 1977, vol. 66, 1
- **LOIC BELLOUARD.** *J CHEM S Perkin,* 1999, vol. 1 (23), 3499-3505
- *Tetrahedron,* vol. 57 (22), 4713-4718
- **RICHMAN ; ATKINS.** *J. Am. Chem. Soc.,* 1974, vol. 96, 2268-2270
- **SONOGASHIRA.** *Comprehensive Chirality,* 2012, vol. 4, 18-32
- **SCHWARZENBACH, G. ; FLASCHKA, W.** Complexometric Titrations. Methuen & Co, 1969
- **GANS, P. ; SABATINI, A. ; VACCA, A.** *Talanta,* 1996, vol. 43, 1739-1753
- **ALDERIGHI, L. ; GANS, P. ; IENCO, A. ; PETERS, D. ; SABATINI, A. ; VACCA, A.** *Coord. Chem. Rev.,* 1999, vol. 184, 311-318
- **AIME, S. ; BOTTA, M. ; GENINATTI CRICH, S. ; GIOVENZANA, G. B. ; JOMMI, G. ; PAGLIARIN, R. ; SISTI, M.** *Inorg. Chem.,* 1997, vol. 36, 2992-3000
- **DELGADO, R. ; FIGUEIRA, C. ; QUINTINO, S.** *Talanta,* 1997, vol. 45, 451
- **TIRCSÓ, G. ; KOVACS, Z. ; DEAN SHERRY, A.** *Inorg. Chem.,* 2006, vol. 45, 9269
- **CHAVES, S. ; DELGADO, R. ; FRAUSTO DA SILVA, J. J. R.** *Talanta,* 1992, vol. 39, 249
- **KUMAR, K. ; CHANG C. A. ; FRANCESCONI, L. C. ; DISCHINO, D. D. ; MALLEY, M. F. ; GOUGOUTAS, J. Z. ; TWEEDLE, M.F.** *Inorg. Chem.,* 1994, vol. 33, 3567
- **DELGADO, R. ; FRAUSTO DA SILVA, J. J. R.** *Talanta,* 1982, vol. 29, 815
- **COX, J. P. L. ; JANKOWSKI, K. J. ; KATAKY, R. ; PARKER, D. ; BEELEY, N. R. A. ; BOYCE, B. A. ; EATON, M. A. W. ; MILLAR, K. ; MILLICAN, A. T. ; HARRISON, A.** *J. Chem. Soc. Chem. Commun.,* 1989, vol. 797